# EUROPEAN PATENT APPLICATION

(11) **EP 4 681 730 A1**
(43) Date of publication of application: **21.01.2026**
(21) Application number: 24770994.2
(22) Date of filing: 15.03.2024
(51) Int. Cl.: A61K 39/395, A61K 9/08, A61K 47/02, A61K 47/12, A61K 47/18, A61K 47/26, A61P 1/04, A61P 17/06, A61P 19/02, A61P 21/04, A61P 29/00, A61P 35/00, A61P 37/02, A61P 37/06, C07K 16/28, C07K 16/46

(54) **ANTIBODY PREPARATION**

(30) Priority: 16.03.2023 JP 2023041761; 21.09.2023 JP 2023154195
(71) Applicant: ONO Pharmaceutical Co., Ltd., Osaka-shi, Osaka 541-8526 (JP)
(72) Inventor: NOZAKI, Shinya, Mishima-gun, Osaka 618-8585 (JP); INAGAKI, Ryo, Mishima-gun, Osaka 618-8585 (JP); ARIMA, Naoki, Mishima-gun, Osaka 618-8585 (JP); ODA, Atsushi, Mishima-gun, Osaka 618-8585 (JP); MATSUNAGA, Atsushi, Mishima-gun, Osaka 618-8585 (JP); MIYANABE, Kazuhiro, Mishima-gun, Osaka 618-8585 (JP); TOGASHI, Yusuke, Osaka-shi, Osaka 541-8564 (JP); MAEIE, Tadahiro, Mishima-gun, Osaka 618-8585 (JP)
(74) Representative: Grünecker Patent- und Rechtsanwälte PartG mbB
(86) International application number: PCT/JP2024/010151
(87) International publication number: WO 2024/190892

(57) **Abstract**

The problem addressed by the present invention is to provide a novel preparation that contains an anti-PD-1/CD3 bispecific antibody as an active ingredient and that is for the prevention, suppression of symptom development, suppression of recurrence, or treatment of autoimmune diseases, or a graft-versus-host disease (GVHD) or hematological cancer. The invention provides an aqueous pharmaceutical composition and a preparation comprising same. The aqueous pharmaceutical composition contains an anti-PD-1/CD3 bispecific antibody as an active ingredient, and further contains, for example, an acetate buffer, trehalose, L-methionine, polysorbate 80, and sodium chloride. The aqueous pharmaceutical composition can be used for both subcutaneous and intravenous administration in the form of single excipient composition.

## Description

### [Technical field]

The present disclosure relates to a pharmaceutical composition comprising an antibody, in particular a bispecific antibody capable of specifically binding to PD-1 and CD3, respectively (hereinafter, may be abbreviated as an "anti-PD-1/CD3 bispecific antibody") as an active ingredient (hereinafter, may be abbreviated as a "pharmaceutical composition of the present invention") and a formulation thereof (hereinafter, may be abbreviated as a "formulation of the present invention").

### [Background Art]

PD-1 is an immunosuppressive receptor belonging to an immunoglobulin family and is a molecule having a function suppressing immune activation signals of T-cells activated by stimulation through an antigen receptor. From analysis of PD-1 knock-out mice or the like, it is known that PD-1 signals play an important role in suppression of autoimmune diseases such as autoimmune dilated cardiomyopathy, lupus-like syndrome, autoimmune encephalomyelitis, systemic lupus erythematosus, graft-versus-host disease, type I diabetes mellitus and rheumatoid arthritis. Accordingly, it is pointed out that a substance enhancing the PD-1 signals could be a prophylactic or therapeutic agent for autoimmune diseases.

To date, bispecific antibodies recognizing PD-1 have been known as substances enhancing the PD-1 signals (Patent references 1 to 5). The bispecific antibodies are composed of an antigen-recognition site of an antibody recognizing CD3 that is a member of T-cell receptor complex and an antigen-recognition site of an antibody recognizing PD-1, which are linked to each other using genetic engineering, and it has an action of enhancing the inhibitory signal of PD-1 against T-cell receptor complex by increasing the frequency of locating PD-1 to the vicinity of T-cell receptor complex.

The pharmaceutical composition of the present invention is characterized by comprising the anti-PD-1/CD3 bispecific antibody in the concentration range of about 0.01 mg/mL to about 200 mg/mL, as an active ingredient, and containing an acetate buffer, trehalose, L-methionine, polysorbate 80 and sodium chloride, as one embodiment of formulation additives thereof, and the formulations listed in the patent literatures 6 to 14 have been reported as antibody formulations.

The patent literature 6 refers to a formulation in which immunosuppressive effects in target tissues can be minimised by non-systemic administration such as intravenous administration, to tissuespecifically immunomodulate by an anti-CD3 antibody. It discloses, as formulations like that, an oral formulation containing 0.1 to 10 mg of the anti-CD3 antibody per dose as an active ingredient and sodium acetate trihydrate, sodium chloride, polysorbate 80. trehalose and methionine as formulation additives thereof, and a subcutaneous injection formulation containig 2 mg/mL of the anti-CD3 antibody as an active ingredient and sodium acetate trihydrate, sodium chloride and polysorbate 80 as formulation additives thereof, respectively. However, it does not substantially disclose that any formulations comprising those additives can be used as intravenous formulations which are difficult to minimise immunosuppressive effects in target tissues, and further does not at all indicate that trehalose and L-methionine could be further added to the above subcutaneous injection formulation. Furthermore, the concentration of polysorbate 80 in both formulations of this literature is equivalent to 0.02 % (w/v), but it does not at all indicate that about 0.1 % (w/v) is optimal or necessary for the pharmaceutical composition of the present invention in terms of supressing aggregation.

The patent literature 7 discloses a formulation containig 150 mg/mL of anti-OX40L antibody as an active ingredient and an acetate buffer, trehalose, polysorbate 20 and methionine as formulation additives thereof. Herein, among the additives of the formulation of this literature, trehalose and methionine are used as tonicity agents or stabilizing agents, but it does not at all indicate that in the presence of trehalose and methionine, sodium chloride that can similarly function as a tonicity agent or stabilizing agent is further added thereto. Furthermore, it does not at all indicate that polysorbate 20 contained in the formulation example showing preferable performance is used as a substitute for polysorbate 80.

The patent literature 8 discloses a formulation containing 150 mg/mL of anti-GM-CSF-Rα antibody as an active ingredient and an acetate buffer, sodium chloride, polysorbate 80 and trehalose as formulation additives thereof, but does not at all indicate about the addition of L-methionine.

Furthermore, while it is usual for a formulation which can be applied to an antibody having a low or neutral pI value to be set at a pH deviating by 1 pH from a pI value of the antibody, taking into consideration the colloidal stability of the antibody, the patent literature 8 is designed to apply to a formulation of which the pH is set in the range of 5.5 to 7.5 close to the pI value of the antibody. Thus, the composition in the formulation can not be applied to any pharmaceutical compositions with a pH other than that (e.g. the pharmaceutical compositions of the present invention with a pH of 4.7 to 5.3).

The patent literature 9 discloses a formulation containing about 45 to 55 mg/mL of adalimumab as an active ingredient and an acetate buffer, sodium chloride, polysorbate 80 and trehalose as formulation additives thereof, but indicates that it does not contain substantially or at all any free amino acids, or even if present, at 0.1 mM or less. Further, it discloses that the presence of free amino acids, including L-methionine, significantly worsens the stability of antibody (fragmentation of antibody at pH 5.0 or less). Furthermore, while uncomplicated formulations adopting fewer ingredients are desired, it indicates that since trehalose is very promising with regard to antioxidant properties, the further addition of antioxidants is not required.

While the patent literatures 10 and 11 disclose formulations containing 20 mg/mL of anti-EGFR antibody as an active ingredient and an acetate buffer, sodium chloride, polysorbate 80 and trehalose as formulation additives thereof, the concentration of sodium chloride therein is equivalent to 100 mM. On the other hand, the concentration of sodium chloride in the pharmaceutical compositions of the present invention is set to be about 34.2 mM or less, and thus at a concentration equivalent to 100 mM, the generation of aggregates is significant. which is not applicable to it. Furthermore, it does not at all indicate about the addition of L-methionine.

While the patent literatures 12 and 13 disclose formulations containing an acetate buffer, polysorbate 80, trehalose, methionine and DTPA to deal with visible particulates occuring prominently in IgG4 isotype antibodies, it does not at all indicate as to whether it can be applied to the anti-PD-1/CD3 bispecificity antibody of the present invention that is an IgG1 isotype antibody with the physical properties different from it (DOI(JaLC): 10.15083/00006491). Furthermore, while the concentration of polysorbate 80 in the formulation of these literatures is set at 0.02 % (w/v). it also does not at all indicate that in terms of suppressing aggregation, about 0.1 % (w/v) is optimal or necessary for the pharmaceutical composition of the present invention.

The patent literature 14 discloses a formulation containing an anti-TIGIT antibody as an active ingredient and as an embodiment of the formulation, an acetate buffer, trehalose. sodium chloride, and polysorbate 80 as additives thereof, but in fact, it discloses that since the addition of sodium chloride showed a rapid decrease in the monomer purity of the antibody, it is preferable not to include it, and that as a result of comparing at 0.02 % with at 0.04 % for the concentration of polysorbate 80, the 0.02 % lower concentration is preferable in terms of rapid decrease in the monomer purity. On the other hand, sodium chloride is added to the pharmaceutical compositions of the present invention, and about 0.1 % (w/v) as a concentration of polysorbate 80 is more preferred.

To date, the pharmaceutical composition of the present invention that contains the anti-PD-1/CD3 bispecific antibody as an active ingredient and is in the composition comprising the above additives, and furthermore of which a single composition can be applied to both subcutaneous and intravenous administrations of the antibody in a wide concentration range has not been found, and it is not suggested at all from any of the formulations disclosed in the above patent literatures.

### [Citation List]

### [Patent Literature]

Patent Literature 1: International Publication No. 2003/011911
Patent Literature 2: International Publication No. 2004/072286
Patent Literature 3: International Publication No. 2013/022091
Patent Literature 4: International Publication No. 2019/156199
Patent Literature 5: International Publication No. 2021/020416
Patent Literature 6: Japanese Translation of PCT International Application Publication No. 2019-526627
Patent Literature 7: International Publication No. 2011/161226
Patent Literature 8: Japanese Translation of PCT International Application Publication No. 2020-509025
Patent Literature 9: Japanese Translation of PCT International Application Publication No. 2017-516846
Patent Literature 10: Chinese Patent Application Publication No. 107987161
Patent Literature 11: Chinese Patent Application Publication No. 115869395
Patent Literature 12: International Publication No. 2023/031969
Patent Literature 13: International Publication No. 2023/031970
Patent Literature 14: International Publication No. 2022/152245

### [Summary of Invention]

### [Technical Problem]

The problem of the present invention is to provide a single pharmaceutical composition and formulation thereof, applicable for both subcutaneous and intravenous administrations with antibodies, particulally the anti-PD-1/CD3 bispecific antibody, which can be used in a wide concentration range.

### [Solution to Problem]

The present inventors diligently studied, and found the pharmaceutical composition and formulation thereof, capable of solving the above-mentioned problem, and then completed the present invention.

In other words, the present invention mainly provides the following embodiments.
<1> an aqueous pharmaceutical composition or a formulation thereof for subcutaneous or intravenous administration, containing an antibody (preferably the anti-PD-1/CD3 bispecific antibody) as an active ingredient and further a buffer (preferably an acetate buffer), trehalose, an antioxidant (preferably L-methionine), polysorbate 80 and sodium chloride,
<2> an aqueous pharmaceutical composition or a formulation thereof for subcutaneous or intravenous administration bioequivalent to the aqueous pharmaceutical composition or formulation thereof described in the preceding item <1>, and
<3> use of the aqueous pharmaceutical composition or formulation thereof described in the preceding item <1> or <2> in the prevention, suppression of symptom progression, suppression of recurrence and/or treatment of autoimmune diseases or graft-versus-host disease (GVHD), or hematological cancers.

### [Advantage Effects of Invention]

The pharmaceutical composition or formulation thereof of the present invention for the anti-PD-1/CD3 bispecific antibody can be a new drug for the prevention, suppression of symptom progression, suppression of recurrence and/or treatment of autoimmune diseases or graft-versus-host disease (GVHD), or hematological cancers.

### [Brief Description of Drawings]

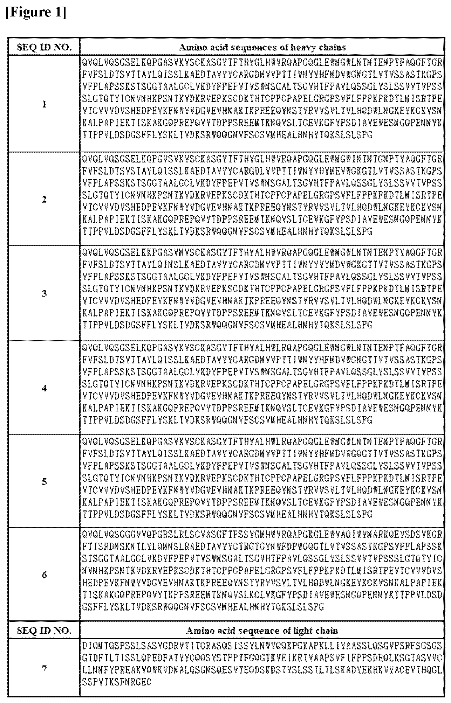

[Figure 1] It shows the respective amino acid sequences of the heavy chain and light chain constituting an antigen-binding site specifically binding to PD-1 and the heavy chain and light chain constituting an antigen-binding site specifically binding to CD3, constituting Anti-PD-1/CD3 bispecific antibody A (details are described below).

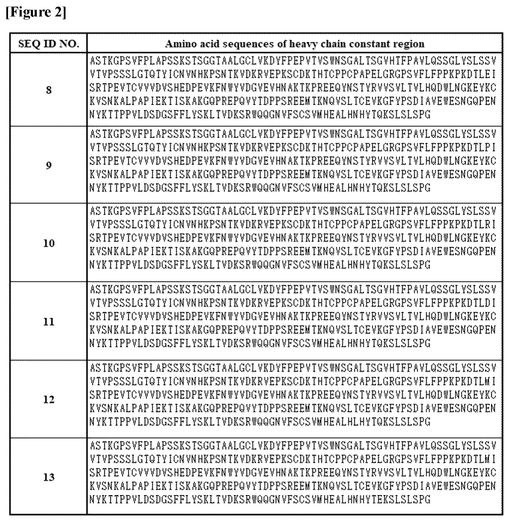

[Figure 2] It shows the respective amino acid sequences of the heavy chain constant regions in the heavy chain and light chain constituting the antigen-binding site specifically binding to PD-1 among the heavy chain constant region constituting Anti-PD-1/CD3 bispecific antibody B (details are described below).

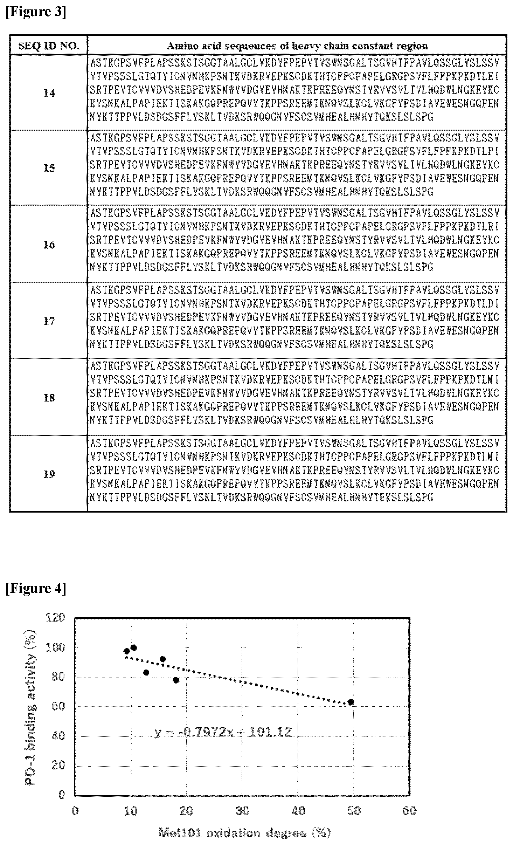

[Figure 3] It shows the respective amino acid sequences of the heavy chain constant region in the heavy chain and light chain constituting the antigen-binding site specifically binding to CD3 among the heavy chain constant region constituting Anti-PD-1/CD3 bispecific antibody B (details are described below).
[Figure 4] It shows the relationship between the binding activity (%) of Antibody α (details are described below) to PD-1 (the relative activity in case that the activity of Antibody α as a standard is as 100 %) and the oxidation degree (%) of the 101st methionine residue (hereinafter, may be abbreviated as "Met101") located in a complementarity-determining region to PD-1 on the antibody (the rate of Antibody α in which the 101st methionine residue is oxidized).

### [Description of Embodiments]

The present invention relates to an antibody, in particular an antibody having the binding specificity for two different antigen molecules or epitopes in one molecule, i.e., a bispecific antibody. The anti-PD-1/CD3 bispecificity antibody of the present invention is,
(i) the heavy chain and light chain constituing the antigen-binding site specifically binding to PD-1, and
(ii) the heavy chain and light chain constituing the antigen-binding site specifically binding to CD3. Herein,
   (a) the heavy chain of the preceding item (i) comprises any one of the amino acid sequences selected from SEQ ID NO:1, SEQ ID NO:2, SEQ ID NO:3, SEQ ID NO:4 and SEQ ID NO:5,
   (b) the heavy chain of the preceding item (ii) comprises the amino acid sequence of SEQ ID NO:6, and
   (c) both of the light chains of the preceding items (i) and (ii) comprise the amino acid sequence of SEQ ID NO:7.

Herein, the term "heavy chain and light chain comprising the antigen-binding site specifically binding to PD-1" means a heavy chain/light chain complex in which the heavy chain and light chain associate via disulfide bonds to form the antigen-binding site specifically binding to PD-1. The term "antigen-binding site" is a minimum unit for an antibody to have the binding activity to an antigen thereof, and the term "specifically binding to PD-1" is used as a feature of directly binding to PD-1 with the binding activity higher than at least 1 x 10⁻⁵ M, preferably 1 x 10⁻⁷ M, and more preferably 1 x 10⁻⁹ M affinity (dissociation constant (Kd value)) and not substantially binding to any other receptor members belonging to a so-called CD28 family receptor such as at least CD28, CTLA-4 and ICOS.

Herein, the term "heavy chain and light chain comprising the antigen-binding site specifically binding to CD3" means a heavy chain/light chain complex in which the heavy chain and light chain associate via disulfide bonds to form the antigen-binding site specifically binding to CD3. Herein, the term "specifically binding to CD3" is used as a feature of directly binding to CD3 with the binding activity higher than at least 1 x 10⁻⁵ M, preferably 1 x 10⁻⁷ M, and more preferably 1 x 10⁻⁹ M affinity (dissociation constant (Kd value)), and not substantially binding to any other proteins.

Among the above-mentioned anti-PD-1/CD3 bispecific antibodies (hereinafter, may be collectively abbreviated as "Anti-PD-1/CD3 bispecific antibody A"), an antibody to which the pharmaceutical composition or formulation thereof of the present invention is applied is preferably the anti-PD-1/CD3 bispecific antibody (hereinafter, may be abbreviated as the "Antibody α") of which the heavy chain comprising the antigen binding site specifically binding to PD-1 comprises the amino acid sequence set forth in SEQ ID NO: 5.

Anti-PD-1/CD3 bispecific antibody A can be produced according to the method disclosed in WO2019/156199, and corresponds to the anti-PD-1/CD3 bispecific antibody clones PD1-1(Bi) to PD1-5(Bi), respectively. and among them, Antibody α corresponds to the clone PD1-5(Bi).

Furthermore, the anti-PD-1/CD3 bispecific antibody in which the heavy chain constant region in the heavy chain constituting the antigen-binding site specifically binding to PD-1 of Antibody α was replaced with a heavy chain constant region comprising any one of the amino acid sequences selected from SEQ ID NO:8, SEQ ID NO:9, SEQ ID NO:10, SEQ ID NO:11, SEQ ID NO:12 and SEQ ID NO:13, and in which the heavy chain constant region in the heavy chain constituting the antigen-binding site specifically binding to CD3 was replaced with a heavy chain constant region comprising any one of the amino acid sequences selected from SEQ ID NO:14, SEQ ID NO:15, SEQ ID NO:16, SEQ ID NO:17, SEQ ID NO:18 and SEQ ID NO:19, (hereinafter. may be collectively abbreviated as "Anti-PD-1/CD3 bispecific antibody B") also can be applied to the pharmaceutical composition or formulation thereof of the present invention. Note that Anti-PD-1/CD3 bispecific antibody B can be produced according to the method disclosed in WO2021/020416.

Note that both of Anti-PD-1/CD3 bispecific antibody A and Anti-PD-1/CD3 bispecific antibody B are IgG1 isotypes.

The pharmaceutical composition of the present invention contains the anti-PD-1/CD3 bispecific antibody at an arbitrary concentration from about 0.01 to about 200 mg/mL (e.g., about 0.01 mg/mL, about 0.02 mg/mL, about 0.03 mg/mL, about 0.04 mg/mL. about 0.05 mg/mL, about 0.06 mg/mL, about 0.07 mg/mL, about 0.08 mg/mL, about 0.09 mg/mL, about 0.1 mg/mL, about 0.2 mg/mL, about 0.3 mg /mL, about 0.4 mg/mL, about 0.5 mg/mL, about 0.6 mg/mL. about 0.7 mg/mL, about 0.8 mg/mL, about 0.9 mg/mL, about 1 mg/mL, about 1.1 mg/mL. about 1.2 mg/mL, about 1.3 mg/mL, about 1.4 mg/mL, about 1.5 mg/mL, about 2 mg/mL, about 5 mg/mL, about 10 mg/mL, about 15 mg/mL, about 20 mg/mL, about 25 mg/mL, about 30 mg/mL, about 40 mg/mL, about 50 mg/mL, about 60 mg/mL, about 70 mg/mL, about 80 mg/mL, about 90 mg/mL, about 100 mg/mL, about 120 mg/mL, about 130 mg/mL, about 140 mg/mL, about 150 mg/mL, about 160 mg/mL, about 170 mg/mL. about 180 mg/mL, about 190 mg/mL or about 200 mg/mL), preferably at an arbitrary concentration from about 0.1 to about 150 mg/mL, more preferably at an arbitrary concentration from about 0.1 to about 1.2 mg/mL (e.g., about 0.1 mg/mL. about 0.2 mg/mL, about 0.3 mg/mL, about 0.4 mg/mL, about 0.5 mg/mL. about 0.6 mg/mL, about 0.7 mg/mL, about 0.8 mg/mL, about 0.9 mg/mL, about 1 mg/mL. about 1.1 mg/mL or about 1.2 mg/mL), at an arbitrary concentration from about 5 to about 25 mg/mL (e.g., about 5 mg/mL, about 10 mg/mL, about 15 mg/mL, about 20 mg/mL or about 25 mg/mL), or at an arbitrary concentration from about 80 to about 150 mg/mL (e.g., about 80 mg/mL, about 90 mg/mL, about 100 mg/mL, about 110 mg/mL, about 120 mg/mL, about 130 mg/mL, about 140 mg/mL or about 150 mg/mL). Even more preferably, it is about 0.5 mg/mL, about 1 mg/mL, about 10 mg/mL or about 100 mg/mL.

The anti-PD-1/CD3 bispecific antibody applicable to the aqueous pharmaceutical composition of the present invention (preferably for intravenous administration) of which the volume per formulation is, for example, 5 mL is at an arbitrary amount from about 0.05 to about 1000 mg (preferably about 0.5 to about 750 mg, more preferably about 0.5 to about 6 mg, about 25 to about 125 mg or about 400 to about 750 mg, even more preferably about 2.5 mg, about 5 mg, about 50 mg or about 500 mg), and on the other hand, the anti-PD-1/CD3 bispecific antibody applicable to the aqueous pharmaceutical composition (preferably for subcutaneous administration) of which the volume is, for example, 1 mL is at 1/5 amount of the above amount of the antibody applicable to 5 mL volume of the above aqueous pharmaceutical composition, and if the volumes are, for example, about 2 mL, about 0.8 mL, about 0.6 mL, about 0.5 mL, about 0.4 mL and about 0.2 mL, respectively, it is at about 2/5 amount, about 4/25 amount, about 3/25 amount, about 1/10 amount, about 2/25 amount and about 1/25 amount of the above amount of the antibody applicable to 5 mL volume of the above aqueous pharmaceutical composition, respectively.

Note that the term "pharmaceutical composition" as used in the present specification refers to a mixture of an active ingredient and one or more other additives, and the term "aqueous pharmaceutical composition" means one in which the pharmaceutical composition is substantially dissolved in an aqueous solvent. A preferable form of the pharmaceutical composition of the present invention is of an aqueous pharmaceutical composition.

The term "formulation" as used in the present specification refers to a drug in a form suitable for a route or method of administration appropriately selected for the pharmaceutical composition. The term "about" as used in the present specification means that the indicated numerical value may vary within a range of 10 % or less below or above, and the term "to" indicating a range means including each of the lower and upper limits thereof.

The aqueous pharmaceutical composition of the present invention contains a buffer to stabilize a pH thereof. As the buffer, anything is acceptable as long as it can sufficiently stabilize the pH at which the aqueous pharmaceutical composition of the present invention can take and does not affect the stabilization of the anti-PD-1/CD3 bispecific antibody, but it is preferably an acetate buffer. The concentration of an acetate buffer in the aqueous pharmaceutical composition of the present invention is an arbitrary concentration from about 7.3 to about 73.0 mM (e.g., about 7.3 mM, about 10 mM, about 14.6 mM, about 15 mM, about 18 mM, about 20 mM, about 20.07 mM, about 21 mM, about 22 mM, about 25 mM, about 30 mM, about 35 mM. about 40 mM, about 43.8 mM, about 45 mM, about 50 mM, about 55 mM, about 60 mM, about 70 mM and about 73.0 mM). preferably an arbitrary concentration from about 14.6 to about 43.8 mM, more preferably an arbitrary concentration from about 18 to about 22 mM, and even more preferably about 20 mM, and more specifically, about 20.07 mM. Note that in the present specification, for example, if the term "10 mM of an acetate buffer" or "10 mM of an acetate buffer solution" is used, it means that the aqueous pharmaceutical composition of the present invention contains the acetate buffer which is equivalent to 10 mM concentration.

Herein, such an acetate buffer can be prepared by the known methods of adding acetic acid to a sodium acetate solution (e.g., an aqueous solution of sodium acetate anhydrate. sodium acetate dihydrate or sodium acetate trihydrate) to achieve an arbitrary pH. For example, an acetate buffer applicable to the aqueous pharmaceutical composition of which the volume per formulation is 5 mL (preferably for intravenous administration) consists of (i) sodium acetate trihydrate equivalent to an arbitrary amount from about 3.4 to about 34 mg (preferably about 6.8 to about 20.4 mg, more preferably about 8.4 to about 10.2 mg), and (ii) glacial acetic acid, for example, equivalent to an arbitrary amount to make a pH of the acetate buffer solution about 4.9 to 5.1, corresponding to the above amount of sodium acetate trihydrate, and further in this case, a preferable embodiment of the acetate buffer consists of about 9.4 mg of sodium acetate trihydrate (specifically, about 9.35 mg) and about 1.9 mg of glacial acetic acid.

On the other hand, an acetate buffer applicable to the aqueous pharmaceutical composition of which the volume is, for example, about 1 mL (preferably for subcutaneous administration) consists of about 1/5 amounts of the above respective amounts of sodium acetate trihydrate and glacial acetic applicable to 5 mL volume of the above aqueous pharmaceutical composition, and if the volumes are, for example, about 2 mL, about 0.8 mL, about 0.6 mL, about 0.5 mL, about 0.4 mL and about 0.2 mL, respectively, it consists of about 2/5 amounts, about 4/25 amounts, about 3/25 amounts, about 1/10 amounts, about 2/25 amounts and about 1/25 amounts of the above respective amounts of sodium acetate trihydrate and glacial acetic acid applicable to 5 mL volume of the above aqueous pharmaceutical composition, respectively.

Buffers applicable to the aqueous pharmaceutical compositions of the present invention also include those with the pH buffering performance equivalent to that of the above acetate buffer.

The aqueous pharmaceutical composition of the present invention has an arbitrary pH from about 4.7 to about 5.3 (i.e. the range of about 5.0 ± 0.3) (e.g. about 4.7, about 4.8, about 4.9, about 5.0, about 5.1, about 5.2 or about 5.3). preferably an arbitrary pH from about 4.9 to about 5.1 (i.e. the range of about 5.0 ± 0.1), more preferably a pH of about 5.0.

The aqueous pharmaceutical composition of the present invention contains a sugar, mainly for the adjustment of osmotic pressure thereof. As such a sugar, anything is acceptable as long as it can sufficiently adjust the osmotic pressure of the aqueous pharmaceutical composition of the present invention and does not affect the stabilization of the anti-PD-1/CD3 bispecific antibody, but it is preferably trehalose, which may be added in the form of hydrate (e.g., monohydrate, preferably dihydrate) or anhydrate. The concentration of trehalose in the aqueous pharmaceutical composition of the present invention is an arbitrary concentration from about 5 to about 286.1 mM (e.g. about 5 mM, about 10 mM, about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 172 mM, about 180 mM, about 182 mM, about 182.3 mM, about 182.38 mM, about 182.4 mM, about 190 mM, about 193 mM, about 200 mM, about 207.9 mM, about 210 mM, about 220 mM, about 225 mM, about 230 mM, about 240 mM, about 250 mM, about 260 mM, about 270 mM, about 280 mM, about 285 mM, about 286 mM or about 286.1 mM), preferably an arbitrary concentration from about 10 to about 207.9 mM, more preferably an arbitrary concentration from about 172 to about 193 mM, even more preferably at about 182 mM, specifically about 182.4 mM and more specifically about 182.38 mM.

The sugar in the aqueous pharmaceutical composition of the present invention is trehalose, and trehalose applicable to the aqueous pharmaceutical composition of which the volume per formulation is, for example, 5 mL (preferably for intravenous administration) is at an arbitrary amount from about 9.46 to about 541.2 mg of dihydrate thereof (preferably about 18.9 to about 393.3 mg, more preferably about 325 to about 365.1 mg, even more preferably about 345 mg), and on the other hand, trehalose applicable to the aqueous pharmaceutical composition of which the volume is, for example, 1 mL (preferably for subcutaneous administration) is at 1/5 amount of the above amount of trehalose dihydrate applicable to 5 mL volume of the above aqueous pharmaceutical composition, and if the volumes are, for example, about 2 mL, about 0.8 mL, about 0.6 mL, about 0.5 mL, about 0.4 mL and about 0.2 mL, respectively, it is at about 2/5 amount, about 4/25 amount, about 3/25 amount, about 1/10 amount, about 2/25 amount and about 1/25 amount of the above amount of trehalose dihydrate applicable to 5 mL volume of the above aqueous pharmaceutical composition, respectively.

Sugars applicable to the aqueous pharmaceutical compositions of the present invention also include those with the osmotic pressure regulating capacity equivalent to that of the above trehalose.

The aqueous pharmaceutical composition of the present invention contains an antioxidant to inhibit the oxidation of the anti-PD-1/CD3 bispecific antibody or the resulting reduction in pharmacological activity. Sucg an antioxidant, anything is acceptable as long as it can suppress the oxidation of the anti-PD-1/CD3 bispecific antibody or the resulting reduction in pharmacological activity, to the desired extent, but it is preferably L-methionine or DTPA (Diethylene Triamine Pentaacetic Acid), more preferably L-methionine. If the antioxidant in the aqueous pharmaceutical composition of the present invention is L-methionine, it is at an arbitrary concentration from about 5 to about 30.2 mM (e.g. about 5 mM, about 8 mM, about 10 mM, about 10.05 mM, about 12 mM, about 15 mM. about 20 mM, about 25 mM, about 30 mM or about 30.2 mM), preferably at an arbitrary concentration from about 5 to about 20 mM, more preferably at an arbitrary concentration from about 5 to about 15 mM, even more preferably at an arbitrary concentration from about 8 to about 12 mM, most preferably at about 10 mM, and more specifically about at 10.05 mM.

The antioxidant in the aqueous pharmaceutical composition of the present invention is L-methionine, and L-methionine applicable to the aqueous pharmaceutical composition of which the volume per formulation is, for example, 5 mL (preferably for intravenous administration) is at an arbitrary amount from about 3.75 to about 22.5 mg (preferably about 3.75 to about 15.0 mg, more preferably about 3.75 to about 11.25 mg, even more preferably about 6 to about 9 mg, most preferably about 7.5 mg), and on the other hand, L-methionine applicable to the aqueous pharmaceutical composition of which the volume is, for example, about 1 mL (preferably for subcutaneous administration) is at 1/5 amount of the above amount of L-methionine applicable to 5 mL volume of the above aqueous pharmaceutical composition, and if the volumes are, for example, about 2 mL, about 0.8 mL, about 0.6 mL, about 0.5 mL, about 0.4 mL and about 0.2 mL, respectively, it is at about 2/5 amount, about 4/25 amount, about 3/25 amount, about 1/10 amount, about 2/25 amount and about 1/25 amount of the above amount of L-methionine applicable to 5 mL volume of the above aqueous pharmaceutical composition, respectively.

Antioxidants applicable to the aqueous pharmaceutical composition of the present invention also include those with the antioxidant capacity against the anti-PD-1/CD3 bispecific antibody, equivalent to the above L-methionine.

The aqueous pharmaceutical composition of the present invention contains a surfactant to mainly suppress the aggregation of the anti-PD-1/CD3 bispecific antibody. Such a surfactant, anything is acceptable as long as it can inhibit the aggregation of the anti-PD-1/CD3 bispecific antibody, but preferably polysorbate 80, and a concentration thereof is an arbitrary one from about 0.01 to about 0.5 % (w/v) (e.g., about 0.01 % (w/v), about 0.02 % ( w/v), about 0.04 % (w/v), about 0.05 % (w/v), about 0.06 % (w/v), about 0.08 % (w/v). about 0.1 % (w/v), about 0.12 % (w/v), about 0.15 % (w/v), about 0.2 % (w/v), about 0.25 % (w/v), about 0.3 % (w/v), about 0.35 % (w/v), about 0.4 % (w/v), about 0.45 % (w/v) or about 0.5 % (w/v)), preferably an arbitrary concentration from about 0.05 to about 0.3 % (w/v), more preferably an arbitrary concentration from about 0.8 to about 1.2 % (w/v), even more preferably about 0.1 % (w/v).

The surfactant in the aqueous pharmaceutical composition of the present invention is polysorbate 80, and polysorbate 80 applicable to the aqueous pharmaceutical composition of which the volume per formulation is, for example, 5 mL (preferably for intravenous administration) is at an arbitrary amount from about 0.5 to about 25 mg (preferably about 2.5 to about 15 mg, more preferably about 4 to about 6 mg, even more preferably about 5 mg), and on the other hand, polysorbate 80 applicable to the aqueous pharmaceutical composition of which the volume is, for example, 1 mL (preferably for subcutaneous administration) is at 1/5 amount of the above amount of polysorbate 80 applicable to 5 mL volume of the above aqueous pharmaceutical composition, and if the volumes are, for example, about 2 mL, about 0.8 mL, about 0.6 mL, about 0.5 mL, about 0.4 mL and about 0.2 mL, respectively, it is at about 2/5 amount, about 4/25 amount, about 3/25 amount, about 1/10 amount, about 2/25 amount and about 1/25 amount of the above respective amounts of polysorbate 80 applicable to 5 mL volume of the above aqueous pharmaceutical composition, respectively.

Surfactants applicable to the aqueous pharmaceutical compositions of the present invention also include those with the aggregation suppressive property of the anti-PD-1/CD3 bispecific antibody equivalent to that of the above polysorbate 80.

The aqueous pharmaceutical composition of the present invention contains a salt for stabilization (suppression of the aggregation). Such a salt, anything is acceptable as long as it enables such stabilization, but preferably sodium chloride, and a concentration thereof is an arbitrary one from about 0.5 to about 34.2 mM (e.g. about 0.5 mM, about 1 mM, about 2 mM, about 3 mM, about 4 mM, about 5 mM, about 6 mM, about 7 mM, about 8 mM, about 9 mM, about 10 mM. about 11 mM, about 12 mM, about 13 mM, about 14 mM, about 15 mM, about 16 mM, about 17 mM, about 18 mM, about 19 mM, about 19.6 mM, about 20 mM, about 21 mM, about 22 mM, about 23 mM, about 23.27 mM, about 24.0 mM, about 25 mM, about 25.7 mM, about 30 mM or about 34.2 mM), preferably an arbitrary concentration from about 1.0 to about 25.7 mM, more preferably an arbitrary concentration from about 19.6 to about 25.7 mM, even more preferably about 23 mM, more specifically about 23.27 mM.

Note that the aqueous pharmaceutical composition of the present invention is preferably one of which the conductivity is from about 2.6 to about 3.1 mS/cm (e.g. about 2.6 mS/cm, about 2.7 mS/cm, about 2.8 mS/cm, about 2.9 mS/cm, about 3.0 mS/cm or about 3.1 mS/cm), and more preferably about 2.8 mS/cm.

The salt in the aqueous pharmaceutical composition of the present invention is sodium chloride, and sodium chloride applicable to the aqueous pharmaceutical composition of which the volume per formulation is, for example, 5 mL (preferably for intravenous administration) is at an arbitrary amount from about 0.15 to about 10.0 mg (preferably about 0.29 to about 7.5 mg, more preferably about 5.8 to about 7.5 mg, even more preferably about 6.8 mg), and on the other hand, sodium chloride applicable to the aqueous pharmaceutical composition of which the volume is, for example, about 1 mL (preferably for subcutaneous administration) is at 1/5 amount of the above amount of sodium chloride applicable to 5 mL volume of the above aqueous pharmaceutical composition. and when the volumes are, for example, about 2 mL, about 0.8 mL, about 0.6 mL, about 0.5 mL, about 0.4 mL and about 0.2 mL, respectively, it is at about 2/5 amount, about 4/25 amount, about 3/25 amount, about 1/10 amount, about 2/25 amount and about 1/25 amount of the above amount of sodium chloride applicable to the above 5 mL volume of the above aqueous pharmaceutical composition, respectively.

Salts applicable to the aqueous pharmaceutical compositions of the present invention also include those with the stabilizing capacity equivalent to that of the above sodium chloride.

Note that in the present specification, the amount of the respective additives contained in the aqueous pharmaceutical composition of the present invention may be the amount of the above-mentioned respective additives corresponding to a unit volume (e.g., 1 mL), and one example of embodiment thereof contains 1.87 mg/mL of sodium acetate trihydrate, 0.38 mg/mL of acetic acid, 69 mg/mL of trehalose dihydrate, 1.5 mg/mL of L-methionine, 1.0 mg/mL of polysorbate 80 and 1.36 mg/mL of sodium chloride and the like.

Examples of embodiments of the aqueous pharmaceutical composition of the present invention include:
(A) a pharmaceutical composition containing:
   (a) about 0.1 to about 1.2 mg/mL or about 5 to about 25 mg/mL (preferably about 1 mg/mL or about 10 mg/mL, respectively) of the anti-PD-1/CD3 bispecific antibody (preferably Antibody α) as an active ingredient, and further containing:
   (b) a buffer (preferably about 7.3 to about 73.0 mM (preferably about 14.6 to about 43.8 mM, more preferably about 18 to about 22 mM, even more preferably about 20 mM, more specifically about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182 mM, more specifically about 182.38 mM) of trehalose,
   (d) about 5 to about 30.2 mM (preferably about 5 to about 20 mM, more preferably about 5 to about 15 mM, even more preferably about 10 mM, more specifically about 10.05 mM) of L-methionine,
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically about 23.27 mM) of sodium chloride, and of which a pH is about 4.7 to about 5.3 (preferably about 4.9 to about 5.1, more preferably about 5.0),
(B) a pharmaceutical composition containing:
   (a) about 0.1 to about 1.2 mg/mL or about 5 to about 25 mg/mL (preferably about 1 mg/mL or about 10 mg/mL, respectively) of the anti-PD-1/CD3 bispecific antibody (preferably Antibody α) as an active ingredient, and further containing:
   (b) about 7.3 to about 73.0 mM (preferably about 14.6 to about 43.8 mM, more preferably about 18 to about 22 mM, even more preferably about 20 mM. more specifically about 20.07 mM) of an acetate buffer,
   (c) about 5 to about 286.1 mM (preferably about 10 to about 207.9 mM, more preferably about 172 to about 193 mM, even more preferably about 182 mM, more specifically about 182.38 mM) of trehalose,
   (d) an antioxidant (preferably about 5 to about 30.2 mM (preferably about 5 to about 20 mM, more preferably about 5 to about 15 mM, even more preferably about 10 mM, more specifically about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically about 23.27 mM) of sodium chloride, and of which a pH is about 4.7 to about 5.3 (preferably about 4.9 to about 5.1, more preferably about 5.0), and
(C) a pharmaceutical composition containing:
   (a) about 0.1 to about 1.2 mg/mL or about 5 to about 25 mg/mL (preferably about 1 mg/mL or about 10 mg/mL, respectively) of the anti-PD-1/CD3 bispecific antibody (preferably Antibody α) as an active ingredient, and further containing:
   (b) a buffer (preferably about 7.3 to about 73.0 mM (preferably about 14.6 to about 43.8 mM, more preferably about 18 to about 22 mM, even more preferably about 20 mM, more specifically about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182 mM, more specifically about 182.38 mM) of trehalose,
   (d) an antioxidant (preferably about 5 to about 30.2 mM (preferably about 5 to about 20 mM, more preferably about 5 to about 15 mM, even more preferably about 10 mM, more specifically about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically about 23.27 mM) of sodium chloride, and of which a pH is about 4.7 to about 5.3 (preferably about 4.9 to about 5.1, more preferably about 5.0). Note that these aqueous pharmaceutical compositions in (A) to (C) can be used in both intravenous and subcutaneous administrations.

On the other hand, among the aqueous pharmaceutical compositions of the present invention, examples of embodiments for subcutaneous administration include:
(D) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) a buffer (preferably about 7.3 to about 73.0 mM (preferably about 14.6 to about 43.8 mM, more preferably about 18 to about 22 mM, even more preferably about 20 mM, more specifically about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182 mM, more specifically about 182.38 mM) of trehalose,
   (d) about 5 to about 30.2 mM (preferably about 5 to about 20 mM, more preferably about 5 to about 15 mM, even more preferably about 10 mM. more specifically about 10.05 mM) of L-methionine,
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically, about 23.27 mM) of sodium chloride, and of which a pH is about 4.7 to about 5.3 (preferably about 4.9 to about 5.1, more preferably about 5.0),
(E) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) about 7.3 to about 73.0 mM (preferably about 14.6 to about 43.8 mM, more preferably about 18 to about 22 mM, even more preferably about 20 mM. more specifically about 20.07 mM) of an acetate buffer,
   (c) about 5 to about 286.1 mM (preferably about 10 to about 207.9 mM, more preferably about 172 to about 193 mM, even more preferably about 182 mM, more specifically about 182.38 mM) of trehalose,
   (d) an antioxidant (preferably about 5 to about 30.2 mM (preferably about 5 to about 20 mM, more preferably about 5 to about 15 mM, even more preferably about 10 mM. more specifically about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically about 23.27 mM) of sodium chloride, and of which a pH is about 4.7 to about 5.3 (preferably about 4.9 to about 5.1, more preferably about 5.0), and
(F) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) a buffer (preferably about 7.3 to about 73.0 mM (preferably about 14.6 to about 43.8 mM, more preferably about 18 to about 22 mM, even more preferably about 20 mM, more specifically about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182 mM, more specifically about 182.38 mM) of trehalose,
   (d) an antioxidant (preferably about 5 to about 30.2 mM (preferably about 5 to about 20 mM, more preferably about 5 to about 15 mM, even more preferably about 10 mM, more specifically about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23 mM, more specifically about 23.27 mM) of sodium chloride, and of which a pH is about 4.7 to about 5.3 (preferably about 4.9 to about 5.1, more specifically about 5.0).

Furthermore, examples of alternative embodiments of the aqueous pharmaceutical compositions of the present invention include:
(A1) a pharmaceutical composition containing:
   (a) about 0.1 to about 1.2 mg/mL or about 5 to about 25 mg/mL (preferably about 0.5 mg/mL, about 1 mg/mL or about 10 mg/mL. respectively) of the anti-PD-1/CD3 bispecific antibody (preferably Antibody α) as an active ingredient, and further containing:
   (b) about 7.3 to about 73.0 mM of an acetate buffer,
   (c) about 5 to about 286.1 mM of trehalose,
   (d) about 5 to about 30.2 mM of L-methionine,
   (e) about 0.01 to about 0.5 % (w/v) of polysorbate 80, and
   (f) about 0.5 to about 34.2 mM of sodium chloride, and of which a pH is about 4.7 to about 5.3, preferably,
(B1) a pharmaceutical composition containing:
   (a) about 0.1 to about 1.2 mg/mL or about 5 to about 25 mg/mL (preferably about 0.5 mg/mL, about 1 mg/mL or about 10 mg/mL. respectively) of the anti-PD-1/CD3 bispecific antibody (preferably Antibody α) as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose,
   (d) about 5 to about 20 mM (preferably about 5 to about 15 mM) of L-methionine,
   (e) about 0.05 to about 0.3 % (w/v) (preferably about 0.08 to 0.12 % (w/v)) of polysorbate 80, and
   (f) about 1.0 to about 25.7 mM (preferably about 19.6 to about 25.7 mM) of sodium chloride, and of which a pH is about 4.9 to about 5.1, and more preferably,
*(C1)* a pharmaceutical composition containing:
   (a) about 0.5 mg/mL, about 1 mg/mL or about 10 mg/mL of the anti-PD-1/CD3 bispecific antibody as an active ingredient (preferably Antibody α), and further containing:
   (b) about 20.1 mM (specifically about 20.07 mM) of an acetate buffer,
   (c) about 182.4 mM (specifically about 182.38 mM) of trehalose,
   (d) about 10.1 mM (specifically about 10.05 mM) of L-methionine,
   (e) about 0.1 % (w/v) of polysorbate 80, and
   (f) about 23.3 mM (specifically about 23.27 mM) of sodium chloride, and of which a pH is about 5.0. Note that these aqueous pharmaceutical compositions in (A1) to (C1) also can be used in both intravenous and subcutaneous administrations.

On the other hand, among the aqueous pharmaceutical compositions of the present invention, examples of alternative embodiments of the aqueous pharmaceutical compositions for subcutaneous administration include:
(D1) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) about 7.3 to about 73.0 mM of an acetate buffer,
   (c) about 5 to about 286.1 mM of trehalose,
   (d) about 5 to about 30.2 mM of L-methionine,
   (e) about 0.01 to about 0.5 % (w/v) of polysorbate 80, and
   (f) about 0.5 to about 34.2 mM of sodium chloride, and of which a pH is about 4.7 to about 5.3, preferably,
(E1) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose,
   (d) about 5 to about 20 mM (preferably about 5 to about 15 mM) of L-methionine,
   (e) about 0.05 to about 0.3 % (w/v) (preferably about 0.08 to about 0.12 % (w/v)) of polysorbate 80, and
   (f) about 1.0 to about 25.7 mM (preferably about 19.6 to about 25.7 mM) of sodium chloride, and of which a pH is about 4.9 to about 5.1, and more preferably,
(F1) a pharmaceutical composition containing:
   (a) about 100 mg/mL of the anti-PD-1/CD3 bispecific antibody (preferably Antibody α) as an active ingredient, and further containing:
   (b) about 20.1 mM (specifically about 20.07 mM) of an acetate buffer,
   (c) about 182.4 mM (specifically about 182.38 mM) of trehalose
   (d) about 10.1 mM (specifically about 10.05 mM) of L-methionine,
   (e) about 0.1 % (w/v) of polysorbate 80, and
   (f) about 23.3 mM (specifically, about 23.27 mM) of sodium chloride, and of which a pH is about 5.0.

Furthermore, examples of other alternative embodiments of the aqueous pharmaceutical compositions of the present invention include, if the volume per formulation thereof is about 5 mL,
(A2) a pharmaceutical composition containing:
   (a) about 0.5 to about 6 mg or about 25 to about 125 mg (preferably about 2.5 mg, about 5 mg or about 50 mg, respectively) of the anti-PD-1/CD3 bispecific antibody (preferably Antibody α) as an active ingredient, and further containing:
   (b) an acetate buffer consisting of (b1) about 3.4 to about 34.0 mg of sodium acetate trihydrate, and (b2) the amount of glacial acetic acid that makes a pH about 4.7 to about 5.3, corresponding to the amount of sodium acetate trihydrate of the preceding item (b1),
   (c) about 9.46 to about 541.2 mg of trehalose dihydrate,
   (d) about 3.75 to about 22.5 mg of L-methionine,
   (e) about 0.5 to about 25.0 mg of polysorbate 80, and
   (f) about 0.15 to about 10.0 mg of sodium chloride, and of which the pH is about 4.7 to about 5.3, preferably,
(B2) a pharmaceutical composition containing:
   (a) about 0.5 to about 6 mg or about 25 to about 125 mg (preferably about 2.5 mg, about 5 mg or about 50 mg, respectively) of the anti-PD-1/CD3 bispecific antibody (preferably Antibody α) as an active ingredient, and further containing:
   (b) an acetate buffer consisting of (b1) about 6.8 to about 20.4 mg (preferably about 8.4 to about 10.2 mg) of sodium acetate trihydrate, and (b2) the amount of glacial acetic acid that makes a pH about 4.9 to about 5.1, corresponding to the amount of sodium acetate trihydrate of the proceding item (b1),
   (c) about 18.9 to about 393.3 mg (preferably about 325 to about 365.1 mg) of trehalose dihydrate,
   (d) about 3.75 to about 15.0 mg (preferably about 3.75 to 11.25 mg) of L-methionine,
   (e) about 2.5 to about 15.0 mg (preferably about 4 to 6 mg) of polysorbate 80, and
   (f) about 0.29 to about 7.5 mg (preferably about 5.8 to about 7.5 mg) of sodium chloride, and of which the pH is about 4.9 to about 5.1, and more preferably,
(C2) a pharmaceutical composition containing:
   (a) about 2.5 mg, about 5 mg or about 50 mg of the anti-PD-1/CD3 bispecific antibody (preferably Antibody α) as an active ingredient, and further containing:
   (b) an acetate buffer consisting of about 9.4 mg (specifically about 9.35 mg) of sodium acetate trihydrate and about 1.9 mg of glacial acetic acid,
   (c) about 345 mg of trehalose dihydrate,
   (d) about 7.5 mg of L-methionine,
   (e) about 5.0 mg of polysorbate 80, and
   (f) about 6.8 mg of sodium chloride, and of which a pH is about 5.0. Note that these aqueous pharmaceutical compositions in (A2) to (C2) also can be used in both intravenous and subcutaneous administrations.

Note that in the formulation of the aqueous pharmaceutical composition of the present invention, the volume per formulation can be selected according to a dosage per administration, e.g., in case of the pharmaceutical compositions in the preceding items (A2) to (C2). also can be selected from any of about 1 mL, about 2 mL, about 3 mL, about 4 mL, about 6 mL, about 8 mL, about 10 mL and about 20 mL volumes per formulation. In such a case, the amounts of the anti-PD-1/CD3 bispecific antibody and each additive are about 1/5 amounts, about 2/5 amounts, about 3/5 amounts, about 4/5 amounts, about 6/5 amounts, about 8/5 amounts, about twofold amounts and about fourfold amounts of the respective amounts when the volume per formulation, described in the preceding items (A2) to (C2) is about 5 mL.

Furthermore, examples of yet other alternative embodiments of the aqueous pharmaceutical composition of the present invention include,
(D2) a pharmaceutical composition containing:
   (a) about 0.1 to about 1.2 mg/mL or about 5 to about 25 mg/mL (preferably about 0.5 mg/mL, about 1 mg/mL or about 10 mg/mL. respectively) of the anti-PD-1/CD3 bispecific antibody (preferably Antibody α) as an active ingredient, and further containing:
   (b) an acetate buffer consisting of (b1) about 0.68 to about 6.8 mg/mL of sodium acetate trihydrate, and (b2) the amount of acetic acid that makes a pH about 4.7 to about 5.3, corresponding to the amount of sodium acetate trihydrate in the preceding item (b1),
   (c) about 1.9 to about 108.3 mg/mL of trehalose dihydrate,
   (d) about 0.75 to about 4.5 mg/mL of L-methionine,
   (e) about 0.1 to about 5.0 mg/mL of polysorbate 80, and
   (f) about 0.03 to about 2.0 mg/mL of sodium chloride, and of which the pH is about 4.7 to about 5.3, preferably,
(E2) a pharmaceutical composition containing:
   (a) about 0.1 to about 1.2 mg/mL or about 5 to about 25 mg/mL (preferably about 0.5 mg/mL, about 1 mg/mL or about 10 mg/mL. respectively) of the anti-PD-1/CD3 bispecific antibody (preferably Antibody α) as an active ingredient, and further containing:
   (b) an acetate buffer consisting of (b1) about 1.36 to about 4.08 mg/mL (preferably about 1.68 to about 2.05 mg/mL) of sodium acetate trihydrate, and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and of which the pH is about 4.9 to about 5.1, and more preferably,
(F2) a pharmaceutical composition containing:
   (a) about 0.5 mg/mL, about 1 mg/mL or about 10 mg/mL of the anti-PD-1/CD3 bispecific antibody (preferably Antibody α) as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and of which a pH is about 5.0. Note that these aqueous pharmaceutical compositions in (D2) to (F2) also can be used in both intravenous and subcutaneous administrations.

On the other hand, among the aqueous pharmaceutical compositions of the present invention, examples of other alternative embodiments of the aqueous pharmaceutical compositions for subcutaneous administration include, if the volume per formulation thereof is about 1 mL,
(A3) a pharmaceutical composition containing:
   (a) about 80 to about 150 mg (preferably about 100 mg) of the anti-PD-1/CD3 bispecific antibody (preferably Antibody α) as an active ingredient, and further containing:
   (b) an acetate buffer consisting of (b1) about 0.68 to about 6.8 mg of sodium acetate trihydrate, and (b2) the amount of glacial acetic acid that makes a pH about 4.7 to about 5.3, corresponding to the amount of sodium acetate trihydrate of the preceding item (b1),
   (c) about 1.9 to about 108.3 mg of trehalose dihydrate,
   (d) about 0.75 to about 4.5 mg of L-methionine,
   (e) about 0.1 to about 5.0 mg of polysorbate 80, and
   (f) about 0.03 to about 2.0 mg of sodium chloride, and of which the pH is about 4.7 to about 5.3, preferably,
(B3) a pharmaceutical composition containing:
   (a) about 80 to about 150 mg (preferably about 100 mg) of the anti-PD-1/CD3 bispecific antibody (preferably Antibody α) as an active ingredient, and further containing:
   (b) an acetate buffer consisting of (b1) about 1.36 to about 4.08 mg (preferably about 1.68 to about 2.05 mg) of sodium acetate trihydrate, and (b2) the amount of glacial acetic acid that makes a pH about 4.9 to about 5.1, corresponding to the amount of sodium acetate trihydrate of the preceding item (b1),
   (c) about 3.8 to about 78.7 mg (preferably about 65.1 to about 72.6 mg) of trehalose dihydrate,
   (d) about 0.75 to about 3.0 mg (preferably about 0.75 to about 2.25 mg) of L-methionine,
   (e) about 0.5 to about 3.0 mg of polysorbate 80, and
   (f) about 0.06 to about 1.5 mg (preferably about 1.15 to about 1.5 mg) of sodium chloride, and of which the pH is about 4.9 to about 5.1, and more preferably,
(C3) a pharmaceutical composition containing:
   (a) about 100 mg of the anti-PD-1/CD3 bispecific antibody (preferably Antibody α) as an active ingredient, and further containing:
   (b) an acetate buffer consisting of about 1.9 mg (specifically about 1.87 mg) of sodium acetate trihydrate and about 0.4 mg (specifically about 0.38 mg) of glacial acetic acid,
   (c) about 69 mg of trehalose dihydrate,
   (d) about 1.5 mg of L-methionine,
   (e) about 1.0 mg of polysorbate 80, and
   (f) about 1.4 mg (specifically about 1.36 mg) of sodium chloride, and of which a pH is about 5.0.

If the formulation of the aqueous pharmaceutical composition of the present invention comprises the pharmaceutical compositions in the preceding items (A3) to (C3), the volume per formulation thereof can be selected from any of, for example, about 0.1 mL, about 0.2 mL, about 0.4 mL, about 0.5 mL, about 0.6 mL, about 0.8 mL and about 1.2 mL volumes. In such a case, the amounts of the anti-PD-1/CD3 bispecific antibody and each additive is about 1/10 amounts, about 1/5 amounts, about 2/5 amounts, about 3/5 amounts, about 4/5 amounts and about 6/5 amounts of the respective amounts when the volume per formulation, described in the preceding items (A3) to (C3) is about 1 mL.

Furthermore, among the aqueous pharmaceutical compositions of the present invention, examples of yet other alternative embodiments of the aqueous pharmaceutical composition for subcutaneous administration include,
(D3) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) an acetate buffer consisting of (bl) about 0.68 to about 6.8 mg/mL of sodium acetate trihydrate, and (b2) the amount of acetic acid that makes a pH about 4.7 to about 5.3, corresponding to the amount of sodium acetate trihydrate in the preceding item (b1),
   (c) about 1.9 to about 108.3 mg/mL of trehalose dihydrate,
   (d) about 0.75 to about 4.5 mg/mL of L-methionine,
   (e) about 0.1 to about 5.0 mg/mL of polysorbate 80, and
   (f) about 0.03 to about 2.0 mg/mL of sodium chloride, and of which the pH is about 4.7 to about 5.3, preferably,
(E3) a pharmaceutical composition containing:
   (a) about 80 to about 150 mg/mL (preferably about 100 mg/mL) of the anti-PD-1/CD3 bispecific antibody (preferably Antibody α) as an active ingredient, and further containing:
   (b) an acetate buffer consisting of (b1) about 1.36 to about 4.08 mg/mL (preferably about 1.68 to about 2.05 mg/mL) of sodium acetate trihydrate, and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and of which the pH is about 4.9 to about 5.1, and more preferably,
(F3) a pharmaceutical composition containing:
   (a) about 100 mg/mL of the anti-PD-1/CD3 bispecific antibody (preferably Antibody α) as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and of which a pH is about 5.0.

The aqueous pharmaceutical compositions of the present invention also include aqueous pharmaceutical compositions for intravenous or subcutaneous administration equivalent to any of the pharmaceutical compositions listed in the above-mentioned (A) to (F), (A1) to (F1), (A2) to (F2) and (A3) to (F3) in the bioavailability in bioequivalence studies and containing the anti-PD-1/CD3 bispecificity antibody of the present invention (preferably Antibody α) as an active ingredient. The pharmaceutical compositions include those containing the same additives as those in any of the pharmaceutical compositions listed in the above-mentioned (A) to (F), (A1) to (F1), (A2) to (F2) and (A3) to (F3), but also include those with different composition ratio and those of which any one or more of additives are different. Examples include (i) a pharmaceutical composition containing at least trehalose, L-methionine, polysorbate 80 and sodium chloride, (ii) a pharmaceutical composition containing at least an acetate buffer, trehalose, polysorbate 80 and sodium chloride, and (iii) a pharmaceutical composition containing at least trehalose, polysorbate 80 and sodium chloride.

Herein, the bioequivalence can be determined, for example, by the bioequivalence study described in the "Guidelines for Bioequivalence Studies of Generic Drugs" or subsequent revisions thereof, as stipulated by the Ministry of Health, Labour and Welfare, in the Notice No. 1124005 dated November 24, 2006 from the Pharmaceutical and Food Safety Bureau. Specifically, in the guideline, in principle, the crossover method is used, and when blood is used as a sample, AUCt (AUC up to the final sampling time t (area under the blood concentration-time curve)) and Cmax (maximum blood concentration) in the singledose study are used as determination parameters for the bioequivalence.

In accordance with the guideline, the study method is selected as appropriate, and then the above bioequivalence parameters of a formulation to be tested about the bioequivalence and a standard formulation are statistically processed, and when it is within a predetermined range, the test formulation can be determined to be bioequivalent to the standard formulation. For example, according to the guideline, when AUC and Cmax are log-normally distributed, the acceptable range of bioequivalence is from 0.80 to 1.25 when represented as the ratio of the population means of the parameters of the test formulation and the standard formulation, and when the 90 % confidence interval of the difference between the means of the logarithmic values of the determination parameters for the bioequivalence of the test formulation and the standard formulation is in the range of log (0.80) to log (1.25), the test formulation and the standard formulation are judged to be bioequivalent. Even if it does not meet the above criteria, when the difference between the means of the logarithmic values of the determination parameters for the bioequivalence of the test formulation and the standard formulation is in the range of log (0.90) to log (1.11), and when the dissolution behavior is judged to be similar in a dissolution study conducted in accordance with Section 3, item A, V, of the guideline, they are judged to be bioequivalent. However, this provision applies only when a total of 20 or more subjects (10 subjects per group) are used. Note that in the present invention, the formulations comprising the pharmaceutical compositions listed in the above-mentioned (A) to (F), (A1) to (F1), (A2) to (F2), and (A3) to (F3), respectively, correspond to the "standard formulation".

It is preferable that the aqueous pharmaceutical composition of the present invention is aseptically treated prior to production. When an aseptic manipulation method is employed as an aseptic treatment, for example, it can be manufactured by diluting or dissolving the weighed antibody and each additive pertaining to the present invention in water for injection (e.g., sterile purified water and distilled water for injection), and then filter-sterilizing the resulting dissolved solution. The pharmaceutical compositions also include those prepared by reconstituting lyophilized products comprising the antibody and each additive pertaining to the present invention with water for injection.

The formulation of the present invention can take a form suitable for intravenous and subcutaneous administrations, respectively. For example, for intravenous administration, it can be sealed in a vial or ampoule, of which the size can be selected according to the total volume of the aqueous pharmaceutical composition. Note that even when sealed in a vial or ampoule, it can be collected in a syringe at medical sites and then administered subcutaneously.

If the formulation of the present invention takes a form of formulation for subcutaneous administration, it can be provided as a pre-filled syringe formulation in which the pharmaceutical composition is pre-filled into a cleaned and sterilized syringe. Furthermore, the pharmaceutical composition of the present invention can also be provided in the form of which it is filled and sealed in a cleaned and sterilized cartridge and which is further filled in an injector pen, auto-injector or needle-free device (e.g., MediJector and BioJector (registered trademark)).

### [Pharmaceutical Uses of the Formulation of the Present Invention.]

The aqueous pharmaceutical composition or formulation thereof of the present invention pertaining to the anti-PD-1/CD3 bispecific antibody is useful for the prevention, suppression of symptom progression, suppression of reoccurrence and/or treatment of autoimmune diseases or graft-versus-host disease (GVHD), or hematological cancers.

Examples of autoimmune diseases which can be prevented, of which the symptom progression or recurrence can be suppressed, and/or which can be treated, by the aqueous pharmaceutical composition or formulation thereof of the present invention, include Behcet's disease, systemic lupus erythematosus, chronic discoid lupus erythematosus, multiple sclerosis (systemic scleroderma and progressive systemic sclerosis), scleroderma, polymyositis, dermatomyositis, periarteritis nodosa (polyarteritis nodosa and microscopic polyangiitis), aortitis syndrome (Takayasu's arteritis), malignant rheumatoid arthritis, rheumatoid arthritis, juvenile idiopathic arthritis, spondyloarthritis, mixed connective tissue disease, Sjogren's syndrome, adult Still's disease, vasculitis, allergic granulomatous vasculitis, hypersensitivity vasculitis, rheumatoid vasculitis, large vessel vasculitis. ANCA associated vasculitis (e.g., granulomatosis with polyangiitis and eosinophilic granulomatosis with polyangiitis), Cogan's syndrome, RS3PE syndrome, temporal arteritis, polymyalgia rheumatica, fibromyalgia, antiphospholipid antibody syndrome, eosinophilic fasciitis, IgG4-related disease (e.g., primary sclerosing cholangitis and autoimmune insulitis, etc.), Guillain-Barre syndrome, myasthenia gravis, chronic atrophic gastritis, autoimmune hepatitis, nonalcoholic steatohepatitis, primary biliary cirrhosis. Goodpasture's syndrome, rapidly progressive glomerulonephritis, megaloblastic anemia, autoimmune hemolytic anemia, pernicious anemia, autoimmune neutropenia, idiopathic thrombocytopenic purpura, Basedow disease (Graves' disease (hyperthyroidism)), Hashimoto disease, autoimmune adrenal insufficiency, primary hypothyroidism, Addison's disease (chronic hypoadrenocorticism), idiopathic Addison's disease, type I diabetes mellitus, slowly progressive type I diabetes mellitus (latent autoimmune diabetes in adult), focal scleroderma, psoriasis, psoriatic arthritis, bullous pemphigoid, pemphigus, pemphigoid, gestational herpes, linear IgA bullous dermatosis, acquired epidermolysis bullosa, alopecia areata, vitiligo, vitiligo vulgaris, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, habitual abortion, inflammatory bowel disease (e.g., ulcerous colitis and Crohn's disease), celiac disease, ankylosing spondylitis, severe asthma, chronic urticarial, transplantation immunity, familial mediterranean fever, eosinophilic chronic rhinosinusitis, dilated cardiomyopathy, systemic mastocytosis and inclusion body myositis and the like.

Particularly, preferable autoimmune diseases which can be prevented, of which the symptom progression or recurrence can be suppressed, and/or which can be treated, by the aqueous pharmaceutical composition or formulation thereof of the present invention include psoriasis, psoriatic arthritis, rheumatoid arthritis, Crohn's disease, ulcerous colitis and myasthenia gravis and the like.

In addition, examples of hematological cancers which can be prevented, of which the symptom progression or recurrence can be suppressed, and/or which can be treated, by the aqueous pharmaceutical composition or formulation thereof of the present invention include multiple myeloma, malignant lymphoma (e.g., non-Hodgkin's Lymphoma (e.g., B-cell non-Hodgkin's lymphoma and T/NK-cell non-Hodgkin's lymphoma (e.g., precursor T-cell lymphoblastic lymphoma, chronic T-cell lymphocytic leukemia, T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, cutaneous T-cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with TFH phenotype, anaplastic large cell lymphoma, ALK positive, anaplastic large cell lymphoma, ALK negative and breast implant-associated anaplastic large-cell lymphoma)), and Hodgkin's Lymphoma), leukemia (e.g., acute myelogenous leukemia, acute promyelocytic leukemia, acute lymphoblastic leukemia, chronic lymphocytic leukemia, myelodysplastic syndrome and chronic myelogenous leukemia), primary central nervous system lymphoma and myeloproliferative syndrome and the like.

Particulaly, preferable hematological cancers which can be prevented, of which the symptom progression or recurrence can be suppressed, and/or which can be treated, by the aqueous pharmaceutical composition or formulation thereof of the present invention includes peripheral T-cell lymphoma and cutaneous T-cell lymphoma and the like.

Herein, in the present invention, the term "treatment" means cure or improvement of certain disease or symptom thereof, the term "prevention" means that the onset of certain disease or symptom thereof is prevented or delayed for a certain period of time, and the term "suppression of the symptom progression" means that the progress or aggravation of symptom is suppressed to stop the progress of disease conditions. Note that the meaning of "prevention" also includes suppressing the recurrence. The term "suppression of the recurrence" means that the recurrence of certain disease or syndrome thereof is prevented or that a possibility of recurrence is reduced.

The present invention provides examples of embodiments listed in the following [1] to [95], [1-1] to [1-3] and [2-1] to [2-3], respectively.
[1] An aqueous pharmaceutical composition or a formulation thereof for subcutaneous or intravenous administration containing an antibody (preferably an anti-PD-1/CD3 bispecific antibody) as an active ingredient, and further containing a buffer (preferably an acetate buffer), trehalose, an antioxidant (preferably L-methionine), polysorbate 80 and sodium chloride;
[2] the aqueous pharmaceutical composition or formulation thereof according to the preceding item [1], wherein the anti-PD-1/CD3 bispecific antibody is at about 0.01 to about 200 mg/mL (preferably about 0.1 to about 150 mg/mL (preferably about 0.1 to about 1.2 mg/mL, about 5 to about 25 mg/mL or about 80 to about 150 mg/mL));
[3] the aqueous pharmaceutical composition or formulation thereof according to the preceding item [1], wherein the anti-PD-1/CD3 bispecific antibody is at about 0.01 mg/mL, about 0.02 mg/mL, about 0.03 mg/mL, about 0.04 mg/mL, about 0.05 mg/mL, about 0.06 mg/mL, about 0.07 mg/mL, about 0.08 mg/mL, about 0.09 mg/mL, about 0.1 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, about 1 mg/mL, about 1.1 mg/mL. about 1.2 mg/mL, about 1.3 mg/mL, about 1.4 mg/mL, about 1.5 mg/mL, about 2 mg/mL, about 5 mg/mL, about 10 mg/mL, about 15 mg/mL, about 20 mg/mL, about 25 mg/mL, about 30 mg/mL, about 40 mg/mL, about 50 mg/mL, about 60 mg/mL. about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL or about 200 mg/mL;
[4] the aqueous pharmaceutical composition or formulation thereof according to the preceding item [1], wherein the anti-PD-1/CD3 bispecific antibody is at about 0.5 mg/mL, about 1 mg/mL, about 10 mg/mL or about 100 mg/mL;
[5] the aqueous pharmaceutical composition or formulation thereof according to any one of the preceding items [1] to [4], wherein the acetate buffer is at about 7.3 to about 73.0 mM (preferably about 14.6 to about 43.8 mM, more preferably about 18 to about 22 mM);
[6] the aqueous pharmaceutical composition or formulation thereof according to any one of the preceding items [1] to [4], wherein the acetate buffer is at about 7.3 mM, about 10 mM, about 14.6 mM, about 15 mM, about 18 mM, about 20 mM, about 20.07 mM, about 21 mM, about 22 mM, about 25 mM, about 30 mM, about 35 mM, about 40 mM, about 43.8 mM, about 45 mM, about 50 mM, about 55 mM, about 60 mM, about 70 mM or about 73.0 mM;
[7] the aqueous pharmaceutical composition or formulation thereof according to any one of the preceding items [1] to [4], wherein the acetate buffer is at about 20.07 mM;
[8] the aqueous pharmaceutical composition or formulation thereof according to any one of the preceding items [5] to [7], wherein the acetate buffer consists of a combination of sodium acetate trihydrate and acetic acid;
[9] the aqueous pharmaceutical composition or formulation thereof according to any one of the preceding items [1] to [8], wherein trehalose is at about 5 to about 286.1 mM (preferably about 10 to about 207.9 mM, more preferably about 172 to about 193 mM);
[10] the aqueous pharmaceutical composition or formulation thereof according to any one of the preceding items [1] to [8], wherein trehalose is at about 5 mM, about 10 mM. about 20 mM, about 30 mM, about 40 mM, about 50 mM, about 60 mM, about 70 mM, about 80 mM, about 90 mM, about 100 mM, about 110 mM, about 120 mM, about 130 mM, about 140 mM, about 150 mM, about 160 mM, about 170 mM, about 172 mM, about 180 mM, about 182 mM, about 182.3 mM, about 182.38 mM, about 182.4 mM, about 190 mM, about 193 mM, about 200 mM, about 207.9 mM, about 210 mM, about 220 mM, about 225 mM, about 230 mM, about 240 mM, about 250 mM, about 260 mM, about 270 mM, about 280 mM, about 285 mM, about 286 mM or about 286.1 mM;
[11] the aqueous pharmaceutical composition or formulation thereof according to any one of the preceding items [1] to [8], wherein trehalose is at about 182.38 mM;
[12] the aqueous pharmaceutical composition or formulation thereof according to any one of the preceding items [1] to [8], wherein trehalose is trehalose dihydrate;
[13] the aqueous pharmaceutical composition or formulation thereof according to any one of the preceding items [1] to [12], wherein L-methionine is at about 5 to about 30.2 mM (preferably about 5 to about 20 mM, more preferably about 5 to about 15 mM);
[14] the aqueous pharmaceutical composition or formulation thereof according to any one of the preceding items [1] to [12], wherein L-methionine is at about 5 mM, about 10 mM, about 10.05 mM, about 15 mM, about 20 mM, about 25 mM, about 30 mM or about 30.2 mM;
[15] the aqueous pharmaceutical composition or formulation thereof according to any one of the preceding items [1] to [12], wherein L-methionine is at about 10.05 mM;
[16] the aqueous pharmaceutical composition or formulation thereof according to any one of the preceding items [1] to [15], wherein polysorbate 80 is at about 0.01 to about 0.5 % (w/v) (preferably about 0.05 to about 0.3 % (w/v), more preferably about 0.08 to about 0.12 % (w/v));
[17] the aqueous pharmaceutical composition or formulation thereof according to any one of the preceding items [1] to [15], wherein polysorbate 80 is at about 0.01 % (w/v), about 0.02 % (w/v), about 0.0 4% (w/v), about 0.05 % (w/v), about 0.06 % (w/v), about 0.08 % (w/v), about 0.1 % (w/v), about 0.12 % (w/v), about 0.15 % (w/v), about 0.2 % (w/v), about 0.25 % (w/v), about 0.3 % (w/v), about 0.35 % (w/v), about 0.4 % (w/v), about 0.45 % (w/v) or about 0.5 % (w/v);
[18] the aqueous pharmaceutical composition or formulation thereof according to any one of the preceding items [1] to [15], wherein polysorbate 80 is at about 0.1 % (w/v);
[19] the aqueous pharmaceutical composition or formulation thereof according to any one of the preceding items [1] to [18], wherein sodium chloride is at about 0.5 to about 34.2 mM (preferably about 1.0 to about 25.7 mM, more preferably about 19.6 to about 25.7 mM);
[20] the aqueous pharmaceutical composition or formulation thereof according to any one of the preceding items [1] to [18], wherein sodium chloride is at about 0.5 mM, about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 19.6 mM, about 20 mM, about 21 mM, about 22 mM, about 23 mM, about 23.27 mM, about 24.0 mM, about 25 mM, about 25.7 mM, about 30 mM or about 34.2 mM;
[21] the aqueous pharmaceutical composition or formulation thereof according to any one of the preceding items [1] to [18], wherein sodium chloride is at about 23.27 mM;
[22] the aqueous pharmaceutical composition or formulation thereof according to any one of the preceding items [1] to [21], wherein a volume per formulation of the aqueous pharmaceutical composition (preferably for intravenous administration) is about 5 mL;
[23] the aqueous pharmaceutical composition or formulation thereof according to the preceding item [22], wherein the acetate buffer applicable to the aqueous pharmaceutical composition of the preceding item [22] consists of (i) about 3.4 to about 34 mg of sodium acetate trihydrate and (ii) the amount of glacial acetic acid that makes a pH about 4.7 to about 5.3 (preferably about 4.9 to about 5.1, more preferably about 5.0), corresponding to the amount of sodium acetate trihydrate of the preceding item (i);
[24] the aqueous pharmaceutical composition or formulation thereof according to the preceding item [22], wherein the acetate buffer applicable to the aqueous pharmaceutical composition of the preceding item [22] consists of (i) about 6.8 to about 20.4 mg (preferably about 8.4 to about 10.2 mg) of sodium acetate trihydrate and (ii) the amount of glacial acetic acid that makes the pH about 4.7 to about 5.3 (preferably about 4.9 to about 5.1, more preferably about 5.0), corresponding to the amount of sodium acetate trihydrate of the preceding item (i);
[25] the aqueous pharmaceutical composition or formulation thereof according to the preceding item [22], wherein the acetate buffer applicable to the aqueous pharmaceutical composition of the preceding item [22] consists of about 9.4 mg (specifically about 9.35 mg) of sodium acetate trihydrate and about 1.9 mg of glacial acetic acid;
[26] the aqueous pharmaceutical composition or formulation thereof according to any one of the preceding items [22] to [25], wherein trehalose is trehalose dihydrate;
[27] the aqueous pharmaceutical composition or formulation thereof according to the preceding item [26], wherein trehalose dihydrate is at about 9.46 to about 541.2 mg (preferably about 18.9 to about 393.3 mg, more preferably about 325 to about 365.1 mg, even more preferably about 345 mg);
[28] the aqueous pharmaceutical composition or formulation thereof according to any one of the preceding items [22] to [27], wherein the antioxidant is L-methionine;
[29] the aqueous pharmaceutical composition or formulation thereof according to the preceding item [28], wherein L-methionine is at about 3.75 to about 22.5 mg (preferably about 3.75 to about 15.0 mg, more preferably about 3.75 to about 11.25 mg, even more preferably about 7.5 mg);
[30] the aqueous pharmaceutical composition or formulation thereof according to any one of the preceding items [22] to [29], wherein polysorbate 80 is at about 0.5 to about 25 mg (preferably about 2.5 to about 15 mg, more preferably about 5 mg);
[31] the aqueous pharmaceutical composition or formulation thereof according to any one of the preceding items [22] to [30], wherein sodium chloride is at about 0.15 to about 10.0 mg (preferably about 0.29 to about 7.5 mg, more preferably about 5.8 to about 7.5 mg, even more preferably about 6.8 mg);
[32] the aqueous pharmaceutical composition or formulation thereof according to any one of the preceding items [1] to [21], wherein a volume per formulation of the aqueous pharmaceutical composition is about 2 mL, and the amounts of the respective additives are about 2/5 amounts of the amounts of the respective additives of any one or more of the preceding items [23] to [31];
[33] the aqueous pharmaceutical composition or formulation thereof according to any one of the preceding items [1] to [21], wherein a volume per formulation of the aqueous pharmaceutical composition (preferably for subcutaneous administration) is about 1 mL;
[34] the aqueous pharmaceutical composition or formulation thereof according to the preceding item [33], wherein the acetate buffer applicable to the aqueous pharmaceutical composition of the preceding item [33] consists of (i) about 0.68 to about 6.8 mg of sodium acetate trihydrate and (ii) the amount of glacial acetic acid that makes the pH about 4.7 to about 5.3 (preferably about 4.9 to about 5.1, more preferably about 5.0), corresponding to the amount of sodium acetate trihydrate of the preceding item (i);
[35] the aqueous pharmaceutical composition or formulation thereof according to the preceding item [33], wherein the acetate buffer applicable to the aqueous pharmaceutical composition of the preceding item [33] consists of (i) about 1.36 to about 4.08 mg (preferably about 1.68 to about 2.05 mg) of sodium acetate trihydrate and (ii) the amount of glacial acetic acid that makes the pH about 4.7 to about 5.3 (preferably about 4.9 to about 5.1, more preferably about 5.0), corresponding to the amount of sodium acetate trihydrate of the preceding item (i);
[36] the aqueous pharmaceutical composition or formulation thereof according to the preceding item [33], wherein the acetate buffer applicable to the aqueous pharmaceutical composition of the preceding item [33] consists of about 1.9 mg (specifically about 1.87 mg) of sodium acetate trihydrate and about 0.4 mg (specifically about 0.38 mg) of glacial acetic acid;
[37] the aqueous pharmaceutical composition or formulation thereof according to any one of the preceding items [33] to [36], wherein trehalose is trehalose dihydrate;
[38] the aqueous pharmaceutical composition or formulation thereof according to the preceding item [37], wherein trehalose dihydrate is at about 1.9 to about 108.3 mg (preferably about 3.8 to about 78.7 mg, more preferably about 65.1 to about 72.6 mg, even more preferably about 69 mg);
[39] the aqueous pharmaceutical composition or formulation thereof according to any one of the preceding items [33] to [38], wherein the antioxidant is L-methionine;
[40] the aqueous pharmaceutical composition or formulation thereof according to the preceding item [39], wherein L-methionine is at about 0.75 to about 4.5 mg (preferably about 0.75 to about 3.0 mg, more preferably about 0.75 to about 2.25 mg, even more preferably about 1.5 mg);
[41] the aqueous pharmaceutical composition or formulation thereof according to any one of the preceding items [33] to [40], wherein polysorbate 80 is at about 0.1 to about 5.0 mg (preferably about 0.5 to about 3.0 mg, more preferably about 1.0 mg);
[42] the aqueous pharmaceutical composition or formulation thereof according to any one of the preceding items [33] to [41], wherein sodium chloride is at about 0.03 to about 2.0 mg (preferably about 0.06 to about 1.5 mg, more preferably about 1.15 to about 1.5 mg, even more preferably about 1.4 mg (specifically about 1.36 mg));
[43] the aqueous pharmaceutical composition or formulation thereof according to any one of the preceding items [1] to [42], wherein the pH of the aqueous pharmaceutical composition is about 4.7 to about 5.3 (preferably about 4.9 to about 5.1. more preferably about 5.0);
[44] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) about 7.3 to 73.0 mM of an acetate buffer,
   (c) about 5 to about 286.1 mM of trehalose,
   (d) about 5 to about 30.2 mM of L-methionine,
   (e) about 0.01 to about 0.5 % (w/v) of polysorbate 80, and
   (f) about 0.5 to about 34.2 mM of sodium chloride, and
   of which a pH is about 4.7 to about 5.3;
[45] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose
   (d) about 5 to about 20 mM (preferably about 5 to about 15 mM) of L-methionine
   (e) about 0.05 to about 0.3 % (w/v) (preferably about 0.08 to about 0.12 % (w/v)) of polysorbate 80, and
   (f) about 1.0 to about 25.7 mM (preferably about 19.6 to about 25.7 mM) of sodium chloride, and
   of which a pH is about 4.9 to about 5.1;
[46] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) about 20.07 mM of an acetate buffer,
   (c) about 182.38 mM of trehalose,
   (d) about 10.05 mM of L-methionine,
   (e) about 0.1 % (w/v) of polysorbate 80, and
   (f) about 23.27 mM of sodium chloride, and
   of which a pH is about 5.0;
[47] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) about 7.3 to about 73.0 mM of an acetate buffer,
   (c) about 5 to about 286.1 mM of trehalose,
   (d) about 5 to about 30.2 mM of L-methionine,
   (e) about 0.01 to about 0.5 % (w/v) of polysorbate 80, and
   (f) about 0.5 to about 34.2 mM of sodium chloride, and
   of which a pH is about 4.7 to about 5.3;
[48] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose,
   (d) about 5 to about 20 mM (preferably about 5 to about 15 mM) of L-methionine,
   (e) about 0.05 to about 0.3 % (w/v) (preferably about 0.08 to about 0.12 % (w/v)) of polysorbate 80, and
   (f) about 1.0 to about 25.7 mM (preferably about 19.6 to about 25.7 mM) of sodium chloride, and
   of which a pH is about 4.9 to about 5.1;
[49] an aqueous pharmaceutical composition or a formulation thereof for subcutaneous administration, containing:
   (a) about 100 mg/mL of an anti-PD-1/CD3 bispecific antibody as an active ingredient, and further containing:
   (b) about 20.07 mM of an acetate buffer,
   (c) about 182.38 mM of trehalose,
   (d) about 10.05 mM of L-methionine,
   (e) about 0.1 % (w/v) of polysorbate 80, and
   (f) about 23.27 mM of sodium chloride, and
   of which a pH is about 5.0;
[50] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) an acetate buffer consisting of (b1) about 0.68 to about 6.8 mg/mL of sodium acetate trihydrate and (b2) the amount of acetic acid that makes a pH about 4.7 to about 5.3, corresponding to the amount of sodium acetate trihydrate of the preceding item (b1),
   (c) about 1.9 to about 108.3 mg/mL of trehalose dihydrate,
   (d) about 0.75 to about 4.5 mg/mL of L-methionine
   (e) about 0.1 to about 5.0 mg/mL of polysorbate 80, and
   (f) about 0.03 to about 2.0 mg/mL of sodium chloride, and
   of which the pH is about 4.7 to about 5.3;
[51] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) an acetate buffer consisting of (b1) about 1.36 to about 4.08 mg/mL (preferably about 1.68 to about 2.05 mg/mL) of sodium acetate trihydrate and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and
   of which the pH is about 4.9 to about 5.1;
[52] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and
   of which a pH is about 5.0;
[53] an aqueous pharmaceutical composition or a formulation thereof for subcutaneous or intravenous administration, containing:
   (a) about 0.5 to about 6 mg or about 25 to about 125 mg (preferably about 2.5 mg, about 5 mg or about 50 mg, respectively) of an anti-PD-1/CD3 bispecific antibody as an active ingredient, and further containing:
   (b) an acetate buffer consisting of (b1) about 3.4 to about 34.0 mg of sodium acetate trihydrate and (b2) the amount of glacial acetic acid that makes a pH about 4.7 to about 5.3, corresponding to the amount of sodium acetate trihydrate of the preceding item (b1),
   (c) about 9.46 to about 541.2 mg of trehalose dihydrate,
   (d) about 3.75 to about 22.5 mg of L-methionine,
   (e) about 0.5 to about 25.0 mg of polysorbate 80, and
   (f) about 0.15 to about 10.0 mg of sodium chloride, and
   of which the pH is about 4.7 to about 5.3, and a volume per formulation is about 5 mL;
[54] an aqueous pharmaceutical composition or a formulation thereof for subcutaneous or intravenous administration, containing:
   (a) about 0.5 to about 6 mg or about 25 to about 125 mg (preferably about 2.5 mg, about 5 mg or about 50 mg, respectively) of an anti-PD-1/CD3 bispecific antibody as an active ingredient, and further containing:
   (b) an acetate buffer consisting of (b1) about 6.8 to about 20.4 mg (preferably about 8.4 to about 10.2 mg) of sodium acetate trihydrate and (b2) the amount of glacial acetic acid that makes a pH about 4.9 to about 5.1, corresponding to the amount of sodium acetate trihydrate of the preceding item (b1),
   (c) about 18.9 to about 393.3 mg (preferably about 325 to about 365.1 mg) of trehalose dihydrate,
   (d) about 3.75 to about 15.0 mg (preferably about 3.75 to about 11.25 mg) of L-methionine,
   (e) about 2.5 to about 15.0 mg (preferably about 4 to 6 mg) of polysorbate 80, and
   (f) about 0.29 to about 7.5 mg (preferably about 5.8 to about 7.5 mg) of sodium chloride, and
   of which the pH is about 4.9 to about 5.1, and a volume per formulation is about 5 mL;
[55] an aqueous pharmaceutical composition or a formulation thereof for subcutaneous or intravenous administration, containing:
   (a) about 2.5 mg, about 5 mg or about 50 mg of an anti-PD-1/CD3 bispecific antibody as an active ingredient, and further containing:
   (b) an acetate buffer consisting of about 9.4 mg (specifically about 9.35 mg) of sodium acetate trihydrate and about 1.9 mg of glacial acetic acid,
   (c) about 345 mg of trehalose dihydrate,
   (d) about 7.5 mg of L-methionine,
   (e) about 5.0 mg of polysorbate 80, and
   (f) about 6.8 mg of sodium chloride, and
   of which a pH is about 5.0, and a volume per formulation is about 5 mL;
[56] an aqueous pharmaceutical composition or a formulation thereof for subcutaneous or intravenous administration, containing:
   (a) about 0.2 to about 2.4 mg or about 10 to about 50 mg (preferably about 1 mg, about 2 mg or about 20 mg, respectively) of an anti-PD-1/CD3 bispecific antibody as an active ingredient, and further containing:
   (b) an acetate buffer consisting of (b1) about 1.36 to about 13.6 mg of sodium acetate trihydrate and (b2) the amount of glacial acetic acid that makes a pH about 4.7 to about 5.3, corresponding to the amount of sodium acetate trihydrate of the preceding item (b1),
   (c) about 3.8 to about 216.6 mg of trehalose dihydrate,
   (d) about 1.5 to about 9.0 mg of L-methionine,
   (e) about 0.2 to about 10.0 mg of polysorbate 80, and
   (f) about 0.06 to about 4.0 mg of sodium chloride, and
   of which the pH is about 4.7 to about 5.3, and a volume per formulation is about 2 mL;
[57] an aqueous pharmaceutical composition or a formulation thereof for subcutaneous or intravenous administration, containing:
   (a) about 0.2 to about 2.4 mg or about 10 to about 50 mg (preferably about 1 mg, about 2 mg or about 20 mg, respectively) of an anti-PD-1/CD3 bispecific antibody as an active ingredient, and further containing:
   (b) an acetate buffer consisting of (b1) about 2.72 to about 8.16 mg (preferably about 3.36 to about 4.1 mg) of sodium acetate trihydrate and (b2) the amount of glacial acetic acid that makes a pH about 4.9 to about 5.1, corresponding to the amount of sodium acetate trihydrate of the preceding item (b1),
   (c) about 7.6 to about 157.4 mg (preferably about 130.2 to about 145.2 mg) of trehalose dihydrate,
   (d) about 1.5 to about 6.0 mg (preferably about 1.5 to about 4.5 mg) of L-methionine,
   (e) about 1.0 to about 6.0 mg of polysorbate 80, and
   (f) about 0.12 to about 3.0 mg (preferably about 2.3 to about 3.0 mg) of sodium chloride, and
   of which the pH is about 4.9 to about 5.1, and a volume per formulation is about 2 mL;
[58] an aqueous pharmaceutical composition or a formulation thereof for subcutaneous or intravenous administration, containing:
   (a) about 1 mg, about 2 mg or about 20 mg of an anti-PD-1/CD3 bispecific antibody as an active ingredient, and further containing:
   (b) an acetate buffer consisting of about 3.7 mg (specifically about 3.74 mg) of sodium acetate trihydrate and about 0.8 mg (specifically about 0.76 mg) of glacial acetic acid,
   (c) about 138 mg of trehalose dihydrate,
   (d) about 3.0 mg of L-methionine,
   (e) about 2.0 mg of polysorbate 80, and
   (f) about 2.7 mg (specifically about 2.72 mg) of sodium chloride, and
   of which a pH is about 5.0, and a volume per formulation is about 2 mL;
[59] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) an acetate buffer consisting of (b1) about 0.68 to about 6.8 mg/mL of sodium acetate trihydrate and (b2) the amount of acetic acid that makes a pH about 4.7 to about 5.3, corresponding to the amount of sodium acetate trihydrate of the preceding item (b1),
   (c) about 1.9 to about 108.3 mg/mL of trehalose dihydrate,
   (d) about 0.75 to about 4.5 mg/mL of L-methionine,
   (e) about 0.1 to about 5.0 mg/mL of polysorbate 80, and
   (f) about 0.03 to about 2.0 mg/mL of sodium chloride, and
   of which the pH is about 4.7 to about 5.3;
[60] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) an acetate buffer consisting of (b1) about 1.36 to about 4.08 mg/mL (preferably about 1.68 to about 2.05 mg/mL) of sodium acetate trihydrate and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and
   of which the pH is about 4.9 to about 5.1;
[61] an aqueous pharmaceutical composition or a formulation thereof for subcutaneous administration, containing:
   (a) about 100 mg/mL of an anti-PD-1/CD3 bispecific antibody as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and
   of which a pH is about 5.0;
[62] an aqueous pharmaceutical composition or a formulation thereof for subcutaneous administration, containing:
   (a) about 80 to about 150 mg (preferably about 100 mg) of an anti-PD-1/CD3 bispecific antibody as an active ingredient, and further containing:
   (b) an acetate buffer consisting of (b1) about 0.68 to about 6.8 mg of sodium acetate trihydrate, and (b2) the amount of glacial acetic acid that makes a pH about 4.7 to about 5.3, corresponding to the amount of sodium acetate trihydrate of the preceding item (b1),
   (c) about 1.9 to about 108.3 mg of trehalose dihydrate,
   (d) about 0.75 to about 4.5 mg of L-methionine,
   (e) about 0.1 to about 5.0 mg of polysorbate 80, and
   (f) about 0.03 to about 2.0 mg of sodium chloride, and
   of which the pH is about 4.7 to about 5.3, and a volume per formulation is about 1 mL;
[63] an aqueous pharmaceutical composition or a formulation thereof for subcutaneous administration, containing:
   (a) about 80 to about 150 mg (preferably about 100 mg) of an anti-PD-1/CD3 bispecific antibody as an active ingredient, and further containing:
   (b) an acetate buffer consisting of (b1) about 1.36 to about 4.08 mg (preferably about 1.68 to about 2.05 mg) of sodium acetate trihydrate and (b2) the amount of glacial acetic acid that makes a pH about 4.9 to about 5.1, corresponding to the amount of sodium acetate trihydrate of the preceding item (b1),
   (c) about 3.8 to about 78.7 mg (preferably about 65.1 to about 72.6 mg) of trehalose dihydrate,
   (d) about 0.75 to about 3.0 mg (preferably about 0.75 to about 2.25 mg) of L-methionine,
   (e) about 0.5 to about 3.0 mg of polysorbate 80, and
   (f) about 0.06 to about 1.5 mg (preferably about 1.15 to about 1.5 mg) of sodium chloride, and
   of which the pH is about 4.9 to about 5.1, and a volume per formulation is about 1 mL;
[64] an aqueous pharmaceutical composition or a formulation thereof for subcutaneous administration, containing:
   (a) about 100 mg of an anti-PD-1/CD3 bispecific antibody as an active ingredient, and further containing:
   (b) an acetate buffer consisting of about 1.9 mg (specifically about 1.87 mg) of sodium acetate trihydrate and about 0.4 mg (specifically about 0.38 mg) of glacial acetic acid,
   (c) about 69 mg of trehalose dihydrate,
   (d) about 1.5 mg of L-methionine,
   (e) about 1.0 mg of polysorbate 80, and
   (f) about1.4 mg (specifically about 1.36 mg) of sodium chloride, and
   of which a pH is about 5.0, and a volume per formulation is about 1 mL;
[65] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) a buffer (preferably about 7.3 to about 73.0 mM (preferably about 14.6 to about 43.8 mM, more preferably about 18 to about 22 mM, even more preferably about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182.38 mM) of trehalose,
   (d) about 5 to about 30.2 mM (preferably about 5 to about 20 mM, more preferably about 5 to about 15 mM, even more preferably about 10.05 mM) of L-methionine,
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[66] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) about 7.3 to about 73.0 mM (preferably about 14.6 to about 43.8 mM, more preferably about 18 to about 22 mM, even more preferably about 20.07 mM) of an acetate buffer,
   (c) about 5 to about 286.1 mM (preferably about 10 to about 207.9 mM, more preferably about 172 to about 193 mM, even more preferably about 182.38 mM) of trehalose,
   (d) an antioxidant (preferably about 5 to about 30.2 mM (preferably about 5 to about 20 mM, more preferably about 5 to about 15 mM, even more preferably about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[67] an aqueous pharmaceutical composition or a formulation thereof for subcutaneous or intravenous administration, containing:
   (a) about 0.1 to about 1.2 mg/mL or about 5 to about 25 mg/mL (preferably about 0.5 mg/mL, about 1 mg/mL or about 10 mg/mL. respectively) of an anti-PD-1/CD3 bispecific antibody as an active ingredient, and further containing:
   (b) a buffer (preferably about 7.3 to about 73.0 mM (preferably about 14.6 to about 43.8 mM, more preferably about 18 to about 22 mM, even more preferably about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182.38 mM) of trehalose,
   (d) an antioxidant (preferably about 5 to about 30.2 mM (preferably about 5 to about 20 mM, more preferably about 5 to about 15 mM, even more preferably about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[68] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) a buffer (preferably about 7.3 to about 73.0 mM (preferably about 14.6 to about 43.8 mM, more preferably about 18 to about 22 mM, even more preferably about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182.38 mM) of trehalose,
   (d) about 5 to about 30.2 mM (preferably about 5 to about 20 mM, more preferably about 5 to about 15 mM, even more preferably about 10.05 mM) of L-methionine,
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[69] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) about 7.3 to about 73.0 mM (preferably about 14.6 to about 43.8 mM, more preferably about 18 to about 22 mM, even more preferably about 20.07 mM) of an acetate buffer,
   (c) about 5 to about 286.1 mM (preferably about 10 to about 207.9 mM, more preferably about 172 to about 193 mM, even more preferably about 182.38 mM) of trehalose,
   (d) an antioxidant (preferably about 5 to about 30.2 mM (preferably about 5 to about 20 mM, more preferably about 5 to about 15 mM, even more preferably about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[70] an aqueous pharmaceutical composition or a formulation thereof for subcutaneous administration, containing:
   (a) about 80 to about 150 mg/mL (preferably about 100 mg/mL) of an anti-PD-1/CD3 bispecific antibody as an active ingredient, and further containing:
   (b) a buffer (preferably about 7.3 to about 73.0 mM (preferably about 14.6 to about 43.8 mM, more preferably about 18 to about 22 mM, even more preferably about 20.07 mM) of an acetate buffer or a buffer with the pH buffering performance equivalent to the above acetate buffer),
   (c) about 5 to about 286.1 mM (preferably about 10 to about 207.9 mM, more preferably about 172 to about 193 mM, even more preferably about 182.38 mM) of trehalose,
   (d) an antioxidant (preferably about 5 to about 30.2 mM (preferably about 5 to about 20 mM, more preferably about 5 to about 15 mM, even more preferably about 10.05 mM) of L-methionine or an antioxidant with the antioxidant capacity equivalent to the above L-methionine),
   (e) about 0.01 to about 0.5 % (w/v) (preferably about 0.05 to about 0.3 % (w/v), more preferably about 0.08 to about 0.12 % (w/v), even more preferably about 0.1 % (w/v)) of polysorbate 80, and
   (f) about 0.5 to about 34.2 mM (preferably about 1.0 to about 25.7 mM, more preferably about 19.6 to about 25.7 mM, even more preferably about 23.27 mM) of sodium chloride, and
   of which a pH is about 4.7 to about 5.3 (preferably about 4.9 to about 5.1, more preferably about 5.0);
[71] the aqueous pharmaceutical composition or formulation thereof according to any one of the preceding items [1] to [70], wherein the anti-PD-1/CD3 bispecific antibody is the anti-PD-1/CD3 bispecific antibody disclosed in the specification of the patent application identified by WO2019/156199;
[72] the aqueous pharmaceutical composition or formulation thereof according to any one of the preceding items [1] to [70], wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and

   wherein (a) the heavy chain of the preceding item (i) comprises any one of the amino acid sequences selected from SEQ ID NO:1, SEQ ID NO:2, SEQ ID NO:3, SEQ ID NO:4 and SEQ ID NO:5,
   (b) the heavy chain of the preceding item (ii) comprises the amino acid sequence set forth in SEQ ID NO:6, and
   (c) both of the light chains of the preceding items (i) and (ii) comprise the amino acid sequence set forth in SEQ ID NO:7;
[73] the aqueous pharmaceutical composition or formulation thereof according to the preceding item [72], wherein the heavy chain constituting the antigen-binding site specifically binding to PD-1 comprises the amino acid sequence set forth in SEQ ID NO:5;
[74] the aqueous pharmaceutical composition or formulation thereof according to any one of the preceding items [71] to [73], wherein a heavy chain constant region of the heavy chain constituting the antigen-binding site specifically binding to PD-1, of the preceding item [72] or [73] was replaced with a heavy chain constant region comprising any one of the amino acid sequences selected from SEQ ID NO:8, SEQ ID NO:9, SEQ ID NO:10, SEQ ID NO:11, SEQ ID NO:12 and SEQ ID NO:13, and a heavy chain constant region of the heavy chain constituting the antigen-binding site specifically binding to CD3, of the preceding item [72] was replaced with a heavy chain constant region comprising any one of the amino acid sequences selected from SEQ ID NO:14, SEQ ID NO:15, SEQ ID NO:16, SEQ ID NO:17, SEQ ID NO:18 and SEQ ID NO:19;
[75] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose,
   (d) about 5 to about 20 mM (preferably about 5 to about 15 mM) of L-methionine,
   (e) about 0.05 to about 0.3 % (w/v) (preferably about 0.08 to about 0.12 % (w/v)) of polysorbate 80, and
   (f) about 1.0 to about 25.7 mM (preferably about 19.6 to about 25.7 mM) of sodium chloride, and
   of which a pH is about 4.9 to about 5.1, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[76] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) about 20.07 mM of an acetate buffer,
   (c) about 182.38 mM of trehalose,
   (d) about 10.05 mM of L-methionine,
   (e) about 0.1 % (w/v) of polysorbate 80, and
   (f) about 23.27 mM of sodium chloride, and
   of which a pH is about 5.0, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[77] an aqueous pharmaceutical composition or a formulation thereof for subcutaneous administration, containing:
   (a) about 100 mg/mL of an anti-PD-1/CD3 bispecific antibody as an active ingredient, and further containing:
   (b) about 14.6 to about 43.8 mM (preferably about 18 to about 22 mM) of an acetate buffer,
   (c) about 10 to about 207.9 mM (preferably about 172 to about 193 mM) of trehalose,
   (d) about 5 to about 20 mM (preferably about 5 to about 15 mM) of L-methionine,
   (e) about 0.05 to about 0.3 % (w/v) (preferably about 0.08 to about 0.12 % (w/v)) of polysorbate 80, and
   (f) about 1.0 to about 25.7 mM (preferably about 19.6 to about 25.7 mM) of sodium chloride, and
   of which a pH is about 4.9 to about 5.1, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[78] an aqueous pharmaceutical composition or a formulation thereof for subcutaneous administration, containing:
   (a) about 100 mg/mL of an anti-PD-1/CD3 bispecific antibody as an active ingredient, and further containing:
   (b) about 20.07 mM of an acetate buffer,
   (c) about 182.38 mM of trehalose,
   (d) about 10.05 mM of L-methionine,
   (e) about 0.1 % (w/v) of polysorbate 80, and
   (f) about 23.27 mM of sodium chloride, and
   of which a pH is about 5.0, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[79] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) an acetate buffer consisting of (b1) about 1.36 to about 4.08 mg/mL (preferably about 1.68 to about 2.05 mg/mL) of sodium acetate trihydrate and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and
   of which the pH is about 4.9 to about 5.1, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[80] an aqueous pharmaceutical composition or a formulation thereof for subcutaneous or intravenous administration, containing:
   (a) about 0.5 mg/mL, about 1 mg/mL or about 10 mg/mL of an anti-PD-1/CD3 bispecific antibody as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and
   of which a pH is about 5.0, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[81] an aqueous pharmaceutical composition or a formulation thereof for subcutaneous administration, containing:
   (a) about 100 mg/mL of an anti-PD-1/CD3 bispecific antibody as an active ingredient, and further containing:
   (b) an acetate buffer consisting of (b1) about 1.36 to about 4.08 mg/mL (preferably about 1.68 to about 2.05 mg/mL) of sodium acetate trihydrate and (b2) the amount of acetic acid that makes a pH about 4.9 to about 5.1, corresponding to the amount of sodium acetate trihydrate of the preceding item (b1),
   (c) about 3.8 to about 78.7 mg/mL (preferably about 65.1 to about 72.6 mg/mL) of trehalose dihydrate,
   (d) about 0.75 to about 3.0 mg/mL (preferably about 0.75 to about 2.25 mg/mL) of L-methionine,
   (e) about 0.5 to about 3.0 mg/mL (preferably about 0.8 to about 1.2 mg/mL) of polysorbate 80, and
   (f) about 0.06 to about 1.5 mg/mL (preferably about 1.15 to about 1.5 mg/mL) of sodium chloride, and
   of which the pH is about 4.9 to about 5.1, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[82] an aqueous pharmaceutical composition or a formulation thereof for subcutaneous administration, containing:
   (a) about 100 mg/mL of an anti-PD-1/CD3 bispecific antibody as an active ingredient, and further containing:
   (b) an acetate buffer consisting of about 1.9 mg/mL (specifically about 1.87 mg/mL) of sodium acetate trihydrate and about 0.4 mg/mL (specifically about 0.38 mg/mL) of acetic acid,
   (c) about 69 mg/mL of trehalose dihydrate,
   (d) about 1.5 mg/mL of L-methionine,
   (e) about 1.0 mg/mL of polysorbate 80, and
   (f) about 1.4 mg/mL (specifically about 1.36 mg/mL) of sodium chloride, and
   of which a pH is about 5.0, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[83] an aqueous pharmaceutical composition or a formulation thereof for subcutaneous or intravenous administration, containing:
   (a) about 2.5 mg, about 5 mg or about 50 mg of an anti-PD-1/CD3 bispecific antibody as an active ingredient, and further containing:
   (b) an acetate buffer consisting of (b1) about 6.8 to about 20.4 mg (preferably about 8.4 to about 10.2 mg) of sodium acetate trihydrate and (b2) the amount of glacial acetic acid that makes a pH about 4.9 to about 5.1, corresponding to the amount of sodium acetate trihydrate of the preceding item (b1),
   (c) about 18.9 to about 393.3 mg (preferably about 325 to about 365.1 mg) of trehalose dihydrate,
   (d) about 3.75 to about 15.0 mg (preferably about 3.75 to about 11.25 mg) of L-methionine,
   (e) about 2.5 to about 15.0 mg (preferably about 4 to 6 mg) of polysorbate 80, and
   (f) about 0.29 to about 7.5 mg (preferably about 5.8 to about 7.5 mg) of sodium chloride, and
   of which the pH is about 4.9 to about 5.1, and a volume per formulation is about 5 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[84] an aqueous pharmaceutical composition or a formulation thereof for subcutaneous or intravenous administration, containing:
   (a) about 2.5 mg, about 5 mg or about 50 mg of an anti-PD-1/CD3 bispecific antibody as an active ingredient, and further containing:
   (b) an acetate buffer consisting of about 9.4 mg (specifically about 9.35 mg) of sodium acetate trihydrate and about 1.9 mg of glacial acetic acid,
   (c) about 345 mg of trehalose dihydrate,
   (d) about 7.5 mg of L-methionine,
   (e) about 5.0 mg of polysorbate 80, and
   (f) about 6.8 mg of sodium chloride, and
   of which a pH is about 5.0, and a volume per formulation is about 5 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[85] an aqueous pharmaceutical composition or a formulation thereof for subcutaneous or intravenous administration, containing:
   (a) about 1 mg of an anti-PD-1/CD3 bispecific antibody as an active ingredient, and further containing:
   (b) an acetate buffer consisting of (b1) about 2.72 to about 8.16 mg (preferably about 3.36 to about 4.1 mg) of sodium acetate trihydrate and (b2) the amount of glacial acetic acid that makes a pH about 4.9 to about 5.1, corresponding to the amount of sodium acetate trihydrate of the preceding item (b1),
   (c) about 7.6 to about 157.4 mg (preferably about 130.2 to about 145.2 mg) of trehalose dihydrate,
   (d) about 1.5 to about 6.0 mg (preferably about 1.5 to about 4.5 mg) of L-methionine,
   (e) about 1.0 to about 6.0 mg of polysorbate 80, and
   (f) about 0.12 to about 3.0 mg (preferably about 2.3 to about 3.0 mg) of sodium chloride, and
   of which the pH is about 4.9 to about 5.1, and a volume per formulation is about 2 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[86] an aqueous pharmaceutical composition or a formulation thereof for subcutaneous or intravenous administration, containing:
   (a) about 1 mg of an anti-PD-1/CD3 bispecific antibody as an active ingredient, and further containing:
   (b) an acetate buffer consisting of about 3.7 mg (specifically about 3.74 mg) of sodium acetate trihydrate and about 0.8 mg (specifically about 0.76 mg) of glacial acetic acid,
   (c) about 138 mg of trehalose dihydrate,
   (d) about 3.0 mg of L-methionine,
   (e) about 2.0 mg of polysorbate 80, and
   (f) about 2.7 mg (specifically about 2.72 mg) of sodium chloride, and
   of which a pH is about 5.0, and a volume per formulation is about 2 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[87] an aqueous pharmaceutical composition or a formulation thereof for subcutaneous administration, containing:
   (a) about 100 mg of an anti-PD-1/CD3 bispecific antibody as an active ingredient, and further containing:
   (b) an acetate buffer consisting of (b1) about 1.36 to about 4.08 mg (preferably about 1.68 to about 2.05 mg) of sodium acetate trihydrate and (b2) the amount of glacial acetic acid that makes a pH about 4.9 to about 5.1, corresponding to the amount of sodium acetate trihydrate of the preceding item (b1),
   (c) about 3.8 to about 78.7 mg (preferably about 65.1 to about 72.6 mg) of trehalose dihydrate,
   (d) about 0.75 to about 3.0 mg (preferably about 0.75 to about 2.25 mg) of L-methionine,
   (e) about 0.5 to about 3.0 mg of polysorbate 80, and
   (f) about 0.06 to about 1.5 mg (preferably about 1.15 to about 1.5 mg) of sodium chloride, and
   of which the pH is about 4.9 to about 5.1, and a volume per formulation is about 1 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[88] an aqueous pharmaceutical composition or a formulation thereof for subcutaneous administration, containing:
   (a) about 100 mg of an anti-PD-1/CD3 bispecific antibody as an active ingredient, and further containing:
   (b) an acetate buffer consisting of about 1.9 mg (specifically about 1.87 mg) of sodium acetate trihydrate and about 0.4 mg (specifically about 0.38 mg) of glacial acetic acid,
   (c) about 69 mg of trehalose dihydrate,
   (d) about 1.5 mg of L-methionine,
   (e) about 1.0 mg of polysorbate 80, and
   (f) about1.4 mg (specifically about 1.36 mg) of sodium chloride, and
   of which a pH is about 5.0, and a volume per formulation is about 1 mL, and
   wherein the anti-PD-1/CD3 bispecific antibody comprises:
   (i) a heavy chain and a light chain constituting an antigen-binding site specifically binding to PD-1, and
   (ii) a heavy chain and a light chain constituting an antigen-binding site specifically binding to CD3, and wherein (α) the heavy chain of the preceding item (i) comprises the amino acid sequence set forth in SEQ ID NO:5,

   (β) the heavy chain of the preceding item (ii) comprises the amino acid sequence set forth in SEQ ID NO:6, and
   (γ) both of the light chains of the preceding items (i) and (ii) comprise the amino acid sequence set forth in SEQ ID NO:7;
[89] the aqueous pharmaceutical composition or formulation thereof according to any one of the preceding items [1] to [88], of which a conductivity is about 2.6 to about 3.1 mS/cm;
[90] an aqueous pharmaceutical composition or a formulation thereof for subcutaneous or intravenous administration, containing an antibody (preferably an anti-PD-1/CD3 bispecific antibody) as the active ingredient, and further containing a buffer (preferably an acetate buffer), trehalose, an antioxidant (preferably L-methionine), polysorbate 80, and of which a conductivity is about 2.6 to about 3.1 mS/cm;
[91] the aqueous pharmaceutical composition or formulation thereof according to any one of the preceding items [1] to [90], of which a conductivity is about 2.8 mS/cm;
[92] an aqueous pharmaceutical composition or a formulation thereof for intravenous administration, which is bioequivalent to the aqueous pharmaceutical composition or formulation thereof of any one of the preceding items [75], [76], [79], [80], [83] to [86] and [89] to [91] and contains the anti-PD-1/CD3 bispecific antibody of the same items as an active ingredient;
[93] an aqueous pharmaceutical composition or a formulation thereof for subcutaneous administration, which is bioequivalent to the aqueous pharmaceutical composition or formulation thereof of any one of the preceding items [75] to [91] and contains the anti-PD-1/CD3 bispecific antibody of the said items as an active ingredient;
[94] the aqueous pharmaceutical composition or formulation thereof according to the preceding item [92], wherein the bioequivalence is defined as a relationship that 90 % confidence interval of a difference between the following (i) and (ii) is in a range of log (0.80) to log (1.25):
   (i) the mean of a logarithm value of a determination parameter for the bioequivalence under administration thereof, and
   (ii) the mean of a logarithm value of a determination parameter for the bioequivalence under administration of the aqueous pharmaceutical composition or formulation thereof of any one of the preceding items [75], [76], [79], [80], [83] to [86] and [89] to [91];
[95] the aqueous pharmaceutical composition or formulation thereof according to the preceding item [93], wherein the bioequivalence is defined as a relationship that 90 % confidence interval of a difference between the following (i) and (ii) is in a range of log (0.80) to log (1.25):
   (i) the mean of a logarithm value of a determination parameter for the bioequivalence under administration thereof, and
   (ii) the mean of a logarithm value of a determination parameter for the bioequivalence under administration of the aqueous pharmaceutical composition or formulation thereof of any one of the preceding items [75] to [91];

   [1-1] a pre-filled syringe or cartridge, pre-filled with the aqueous pharmaceutical composition for subcutaneous administration of any one of the preceding items [1] to [91], [93] and [95];
   [1-2] an injector pen, auto-injector or needle-free device filled with the cartridge of the preceding item [1-1];
   [1-3] a vial or ampoule container containing the aqueous pharmaceutical composition (preferably for intravenous administration) of any one of the preceding items [1] to [95];
   [2-1] use of (i) the aqueous pharmaceutical composition or formulation thereof of any one of the preceding items [1] to [95], (ii) the pre-filled syringe or cartridge of the preceding item [1-1], (iii) the injector pen, auto-injector or needle-free device of the preceding item [1-2], or (iv) the vial or ampoule container of the preceding item [1-3], in prevention, suppression of symptom progression, suppression of recurrence and/or treatment of autoimmune disease or graft-versus-host disease (GVHD), or hematological cancer;
   [2-2] the use according to the preceding item [2-1], wherein autoimmune disease is psoriasis, psoriatic arthritis, rheumatoid arthritis, Crohn's disease, ulcerative colitis or myasthenia gravis; and [2-3] the use according to the preceding item [2-1], wherein hematological cancer is peripheral T-cell lymphoma or cutaneous T-cell lymphoma.

In the present specification, the contents of all patent and non-patent literature or references expressly cited herein all may be cited as part of this specification.

The present invention is further described in detail by the following examples, but the scope of the present invention is not limited thereto. Various changes or modifications based on the description of the present invention are possible to those skilled in the art, and these changes or modifications are also included in the present invention.

### [Examples]

### Example 1: Formulation Screening

The basic condition for formulations optimal for the anti-PD-1/CD3 bispecificity antibody of the present invention, Antibody α, was searched. The 30 samples, B1 to B30, were prepared under the conditions shown in Tables 1 and 2, respectively, to achieve an antibody concentration of 20 mg/mL.

**[Table 1]**

| **Sample** | **pH** | **Buffer solution, Salt** |
|---|---|---|
| **B1** | 4.5 | 20 mM acetate buffer solution |
| | | 0 mM sodium chloride |
| **B2** | 4.5 | 20 mM acetate buffer solution |
| | | 20 mM sodium chloride |
| **B3** | 4.5 | 20 mM acetate buffer solution |
| | | 200 mM sodium chloride |
| **B4** | 5.0 | 20 mM acetate buffer solution |
| | | 0 mM sodium chloride |
| **B5** | 5.0 | 20 mM acetate buffer solution |
| | | 20 mM sodium chloride |
| **B6** | 5.0 | 20 mM acetate buffer solution |
| | | 200 mM sodium chloride |
| **B7** | 5.0 | 20 mM citrate buffer solution |
| | | 0 mM sodium chloride |
| **B8** | 5.0 | 20 mM citrate buffer solution |
| | | 20 mM sodium chloride |
| **B9** | 5.0 | 20 mM citrate buffer solution |
| | | 200 mM sodium chloride |
| **B10** | 5.5 | 20 mM acetate buffer solution |
| | | 0 mM sodium chloride |
| **B11** | 5.5 | 20 mM acetate buffer solution |
| | | 20 mM sodium chloride |
| **B12** | 5.5 | 20 mM acetate buffer solution |
| | | 200 mM sodium chloride |
| **B13** | 5.5 | 20 mM citrate buffer solution |
| | | 0 mM sodium chloride |
| **B14** | 5.5 | 20 mM citrate buffer solution |
| | | 20 mM sodium chloride |
| **B15** | 5.5 | 20 mM citrate buffer solution |
| | | 200 mM sodium chloride |

**[Table 2]**

| **Sample** | **pH** | **Buffer solution, Salt** |
|---|---|---|
| **B16** | 5.5 | 20 mM histidine buffer solution |
| | | 0 mM sodium chloride |
| **B17** | 5.5 | 20 mM histidine buffer solution |
| | | 20 mM sodium chloride |
| **B18** | 5.5 | 20 mM histidine buffer solution |
| | | 200 mM sodium chloride |
| **B19** | 6.0 | 20 mM citrate buffer solution |
| | | 0 mM sodium chloride |
| **B20** | 6.0 | 20 mM citrate buffer solution |
| | | 20 mM sodium chloride |
| **B21** | 6.0 | 20 mM citrate buffer solution |
| | | 200 mM sodium chloride |
| **B22** | 6.0 | 20 mM histidine buffer solution |
| | | 0 mM sodium chloride |
| **B23** | 6.0 | 20 mM histidine buffer solution |
| | | 20 mM sodium chloride |
| **B24** | 6.0 | 20 mM histidine buffer solution |
| | | 200 mM sodium chloride |
| **B25** | 6.5 | 20 mM phosphate buffer solution |
| | | 0 mM sodium chloride |
| **B26** | 6.5 | 20 mM phosphate buffer solution |
| | | 20 mM sodium chloride |
| **B27** | 6.5 | 20 mM phosphate buffer solution |
| | | 200 mM sodium chloride |
| **B28** | 6.5 | 20 mM histidine buffer solution |
| | | 0 mM sodium chloride |
| **B29** | 6.5 | 20 mM histidine buffer solution |
| | | 20 mM sodium chloride |
| **B30** | 6.5 | 20 mM histidine buffer solution |
| | | 200 mM sodium chloride |

The diffusion interaction parameter (KD), aggregation initiation temperature (Tagg) and solubility were measured for the 30 types of samples, respectively. In addition, the accelerated and storage tests were conducted, and the concentration and turbidity were measured, as well as monomer residues by SEC-HPLC measurement.

The diffusion interaction parameters were measured and analyzed at 25 °C for the antibody concentration (1.25, 2.5, 5.0, 10, and 15 mg/mL) of each sample, and calculated by plotting the diffusion coefficient (µm²/s) for each formulation.

The aggregation initiation temperature was measured and analyzed by increasing the temperature from 25 to 80 °C at 1 mg/mL of the antibody concentration of each sample. By plotting the particle size (nm) for each formulation, the agglomeration initiation temperature was defined as the value at which the mean particle size increased by 10 % from the mean particle size at 25 °C.

Note that a plate reader (DynaPro PlateReaderII (WYATT Technology)) and analysis software (Dynamics V7) were used to measure the diffusion interaction parameters and the aggregation initiation temperature.

For the solubility measurements, samples at 2 mg/mL were reacted with solvents at 10 points of ammonium sulfate concentrations ranging from 1.1 M to 2.0 M at 0.1 M intervals, and then centrifuged at 15,000 g for 30 minutes. The absorbances at 280 nm were measured for supernatants after centrifugation. The solubilities (%) of the samples were plotted against the concentration of ammonium sulfate, and the concentrations of ammonium sulfate at 50 % solubility were compared. Note that a plate reader (INFINITE 200 PRO M PLEX (TECAN)) was used to measure the absorbance.

For the storage and acceleration tests, samples (1 mg/mL) were sterilized with a 0.22 µm filter and dispensed into vials.

The storage test was conducted at 4°C and 20°C for 0 and 4 weeks, respectively. The stored samples were evaluated for concentration, turbidity and SEC-HPLC analysis. The supernatants obtained by centrifugation at 15,000 g for 30 minutes were all used as samples for measurements. The concentrations were calculated based on the absorbance at 280 nm (optical path length 2 mm). The turbidities were calculated based on the absorbance at 500 nm (optical path length 1 cm). SEC-HPLC was performed on Alliance HPLC system (Waters) under the conditions shown in Table 3.

The acceleration test was conducted by shaking at 500 rpm for 3 days at 20 °C. The evaluation was performed in the same manner as the storage test.

**[Table 3]**

| Items | Details |
|---|---|
| Column | TSKgel G3000SWXL |
| Sample | 1 mg/mL, 20 uL |
| Column Temperature | 30°C |
| Flow rate | 0.5 mL/min |
| Mobile phase | PBS, pH 7.4 |
| Measurement time | 30 min |
| UV detection | 215. 280 nm |

The calculated and measured results of the diffusion interaction parameter (KD), agglomeration initiation temperature (Tagg), and solubility are shown in descending order in Table 4, respectively. In addition, the result of the storage test (for 4 weeks at 4 °C) in Table 5, that of the storage test (for 4 weeks at 20 °C) in Table 6, and that of the acceleration test (shaking for 3 days at 20 °C) in Table 7 are shown, respectively. In each table, samples No. 1 to 30 correspond to samples B1 to B30 shown in Table 1 and 2.

**[Table 4]**

| **Diffusion interaction parameter (KD)** | | **Agglomeration initiation temperature (Tagg)** | | **Solubility** | |
|---|---|---|---|---|---|
| **Sample No.** | **Calculated value** | **Sample No.** | **Measured value** | **Sample No.** | **Measured value** |
| 4 | 25.1 | 28 | 65.9 | 4 | 1.42 |
| 22 | 17.4 | 10 | 65.3 | 7 | 1.41 |
| 10 | 14.3 | 23 | 65.2 | 10 | 1.41 |
| 5 | 12.0 | 22 | 65.1 | 5 | 1.41 |
| 11 | 7.4 | 29 | 65.1 | 6 | 1.39 |
| 28 | 6.6 | 4 | 64.1 | 12 | 1.39 |
| 16 | 0.9 | 25 | 64.0 | 11 | 1.39 |
| 8 | -0.2 | 11 | 63.9 | 13 | 1.39 |
| 23 | -2.6 | 26 | 63.2 | 8 | 1.39 |
| 7 | -3.4 | 16 | 62.6 | 14 | 1.38 |
| 29 | -4.4 | 30 | 62.4 | 17 | 1.38 |
| 17 | -5.0 | 27 | 62.3 | 19 | 1.38 |
| 6 | -6.3 | 20 | 62.1 | 16 | 1.37 |
| 25 | -6.4 | 19 | 62.0 | 20 | 1.36 |
| 12 | -7.5 | 21 | 61.3 | 22 | 1.34 |
| 14 | -7.6 | 5 | 61.2 | 23 | 1.33 |
| 9 | -8.3 | 17 | 60.1 | 26 | 1.32 |
| 26 | -8.6 | 12 | 59.9 | 25 | 1.31 |
| 18 | -8.8 | 14 | 59.8 | 15 | 1.29 |
| 13 | -9.2 | 13 | 59.7 | 9 | 1.29 |
| 15 | -9.5 | 24 | 59.4 | 28 | 1.28 |
| 21 | -11.1 | 15 | 58.4 | 21 | 1.27 |
| 24 | -11.3 | 6 | 56.9 | 29 | 1.27 |
| 27 | -11.3 | 8 | 56.1 | 24 | 1.26 |
| 19 | -11.5 | 7 | 56.0 | 18 | 1.26 |
| 20 | -11.5 | 18 | 55.1 | 27 | 1.26 |
| 30 | -12.3 | 9 | 52.7 | 30 | 1.21 |

**[Table ]**

| | **Initial stage** | | | **After storage test (4°C, 4 weeks)** | | | | | |
|---|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage, %)** | **Turbidity** | **Identification of visible particles** | **SEC-HPLC (Monomer area)** | **SEC-HPLC (Roto of monomer reduction. %)** |
| **1** | 0.995 | 0.000 | 3085772 | 0.972 | 97.7 | 0.0017 | | 2721454 | 11.81 |
| **2** | 0.953 | 0.000 | 3184351 | 0.942 | 98.8 | 0.0007 | | 2929125 | 8.02 |
| **3** | 0.957 | 0.000 | 3218921 | 0.94 | 98.2 | 0.0000 | | 2875482 | 10.67 |
| **4** | 0.923 | 0.000 | 3117715 | 0.908 | 98.4 | 0.0010 | | 2845592 | 8.73 |
| **6** | 0.928 | 0.001 | 3101663 | 0.928 | 100 | 0.0010 | | 2869258 | 7.49 |
| **6** | 0.977 | 0.000 | 3171094 | 0.954 | 97.6 | 0.0013 | | 2938917 | 7.32 |
| **7** | 1.04 | 0.003 | 3516268 | 1.04 | 100 | 0.0010 | | N/A | N/A |
| **8** | 1.04 | 0.000 | 3477293 | 1.02 | 98.1 | 0.0007 | | 3143157 | 9.61 |
| **9** | 1.06 | 0.000 | 3510066 | 1.04 | 98.1 | 0.0037 | x | 3172506 | 9.62 |
| **10** | 0.972 | 0.000 | 3331445 | 0.969 | 99.7 | 0.0010 | | 2982690 | 10.47 |
| **11** | 1.02 | 0.000 | 3396812 | 0.972 | 95.3 | 0.0007 | | 3012343 | 11.32 |
| **12** | 1.02 | 0.000 | 3320481 | 0.996 | 97.6 | 0.0040 | | 3032527 | 8.67 |
| **13** | 0.988 | 0.000 | 3341787 | 0.978 | 99.0 | 0.0023 | | 3149688 | 5.75 |
| **14** | 1.03 | 0.001 | 3403050 | 1 | 97.1 | 0.0023 | | 3161411 | 7.10 |
| **15** | 1.03 | 0.001 | 3419587 | 1.02 | 99.0 | 0.0020 | x | 3074679 | 10.09 |
| **16** | 1.04 | 0.000 | 3501735 | 1.06 | 102 | 0.0013 | | 3300290 | 5.75 |
| **17** | 0.976 | 0.000 | 3297011 | 0.988 | 101 | 0.0020 | | 3082428 | 6.51 |
| **18** | 1.03 | 0.001 | 3302622 | 1.01 | 98.1 | 0.0030 | | 3074519 | 6.91 |
| **19** | 0.962 | 0.000 | 3227278 | 0.947 | 98.4 | 0.0020 | x | 2990165 | 7.35 |
| **20** | 0.992 | 0.001 | 3265647 | 0.967 | 97.5 | 0.0033 | x | 2975534 | 8.88 |
| **21** | 0.985 | 0.000 | 3248329 | 0.966 | 98.1 | 0.0013 | x | 3007958 | 7.40 |
| **22** | 0.926 | -0.001 | 3201106 | 0.908 | 98.1 | 0.0000 | | 2960730 | 7.51 |
| **23** | 0.996 | 0.000 | 3378320 | 0.935 | 93.9 | 0.0000 | | N/A | N/A |
| **24** | 1.03 | -0.001 | 3382440 | 0.999 | 97.0 | 0.0000 | | 3128463 | 7.51 |
| **25** | 1.01 | 0.000 | 3440234 | 0.978 | 96.8 | 0.0013 | | 3108976 | 9.63 |
| **26** | 1.03 | 0.006 | 3421415 | 0.992 | 96.3 | 0.0013 | | 3092862 | 9.60 |
| **27** | 1.04 | 0.007 | 3433584 | 1.01 | 97.1 | 0.0007 | x | 3122367 | 9.06 |
| **28** | 0.953 | 0.001 | 3174457 | 0.912 | 95.7 | 0.0003 | | 2938539 | 7.43 |
| **29** | 0.907 | 0.001 | 3112150 | 0.922 | 102 | 0.0003 | | 2966363 | 4.68 |
| **30** | 0.966 | 0.000 | 3214992 | 0.947 | 98.0 | 0.0023 | | 2869860 | 10.74 |

**[Table 6]**

| | **Initial stage** | | | **Storage test (20°C, 4 weeks)** | | | | | |
|---|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage, %)** | **Turbidity** | **Identification of visible particles** | **SEC-HPLC (Monomer area)** | **SEC-HPLC (Rate of monomor reduction. %)** |
| **1** | 0.995 | 0.000 | 3085772 | 0.975 | 98.0 | 0.002 | | 2755945 | 10.69 |
| **2** | 0.953 | 0.000 | 3184351 | 0.957 | 100 | 0.0003 | | 3048581 | 4.26 |
| **3** | 0.957 | 0.000 | 3218921 | 0.927 | 96.9 | 0.0003 | | 2974749 | 7.59 |
| **4** | 0.923 | 0.000 | 3117715 | 0.929 | 101 | 0.001 | | 2975384 | 4.57 |
| **5** | 0.928 | 0.001 | 3101663 | 0.929 | 100 | 0 | | 2967147 | 4.34 |
| **6** | 0.977 | 0.000 | 3171094 | 0.957 | 98.0 | 0.0017 | | 3003143 | 5.30 |
| **7** | 1.04 | 0.003 | 3516268 | 1.04 | 100 | 0.0013 | | 3291156 | 6.40 |
| **8** | 1.04 | 0.000 | 3477293 | 1.03 | 99.0 | 0.0003 | x | 3268932 | 5.99 |
| **9** | 1.06 | 0.000 | 3510066 | 1.05 | 99.1 | 0.0013 | x | 3261620 | 7.08 |
| **10** | 0.972 | 0.000 | 3331445 | 0.965 | 99.3 | 0.0017 | | N/A | N/A |
| **11** | 1.02 | 0.000 | 3396812 | 1 | 98.0 | 0.0017 | | 3287954 | 3.20 |
| **12** | 1.02 | 0.000 | 3320481 | 0.98 | 96.1 | 0.0017 | x | 3114361 | 6.21 |
| **13** | 0.988 | 0.000 | 3341787 | 0.982 | 99.4 | 0.0027 | x | 3304640 | 1.11 |
| **14** | 1.03 | 0.001 | 3403050 | 1.01 | 98.1 | 0.0003 | x | 3192069 | 6.20 |
| **15** | 1.03 | 0.001 | 3419587 | 1.02 | 99.0 | 0.001 | x | 3356531 | 1.84 |
| **16** | 1.04 | 0.000 | 3501735 | 1.06 | 102 | 0.0017 | | 3432222 | 1.99 |
| **17** | 0.976 | 0.000 | 3297011 | 0.992 | 102 | 0.002 | | 3245598 | 1.56 |
| **18** | 1.03 | 0.001 | 3302622 | 1.02 | 99.0 | 0.0023 | x | 3230998 | 2.17 |
| **19** | 0.962 | 0.000 | 3227278 | 0.951 | 98.9 | 0.0027 | x | 3025422 | 6.25 |
| **20** | 0.992 | 0.001 | 3265647 | 0.969 | 97.7 | 0.0027 | x | 3041018 | 6.88 |
| **21** | 0.985 | 0.000 | 3248329 | 0.97 | 98.5 | 0.0027 | x | 3152816 | 2.94 |
| **22** | 0.926 | -0.001 | 3201106 | 0.939 | 101 | 0 | | 3063093 | 4.31 |
| **23** | 0.996 | 0.000 | 3378320 | 0.978 | 98.2 | 0 | | 3099626 | 8.25 |
| **24** | 1.03 | -0.001 | 3382440 | 1.01 | 98.1 | 0.0013 | | 3254341 | 3.79 |
| **25** | 1.01 | 0.000 | 3440234 | 0.974 | 96.4 | 0.001 | | 3217469 | 6.48 |
| **26** | 1.03 | 0.006 | 3421415 | 0.99 | 96.1 | 0.0007 | | 3253972 | 4.89 |
| **27** | 1.04 | 0.007 | 3433584 | 1 | 96.2 | 0.0023 | x | 3144402 | 8.42 |
| **28** | 0.953 | 0.001 | 3174457 | 0.897 | 94.1 | 0.001 | | 2914637 | 8.18 |
| **29** | 0.907 | 0.001 | 3112150 | 0.919 | 101 | 0.0003 | | 2955218 | 5.04 |
| **30** | 0.966 | 0.000 | 3214992 | 0.95 | 98.3 | 0.0013 | | 2979353 | 7.33 |

**[Table 7]**

| | **Initial stage** | | | **Acceleration test (20°C, Shaking for 3 days)** | | | | |
|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage. %)** | **Turbidity** | **SEC-HPLC (Monomer area)** | **SEC-HPLC (Rate of monomer reduction. %)** |
| **1** | 0.995 | 0.000 | 3085772 | 0.000883 | 0.0887 | 0.225 | 0 | 100 |
| **2** | 0.953 | 0.000 | 3184351 | 0.0208 | 2.18 | 0.887 | 8773 | 100 |
| **3** | 0.957 | 0.000 | 3218921 | 0.0916 | 9.57 | 2.89 | 104479 | 96.8 |
| **4** | 0.923 | 0.000 | 3117715 | 0.0958 | 10.4 | 2.46 | 120599 | 96.1 |
| **5** | 0.928 | 0.001 | 3101663 | 0.00243 | 0.262 | 0.214 | 0 | 100 |
| **6** | 0.977 | 0.000 | 3171094 | 0.00508 | 0.520 | 0.317 | 0 | 100 |
| **7** | 1.04 | 0.003 | 3516268 | 0.00861 | 0.828 | 0.984 | 0 | 100 |
| **8** | 1.04 | 0.000 | 3477293 | 0.00265 | 0.255 | 0.927 | 0 | 100 |
| **9** | 1.06 | 0.000 | 3510066 | 0.000662 | 0.062 | 0.224 | 0 | 100 |
| **10** | 0.972 | 0.000 | 3331445 | 0.00684 | 0.704 | 0.307 | 545 | 100 |
| **11** | 1.02 | 0.000 | 3396812 | 0.00177 | 0.174 | 0.181 | 0 | 100 |
| **12** | 1.02 | 0.000 | 3320481 | 0.00464 | 0.455 | 0.304 | 0 | 100 |
| **13** | 0.988 | 0.000 | 3341787 | 0.00486 | 0.492 | 0.734 | 0 | 100 |
| **14** | 1.03 | 0.001 | 3403050 | 0.00199 | 0.193 | 0.416 | 0 | 100 |
| **15** | 1.03 | 0.001 | 3419587 | 0.00795 | 0.772 | 0.619 | 47 | 100 |
| **16** | 1.04 | 0.000 | 3501735 | 0.0166 | 1.60 | 0.812 | 138 | 100 |
| **17** | 0.976 | 0.000 | 3297011 | 0 | 0.000 | 0.382 | 0 | 100 |
| **18** | 1.03 | 0.001 | 3302622 | 0 | 0.000 | 1.25 | 22 | 100 |
| **19** | 0.962 | 0.000 | 3227278 | 0.00177 | 0.184 | 0.713 | 0 | 100 |
| **20** | 0.992 | 0.001 | 3265647 | 0.0011 | 0.111 | 0.753 | 0 | 100 |
| **21** | 0.985 | 0.000 | 3248329 | 0.00751 | 0.762 | 0.817 | 0 | 100 |
| **22** | 0.926 | -0.001 | 3201106 | 0.00243 | 0.262 | 0.342 | 0 | 100 |
| **23** | 0.996 | 0.000 | 3378320 | 0.00309 | 0.310 | 0.469 | 0 | 100 |
| **24** | 1.03 | -0.001 | 3382440 | 0.0115 | 1.117 | 0.757 | 38 | 100 |
| **25** | 1.01 | 0.000 | 3440234 | 0 | 0.000 | 0.468 | 0 | 100 |
| **26** | 1.03 | 0006 | 3421415 | 0.00243 | 0.236 | 0.379 | 0 | 100 |
| **27** | 1.04 | 0.007 | 3433584 | 0.00464 | 0.446 | 0.754 | 960 | 100 |
| **28** | 0.953 | 0.001 | 3174457 | 0.00464 | 0.487 | 0.229 | 0 | 100 |
| **29** | 0.907 | 0.001 | 3112150 | 0.0011 | 0.121 | 0.135 | 0 | 100 |
| **30** | 0.966 | 0.000 | 3214992 | 0.00132 | 0.137 | 0.67 | 35 | 100 |

Based on the comprehensive evaluation of colloidal stability, thermal stability, and monomer reduction rate by shaking and storage, Sample 4 (20 mM acetate buffer solution (pH 5.0)), Sample 5 (20 mM acetate buffer solution (pH 5.0), 20 mM sodium chloride) and Sample 22 (20 mM histidine buffer solution (pH 6.0)) were selected as formulations to proceed to the next evaluation. Note that the notations "20 mM" shown as the concentration of the acetate buffer solution in the samples listed in Tables 1 and 2 are simplified ones and are exactly 20.07 mM. The same applies to the notations "20 mM acetate buffer solution" in Tables 8 to 11, Table 27, Table 33, Table 38, Table 45 and Table 51 and the corresponding Examples 1 to 10.

### Example 2: Additives evaluation (1)

The 24 types of formulations, S1 to S24, were prepared by adding sugars (sucrose, trehalose, D-mannitol and sorbitol) to the formulations of Samples 4, 5 and 22 selected in the evaluation of Example 1, respectively, as shown in Table 8.

**[Table 8]**

| **Sample** | **pH** | **Buffer solution, Salt** | **Sugar** |
|---|---|---|---|
| **S1** | 5.0 | 20 mM acetate buffer solution | 285 mM sucrose |
| **S2** | 5.0 | 20 mM acetate buffer solution | 142.5 mM sucrose |
| **S3** | 5.0 | 20 mM acetate buffer solution | 285 mM trehalose |
| **S4** | 5.0 | 20 mM acetate buffer solution | 142.5 mM trehalose |
| **S5** | 5.0 | 20 mM acetate buffer solution | 285 mM D-mannitol |
| **S6** | 5.0 | 20 mM acetate buffer solution | 142.5 mM D-mannitol |
| **S7** | 5.0 | 20 mM acetate buffer solution | 285 mM sorbitol |
| **S8** | 5.0 | 20 mM acetate buffer solution | 142.5 mM sorbitol |
| **S9** | 5.0 | 20 mM acetate buffer solution | 285 mM sucrose |
| | | 20 mM sodium chloride | |
| **S10** | 5.0 | 20 mM acetate buffer solution | 142.5 mM sucrose |
| | | 20 mM sodium chloride | |
| **S11** | 5.0 | 20 mM acetate buffer solution | 285 mM trehalose |
| | | 20 mM sodium chloride | |
| **S12** | 5.0 | 20 mM acetate buffer solution | 142.5 mM trehalose |
| | | 20 mM sodium chloride | |
| **S13** | 5.0 | 20 mM acetate buffer solution | 285 mM D-mannitol |
| | | 20 mM sodium chloride | |
| **S14** | 5.0 | 20 mM acetate buffer solution | 142.5 mM D-mannitol |
| | | 20 mM sodium chloride | |
| **S15** | 5.0 | 20 mM acetate buffer solution | 285 mM sorbitol |
| | | 20 mM sodium chloride | |
| **S16** | 5.0 | 20 mM acetate buffer solution | 142.5 mM sorbitol |
| | | 20 mM sodium chloride | |
| **S17** | 6.0 | 20 mM histidine buffer solution | 285 mM sucrose |
| **S18** | 6.0 | 20 mM histidine buffer solution | 142.5 mM sucrose |
| **S19** | 6.0 | 20 mM histidine buffer solution | 285 mM trehalose |
| **S20** | 6.0 | 20 mM histidine buffer solution | 142.5 mM trehalose |
| **S21** | 6.0 | 20 mM histidine buffer solution | 285 mM D-mannitol |
| **S22** | 6.0 | 20 mM histidine buffer solution | 142.5 mM D-mannitol |
| **S23** | 6.0 | 20 mM histidine buffer solution | 285 mM sorbitol |
| **S24** | 6.0 | 20 mM histidine buffer solution | 142.5 mM sorbitol |

The diffusion interaction parameter (KD) and aggregation initiation temperature (Tagg) were measured for the 24 types of samples.

The diffusion interaction parameters were measured and analyzed at 25 °C for the samples at antibody concentrations thereof (1, 2, 4, 8 and 10 mg/mL), and calculated by plotting the diffusion coefficient (µm2/s) for the formulations.

The aggregation initiation temperature was measured and analyzed by increasing the temperature from 25 to 80 °C for the samples at 1 mg/mL of antibody concentration thereof. The agglomeration initiation temperature was defined as the value at which the mean particle size increased by 10 % from the mean particle size at 25 °C by plotting the particle size (nm) in the formulations.

The KD values calculated for the samples are listed in descending order in Table 9.

**[Table 9]**

| **Sample** | **pH** | **Buffer solution, Salt** | **Suger** | **KD (mL/g)** |
|---|---|---|---|---|
| **S3** | 5.0 | 20 mM acetate buffer solution | 285 mM trehalose | 32.5 |
| **S7** | 5.0 | 20 mM acetate buffer solution | 285 mM sorbitol | 31.9 |
| **S4** | 5.0 | 20 mM acetate buffer solution | 142.5 mM trehalose | 28.8 |
| **S11** | 5.0 | 20 mM acetate buffer solution | 285 mM trehalose | 28.2 |
| | | 20 mM sodium chloride | | |
| **S6** | 5.0 | 20 mM acetate buffer solution | 142.5 mM D-mannitol | 26.9 |
| **S5** | 5.0 | 20 mM acetate buffer solution | 285 mM D-mannitol | 25.3 |
| **B4** | 5.0 | 20 mM acetate buffer solution | - | 25.1 |
| **S19** | 6.0 | 20 mM histidine buffer solution | 285 mM trehalose | 21.5 |
| **S14** | 5.0 | 20 mM acetate buffer solution | 142.5 mM D-mannitol | 19.7 |
| | | 20 mM sodium chloride | | |
| **S10** | 5.0 | 20 mM acetate buffer solution | 142.5 mM sucrose | 18.4 |
| | | 20 mM sodium chloride | | |
| **S23** | 6.0 | 20 mM histidine buffer solution | 285 mM sorbitol | 18.4 |
| **S2** | 5.0 | 20 mM acetate buffer solution | 142.5 mM sucrose | 17.4 |
| **B22** | 6.0 | 20 mM histidine buffer solution | - | 17.4 |
| **S21** | 6.0 | 20 mM histidine buffer solution | 285 mM D-mannitol | 16.5 |
| **S15** | 5.0 | 20 mM acetate buffer solution | 285 mM sorbitol | 16.1 |
| | | 20 mM sodium chloride | | |
| **S8** | 5.0 | 20 mM acetate buffer solution | 142.5 mM sorbitol | 15.7 |
| **S1** | 5.0 | 20 mM acetate buffer solution | 285 mM sucrose | 15.6 |
| **S13** | 5.0 | 20 mM acetate buffer solution | 285 mM D-mannitol | 14.9 |
| | | 20 mM sodium chloride | | |
| **S24** | 6.0 | 20 mM histidine buffer solution | 142.5 mM sorbitol | 14.2 |
| **S22** | 6.0 | 20 mM histidine buffer solution | 142.5 mM D-mannitol | 13.6 |
| **S20** | 6.0 | 20 mM histidine buffer solution | 142.5 mM trehalose | 12.2 |
| **B5** | 5.0 | 20 mM acetate buffer solution | - | 12.0 |
| | | 20 mM sodium chloride | | |
| **S16** | 5.0 | 20 mM acetate buffer solution | 142.5 mM sorbitol | 11.0 |
| | | 20 mM sodium chloride | | |
| **S17** | 6.0 | 20 mM histidine buffer solution | 285 mM sucrose | 10.5 |
| **S12** | 5.0 | 20 mM acetate buffer solution | 142.5 mM trehalose | 10.0 |
| | | 20 mM sodium chloride | | |
| **S18** | 6.0 | 20 mM histidine buffer solution | 142.5 mM sucrose | 9.8 |
| **S9** | 5.0 | 20 mM acetate buffer solution | 285 mM sucrose | 8.5 |
| | | 20 mM sodium chloride | | |

The agglomeration initiation temperatures (Tagg) measured for each sample are listed in descending order in Table 10.

**[Table 10]**

| **Sample** | **pH** | **Buffer solution, Salt** | **Suger** | **Tagg (°C)** |
|---|---|---|---|---|
| **S6** | 5.0 | 20 mM acetate buffer solution | 142.5 mM D-mannitol | 66.25 |
| **S23** | 6.0 | 20 mM histidine buffer solution | 285 mM sorbitol | 66.17 |
| **S5** | 5.0 | 20 mM acetate buffer solution | 285 mM D-mannitol | 66.14 |
| **B22** | 6.0 | 20 mM histidine buffer solution | - | 66.09 |
| **S21** | 6.0 | 20 mM histidine buffer solution | 285 mM D-mannitol | 66.07 |
| **S24** | 6.0 | 20 mM histidine buffer solution | 142.5 mM sorbitol | 65.64 |
| **S22** | 6.0 | 20 mM histidine buffer solution | 142.5 mM D-mannitol | 65.43 |
| **B4** | 5.0 | 20 mM acetate buffer solution | - | 65.08 |
| **S17** | 6.0 | 20 mM histidine buffer solution | 285 mM sucrose | 65.07 |
| **S18** | 6.0 | 20 mM histidine buffer solution | 142.5 mM sucrose | 64.97 |
| **S3** | 5.0 | 20 mM acetate buffer solution | 285 mM trehalose | 64.87 |
| **S2** | 5.0 | 20 mM acetate buffer solution | 142.5 mM sucrose | 64.78 |
| **S19** | 6.0 | 20 mM histidine buffer solution | 285 mM trehalose | 64.51 |
| **S4** | 5.0 | 20 mM acetate buffer solution | 142.5 mM trehalose | 64.5 |
| **S1** | 5.0 | 20 mM acetate buffer solution | 285 mM sucrose | 64.48 |
| **S7** | 5.0 | 20 mM acetate buffer solution | 285 mM sorbitol | 64.34 |
| **S9** | 5.0 | 20 mM acetate buffer solution | 285 mM sucrose | 64.22 |
| | | 20 mM sodium chloride | | |
| **S8** | 5.0 | 20 mM acetate buffer solution | 142.5 mM sorbitol | 64.12 |
| **S20** | 6.0 | 20 mM histidine buffer solution | 142.5 mM trehalose | 63.92 |
| **S13** | 5.0 | 20 mM acetate buffer solution | 285 mM D-mannitol | 63.81 |
| | | 20 mM sodium chloride | | |
| **S10** | 5.0 | 20 mM acetate buffer solution | 142.5 mM sucrose | 63.41 |
| | | 20 mM sodium chloride | | |
| **S15** | 5.0 | 20 mM acetate buffer solution | 285 mM sorbitol | 63.08 |
| | | 20 mM sodium chloride | | |
| **S14** | 5.0 | 20 mM acetate buffer solution | 142.5 mM D-mannitol | 62.98 |
| | | 20 mM sodium chloride | | |
| **S11** | 5.0 | 20 mM acetate buffer solution | 285 mM trehalose | 62.88 |
| | | 20 mM sodium chloride | | |
| **S12** | 5.0 | 20 mM acetate buffer solution | 142.5 mM trehalose | 62.67 |
| | | 20 mM sodium chloride | | |
| **B5** | 5.0 | 20 mM acetate buffer solution | - | 62.21 |
| | | 20 mM sodium chloride | | |
| **S16** | 5.0 | 20 mM acetate buffer solution | 142.5 mM sorbitol | 61.89 |
| | | 20 mM sodium chloride | | |

Based on the comprehensive evaluation of the diffusion interaction parameter (KD) and aggregation initiation temperature (Tagg), Samples S3 (20 mM acetate buffer solution (pH 5.0), 285 mM trehalose), S5 (20 mM acetate buffer solution (pH 5.0), 285 mM D-mannitol) and S23 (20 mM histidine buffer solution (pH 6.0), 285 mM sorbitol) were selected. Note that sodium chloride-free formulations were selected because all acetate buffer solutions containing sodium chloride scored low.

### Example 3: Additives evaluation (2)

The 15 types of samples, E1 to E15, were prepared by adding polysorbates (PS) (polysorbate 80 (PS80) and polysorbate 20 (PS20)) to the respective formulations of Samples S3, S5 and S23 selected in the evaluation of Example 2, as shown in Table 11.

**[Table 11]**

| **Sample** | **pH** | **Buffer solution, Salt** | **Suger** | **Polysorbate** |
|---|---|---|---|---|
| **E1** | 5.0 | 20 mM acetate buffer solution | 285 mM trehalose | None |
| **E2** | 5.0 | 20 mM acetate buffer solution | 285 mM trehalose | 0.01 % PS80 |
| **E3** | 5.0 | 20 mM acetate buffer solution | 285 mM trehalose | 0.1 % PS80 |
| **E4** | 5.0 | 20 mM acetate buffer solution | 285 mM trehalose | 0.01 % PS20 |
| **E5** | 5.0 | 20 mM acetate buffer solution | 285 mM trehalose | 0.1 % PS20 |
| **E6** | 5.0 | 20 mM acetate buffer solution | 285 mM D-mannitol | None |
| **E7** | 5.0 | 20 mM acetate buffer solution | 285 mM D-mannitol | 0.01 % PS80 |
| **E8** | 5.0 | 20 mM acetate buffer solution | 285 mM D-mannitol | 0.1 % PS80 |
| **E9** | 5.0 | 20 mM histidine buffer solution | 285 mM D-mannitol | 0.01 % PS20 |
| **E10** | 5.0 | 20 mM histidine buffer solution | 285 mM D-mannitol | 0.1 % PS20 |
| **E11** | 6.0 | 20 mM histidine buffer solution | 285 mM sorbitol | None |
| **E12** | 6.0 | 20 mM histidine buffer solution | 285 mM sorbitol | 0.01 % PS80 |
| **E13** | 6.0 | 20 mM histidine buffer solution | 285 mM sorbitol | 0.1 % PS80 |
| **E14** | 6.0 | 20 mM histidine buffer solution | 285 mM sorbitol | 0.01 % PS20 |
| **E15** | 6.0 | 20 mM histidine buffer solution | 285 mM sorbitol | 0.1 % PS20 |

The 15 types of samples (1 mg/mL) were sterilized with 0.22 µm filters, dispensed into vials, and subjected to the storage test, acceleration test and freeze-thaw test, respectively.

The storage test was conducted at 20 °C and 40 °C for 4 weeks, respectively. The stored samples were evaluated by visual evaluation, concentration, turbidity and SEC-HPLC analysis. Supernatants obtained by centrifugation at 15,000 g for 30 minutes were all used as samples for evaluation. The concentration was calculated based on the absorbance at 280 nm (optical path length 2 mm). The turbidity was calculated based on the absorbance at 500 nm (optical path length 1 cm). SEC-HPLC analysis was performed under the conditions shown in Table 2.

The acceleration test was performed by shaking at 500 times/minutes for 3 days at 20 °C.

The freeze-thaw test was performed by repeating freezing three times (for 10 minutes at -80°C and 10 minutes at 25°C).

All samples immediately after preparation were clear, and no visible particle was observed.

The result of the storage test (for 4 weeks at 20 °C) in Table 12, that of the storage test (for 4 weeks at 40 °C) in Table 13, that of the acceleration test (for 3 days shaking at 20 °C) in Table 14, and that of the freeze-thaw test (for 10 minutes at -80 °C and 10 minutes at 25 °C) in Table 15 are shown, respectively.

**[Table 12]**

| | **Initial stage** | | | **After storage test (20°C, 4 weeks)** | | | | |
|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage, %)** | **Turbidity** | **SEC-HPLC (Monomer area)** | **SEC-HPLC (Rate of monomer reduction. %)** |
| **E1** | 1.01 | 0.0017 | 3241679 | 0.0419 | 4.1 | 0.0030 | 3317090 | -2.33 |
| **E2** | 1.01 | 0.0007 | 3278094 | 1.01 | 100.0 | 0.0013 | 3358820 | -2.46 |
| **E3** | 1.02 | 0.0003 | 3346285 | 1.01 | 99.0 | 0.0010 | 3432042 | -2.56 |
| **E4** | 1 | 0.0010 | 3301324 | 0.996 | 99.6 | 0.0017 | 3366106 | -1.96 |
| **E5** | 1 | 0.0003 | 3391571 | 1 | 100 | 0.0003 | 3436701 | -1.33 |
| **E6** | 1.01 | 0.0003 | 3321244 | 0.0011 | 0.1 | 0.0010 | 3380687 | -1.79 |
| **E7** | 1.02 | 0.0010 | 3371312 | 1.01 | 99 | 0.0003 | 3435324 | -1.90 |
| **E8** | 1 | 0.0020 | 3382366 | 1 | 100.0 | 0.0013 | 3437196 | -1.62 |
| **E9** | 1.02 | 0.0003 | 3428706 | 1 | 98.0 | 0.0013 | 3419499 | 0.27 |
| **E10** | 1.02 | 0.0007 | 3331445 | 1.01 | 99.0 | 0.0003 | 3462965 | -3.95 |
| **E11** | 0.975 | 0.0000 | 3206577 | 0 | 0.0 | 0.0010 | 3235254 | -0.89 |
| **E12** | 0.976 | 0.0010 | 3293023 | 0.979 | 100.3 | 0.0013 | 3336013 | -1.31 |
| **E13** | 0.977 | 0.0010 | 3295242 | 0.977 | 100.0 | 0.0007 | 3379930 | -2.57 |
| **E14** | 0.983 | 0.0007 | 3290613 | 0.977 | 99.4 | 0.0017 | 3349731 | -1.80 |
| **E15** | 0.979 | 0.0017 | 3311911 | 0.977 | 99.8 | 0.0003 | 3305861 | 0.18 |

**[Table 13]**

| | **Initial stage** | | | **After storage test (4°C, 4 weeks)** | | | | |
|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage. %)** | **Turbidity** | **SEC-HPLC (Monomer area)** | **SEC-HPLC (Rate of monomer reduction, %)** |
| **E1** | 1.01 | 0.0017 | 3241679 | 0.999 | 98.9 | 0.0013 | 3166012 | 2.33 |
| **E2** | 1.01 | 0.0007 | 3278094 | 0.998 | 99 | 0.001 | 3193591 | 2.58 |
| **E3** | 1.02 | 0.0003 | 3346285 | 1.02 | 100.0 | 0.0003 | 3360197 | -0.42 |
| **E4** | 1 | 0.0010 | 3301324 | 1 | 100 | 0.0003 | 3313887 | -0.38 |
| **E5** | 1 | 0.0003 | 3391571 | 0.989 | 99 | 0.0007 | 3311961 | 2.35 |
| **E6** | 1.01 | 0.0003 | 3321244 | 1.01 | 100.0 | 0.001 | 3200955 | 3.62 |
| **E7** | 1.02 | 0.0010 | 3371312 | 1 | 98 | 0.0013 | 3242039 | 3.83 |
| **E8** | 1 | 0.0020 | 3382366 | 1.01 | 101.0 | 0.001 | 3231561 | 4.46 |
| **E9** | 1.02 | 0.0003 | 3428706 | 1.01 | 99.0 | 0 | 3223118 | 6.00 |
| **E10** | 1.02 | 0.0007 | 3331445 | 1.02 | 100.0 | 0.0013 | 3381069 | -1.49 |
| **E11** | 0.975 | 0.0000 | 3206577 | 0.97 | 99.5 | 0.001 | 3074218 | 4.13 |
| **E12** | 0.976 | 0.0010 | 3293023 | 0.982 | 100.6 | 0 | 3253861 | 1.19 |
| **E13** | 0.977 | 0.0010 | 3295242 | 0.978 | 100.1 | 0.0007 | 3229981 | 1.98 |
| **E14** | 0.983 | 0.0007 | 3290613 | 0.99 | 100.7 | 0.0007 | 3245870 | 1.36 |
| **E15** | 0.979 | 0.0017 | 3311911 | 0.989 | 101.0 | 0.0007 | 3295443 | 0.50 |

**[Table 14]**

| | **Initial stage** | | | **Acceleration test (20°C, Shaking for 3 days)** | | | | |
|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage, %)** | **Turbidity** | **SEC-HPLC (Monomer area)** | **SEC-HPLC (Rate of monomer reduction. %)** |
| **E1** | 1.01 | 0.0017 | 3241679 | 0.0419 | 4.1485 | 1.314 | 6150 | 99.81 |
| **E2** | 1.01 | 0.0007 | 3278094 | 1.01 | 100.00 | 0.0017 | 2979617 | 9.11 |
| **E3** | 1.02 | 0.0003 | 3346285 | 1.01 | 99.02 | 0 | 3377163 | -0.92 |
| **E4** | 1 | 0.0010 | 3301324 | 0.996 | 99.6 | 0.0013 | 3280635 | 0.63 |
| **E5** | 1 | 0.0003 | 3391571 | 1 | 100.000 | 0.0023 | 3394254 | -0.08 |
| **E6** | 1.01 | 0.0003 | 3321244 | 0.0011 | 0.109 | 0.526 | 1474 | 99.96 |
| **E7** | 1.02 | 0.0010 | 3371312 | 1.01 | 99.020 | 0.0003 | 2990050 | 11.31 |
| **E8** | 1 | 0.0020 | 3382366 | 1 | 100.000 | 0.0007 | 3413786 | -0.93 |
| **E9** | 1.02 | 0.0003 | 3428706 | 1 | 98.039 | 0.001 | 3396603 | 0.94 |
| **E10** | 1.02 | 0.0007 | 3331445 | 1.01 | 99.020 | 0.0007 | 3476404 | -4.35 |
| **E11** | 0.975 | 0.0000 | 3206577 | 0 | 0.000 | 0.287 | 0 | 100.00 |
| **E12** | 0.976 | 0.0010 | 3293023 | 0.979 | 100.307 | 0.0007 | 2405129 | 26.96 |
| **E13** | 0.977 | 0.0010 | 3295242 | 0.977 | 100.000 | 0 | 3345353 | -1.52 |
| **E14** | 0.983 | 0.0007 | 3290613 | 0.977 | 99.390 | 0.0023 | 3079411 | 6.42 |
| **E15** | 0.979 | 0.0017 | 3311911 | 0.977 | 99.796 | 0.0003 | 3367775 | -1.69 |

**[Table 15]**

| | **Initial stage** | | | **After freeze-thaw test** | | | | |
|---|---|---|---|---|---|---|---|---|
| **Sample** | **Concentration (mg/mL)** | **Turbidity** | **SEC-HPLC (Monomer area)** | **Concentration (mg/mL)** | **Concentration (After shaking/ Initial stage. %)** | **Turbidity** | **SEC-HPLC (Monomer area)** | **SEC-HPLC (Rate of monomer reduction. %)** |
| **E1** | 1.01 | 0.0017 | 3241679 | 0.992 | 98.2 | 0.0157 | 3248774 | -0.22 |
| **E2** | 1.01 | 0.0007 | 3278094 | - | - | - | - | - |
| **E3** | 1.02 | 0.0003 | 3346285 | - | - | - | - | - |
| **E4** | 1 | 0.0010 | 3301324 | - | - | - | - | - |
| **E5** | 1 | 0.0003 | 3391571 | - | - | - | - | - |
| **E6** | 1.01 | 0.0003 | 3321244 | 0.992 | 98.2 | 0.025 | 3275972 | 1.36 |
| **E7** | 1.02 | 0.0010 | 3371312 | - | - | - | - | - |
| **E8** | 1 | 0.0020 | 3382366 | - | - | - | - | - |
| **E9** | 1.02 | 0.0003 | 3428706 | - | - | - | - | - |
| **E10** | 1.02 | 0.0007 | 3331445 | - | - | - | - | - |
| **E11** | 0.975 | 0.0000 | 3206577 | 0.964 | 98.9 | 0.0197 | 3004540 | 6.30 |
| **E12** | 0.976 | 0.0010 | 3293023 | - | - | - | - | - |
| **E13** | 0.977 | 0.0010 | 3295242 | - | - | - | - | - |
| **E14** | 0.983 | 0.0007 | 3290613 | - | - | - | - | - |
| **E15** | 0.979 | 0.0017 | 3311911 | - | - | - | - | - |

As the result of the acceleration test shown in Table 14, the aggregation stability was clearly low in Samples E1, E6, and E11, which are formulations without polysorbate, confirming that polysorbate is essential. Furthermore, as results of the comprehensive evaluation of the present examples, as formulations to proceed to the next evaluation, Samples E3 (20 mM acetate buffer solution (pH 5.0), 285 mM trehalose, 0.1 % PS80), E4 (20 mM acetate buffer solution (pH 5.0), 285 mM trehalose, 0.01 % PS20), E10 (20 mM acetate buffer solution (pH 5.0), 285 mM D-mannitol, 0.1 % PS20) and E13 (20 mM histidine buffer solution (pH 6.0), 285 mM sorbitol, 0.1 % PS80) were selected.

### Example 4: Stability evaluation test (1)

For the acetate formulation (20 mM acetate buffer solution (pH 5.0), 285 mM trehalose, 0.1 % PS80) and the histidine formulation (20 mM histidine buffer solution (pH 6.0), 0.1 % PS80) selected for the evaluation in Example 3, samples containing 100 mg/mL of Antibody α were prepared.

The samples were sterilized with 0.22 µm filters, 1 mL each dispensed into sterilized vials, and subjected to the condition shown in Table 16, and then the stability evaluation test was conducted for each sample.

**[Table 16]**

| **Test items** | **Condition** |
|---|---|
| **Shaking** | Shaked at room temperature (20° C) for 3 days |
| **F/T** | Frozen and thawed (-80±5° C x 10 min, 25±5° C x 10 min) x 3 times |
| **5°C/1M** | Stored at 5° C for 4 weeks |
| **25°C/1M** | Stored at 25° C for 4 weeks |
| **40°C/1M** | Stored at 40° C for 4 weeks |
| **5°C/3M** | Stored at 5° C for 12 weeks |
| **25°C/3M** | Stored at 25° C for 12 weeks |
| **40°C/3M** | Stored at 40° C for 12 weeks |

The samples subjected to the conditions shown in the above table were evaluated by (a) pH measurement, (b) antibody concentration measurement, (c) turbidity measurement, (d) SEC-HPLC (size exclusion chromatography) analysis, (e) SDS-PAGE analysis, (f) IE-HPLC (ion exchange chromatography) analysis, (g) viscosity measurement, (h) FIA (flow imaging analysis) and (i) sliding resistance measurement, respectively.
(a) ApH meter (LAQUAF-74 (HORIBA)) and a trace sample measuring electrode (Micro Tou pH electrode (HORIBA)) were used for pH measurement.
(b) For supernatants obtained by centrifuging the samples at 15,000 g for 30 minutes, the antibody concentrations were measured based on the absorbance at 280 nm with 1 cm or 2 mm optical path length. Note that the samples were diluted so that the absorbance thereof is from 0.1 to 1.0, and the concentration was calculated from the absorbance coefficient of antibody, ε280 = 1.51 (mg/mL)⁻¹cm⁻¹. Note that a spectrophotometer (BioSpectrometer (Eppendorf)) was used for these measurements.
(c) In the turbidity measurement, the absorbance at 500 nm was measured at 1 cm optical path length. Note that a spectrophotometer was used for this measurement.
(d) SEC-HPLC analysis was performed under the condition shown in Table 3.
(e) SDS-PAGE analysis was performed by using supernatants obtained by centrifuging the samples at 15,000 g for 30 minutes and subjecting 2 µg of the samples to electrophoresis on 4 to 12 % Bis-Tris gel at 100 V for about 30 minutes, under reducing (100 mM DTT) and non-reducing (-DTT) conditions.
(f) IE-HPLC analysis was performed under the condition shown in Table 17.

**[Table 17]**

| **Items** | **Details** |
|---|---|
| Column | MabPacSCX-10 |
| Sample | 1 mg/mL, 20 uL |
| Column Temperature | 30°C |
| Flow rate | 0.7 mL/min |
| Mobile phase A | CX-1 pH Gradient Buffer A pH 5.6 |
| Mobile phase B | CX-1 pH Gradient Buffer B pH 10.2 |
| Measurement time | 30 min |
| Gradient condition | 50 % B in 1 min, 50 to 80 % B in 45 min |
| | 80 to 50 % B in 0.1 min, 50% in 9 min |
| UV detection | 215, 280 nm |

(g) In the viscosity measurement, for supernatansts obtained by centrifuging the samples at 15,000 g for 30 minutes, the viscosities thereof were measured at 20 °C. Note that the density was set at 1.0 g/cm³ and the relative viscosities were determined.

(h) Sliding resistance measurement: Supernatants obtained by centrifugation at 15,000 g for 30 minutes were used as measurement samples. 400 µL of sample was filled in 29G staked 1 mL COP syringe and allowed to stand for about 5 hours. Using an autograph, the syringe was pushed out at 6 mL/minute and the sliding resistance was measured. Note that the sliding resistance measurement was performed only on samples in their initial state.

(i) In FIA, 150 µL of samples were measured using a flow imaging device at a flow rate of 0.05 mL/minute and evaluated for aggregates larger than 1 µm in size.

### (Results in the stability evaluation)

Samples before and after the stability evaluation test were visually inspected. Both samples of the acetate formulation and histidine formulation in the initial state were yellow in color, but under the respective conditions after 4-weeks and 12-weeks of storages and after shaking for 3 days, there is no apparent change in color. In samples of the histidine formulation, particulates were observed after 4-weeks of storage at 5 °C and after 12-weeks of storages at 5 °C and 25 °C, respectively. In addition, in both samples of the acetate formulation and histidine formulation after 4-weeks of storage at 40 °C, no particulate was showed, but they were entirely cloudy white.
(a) In all samples after the stability evaluation test, no significant pH change was observed.
(b) In all samples after the stability test, no significant concentration change was observed.
(c) The results of turbidity measurements before and after the stability test are shown in Table 18.

**[Table 18]**

| **Samples** | | **Turbidity (500 nm absorbance)** |
|---|---|---|
| | **Initial** | 0.059 |
| | **5°C 1M** | 0.058 |
| | **25°C 1M** | 0.060 |
| | **40°C 1M** | 0.071 |
| Acetate formulation | **Shaking** | 0.061 |
| | **F/T** | 0.059 |
| | **5°C 3M** | 0.064 |
| | **25°C 3M** | 0.066 |
| | **40°C 3M** | 0.090 |
| | **Initial** | 0.066 |
| | **5°C 1M** | 0.073 |
| | **25°C 1M** | 0.111 |
| | **40°C 1M** | 0.093 |
| Histidine formulation | **Shaking** | 0.073 |
| | **F/T** | 0.075 |
| | **5°C 3M** | 0.125 |
| | **25°C 3M** | 0.171 |
| | **40°C 3M** | 0.136 |

After 4-weeks of storage, the storage of the acetate formulation sample at 40 °C and storages of the histidine formulation samples at 25 °C and 40 °C increased the turbidities by more than 20 % compared to that in the initial state ("Initial" in the table). The storage of the acetate formulation sample at 40 °C for 12 weeks increased the turbidity by about 50 % compared to that in the initial state. The storages of the histidine formulation samples at 5 °C, 25 °C and 40 °C increased the turbidities by more than 90 % compared to that in the initial state.
(d) The measurement results of SEC-HPLC analysis before and after the stability test are shown in Table 19.

**[Table 19]**

| **Samples** | | **SEC-HPLC** | | | |
|---|---|---|---|---|---|
| | | **Monomer area** | **Monomer (%)** | **HMWS (%)** | **LMWS (%)** |
| | **Initial** | 2135977 | 95.5 | 3.89 | 0.073 |
| | **5°C 1M** | 2145925 | 99.6 | 0.41 | 0 |
| | **25°C 1M** | 2129841 | 99.4 | 0.63 | 0.015 |
| | **40°C 1M** | 2045384 | 98.2 | 0.64 | 1.17 |
| Acetate formulation | **Shaking** | 2202008 | 95.8 | 4.16 | 0 |
| | **F/T** | 2129255 | 97.1 | 2.87 | 0 |
| | **5°C 3M** | 2171159 | 98.5 | 1.5 | 0.005 |
| | **25°C 3M** | 2124974 | 98.9 | 1.02 | 0.060 |
| | **40°C 3M** | 2071561 | 97.1 | 1.81 | 1.11 |
| | **Initial** | 2149010 | 95.4 | 4.29 | 0.29 |
| | **5°C 1M** | 2251185 | 99.6 | 0.42 | 0.015 |
| | **25°C 1M** | 2145830 | 99.1 | 0.63 | 0.27 |
| | **40°C 1M** | 2128661 | 97.6 | 1.76 | 0.61 |
| Histidine formulation | **Shaking** | 2121867 | 96.1 | 3.71 | 0.17 |
| | **F/T** | 2155511 | 96.7 | 3.33 | 0 |
| | **5°C 3M** | 2182721 | 98.4 | 1.46 | 0.17 |
| | **25°C 3M** | 2112850 | 97.8 | 1.48 | 0.75 |
| | **40°C 3M** | 2063642 | 97.4 | 1.51 | 1.05 |

SEC-HPLC analysis showed that the monomer (monomer area) decreased by 3 to 5 % in all samples of the acetate formulation after 4- and 12-weeks of storages at 40 °C. In the histidine formulation sample, 12-weeks storages at 25°C and 40 °C decreased the monomer derived from the same antibody by 2 to 5 % and 4 %, respectively. It was assumed that these reduced monomers converted to high molecular weight species (HMWS) and the like due to antibody aggregation. Few effects of shaking and freeze-thaw were observed.
(e) SDS-PAGE analysis before and after the stability test showed that bands similar to the standard product were observed in the initial state and in each sample under both reducing and non-reducing conditions, confirming that no degradation occurred during sample preparation such as purification and concentration.
(f) The results of IE-HPLC analysis before and after the stability test are shown in Table 20.

**[Table 20]**

| **Samples** | | IE | | |
|---|---|---|---|---|
| | | **Acidic molecular species (%)** | **Neutral molecular species (%)** | **Basic molecular species (%)** |
| | **Initial** | 40.4 | 59.6 | 0 |
| | **5°C 1M** | 42.4 | 57.6 | 0 |
| | **25°C 1M** | 42.8 | 57.2 | 0 |
| | **40°C 1M** | 55.0 | 40.3 | 4.70 |
| Acetate formulation | **Shaking** | 41.1 | 58.9 | 0 |
| | **F/T** | 40.8 | 59.2 | 0 |
| | **5°C 3M** | 42.6 | 57.4 | 0.005 |
| | **25°C 3M** | 49.7 | 50.3 | 0.045 |
| | **40°C 3M** | 73.4 | 22.7 | 3.98 |
| | **Initial** | 40.8 | 59.2 | 0 |
| | **5°C 1M** | 42.5 | 57.6 | 0 |
| | **25°C 1M** | 43.8 | 56.2 | 0 |
| | **40°C 1M** | 55.6 | 44.4 | 0 |
| Histidine formulation | **Shaking** | 41.5 | 58.5 | 0 |
| | **F/T** | 41.6 | 58.4 | 0 |
| | **5°C 3M** | 43.6 | 56.3 | 0.02 |
| | **25°C 3M** | 48.9 | 50.9 | 0.28 |
| | **40°C 3M** | 72.5 | 27.4 | 0.04 |

Both samples of the acetate formulation and histidine formulation showed little effect by any of the conditions of 4-weeks storage and 12-weeks storage at 5 °C, shaking, and freeze-thaw.

Under the condition of 4-weeks storage at 25 °C, the histidine formulation sample showed 3 % increase in acidic molecular species and 3 % decrease in neutral molecular species.

Under the condition of 4-weeks storage at 40 °C, the acetate formulation sample showed 15 % increase in acidic molecular species, 19 % decrease in neutral molecular species, and 5 % increase in basic molecular species. The histidine formulation sample also showed 15% increase in acidic molecular species and 15 % decrease in neutral molecular species.

Under the condition of 12-weeks storage at 25 °C, the acetate formulation sample showed 9 % increase in acidic molecular species and 9 % decrease in neutral molecular species. The histidine formulation sample also showed 8 % increase in acidic molecular species and 8% decrease in neutral molecular species.

Under the condition of 12-weeks storage at 40 °C, the acetate formulation sample showed 33 % increase in acidic molecular species, 37 % decrease in neutral molecular species, and 4 % increase in basic molecular species. The histidine formulation sample also showed 32 % increase in acidic molecular species and 32 % decrease in neutral molecular species.

The decrease in neutral molecular species was thought to mainly change into acidic molecular species due to oxidation.

The results under 4-weeks storage at 25 °C indicated that the acetate formulation was more stable. (g) The results of the viscosity measurement before and after the stability evaluation test showed that in all formulations, there was no significant change in viscosity before and after the stability evaluation test.

Note that the viscosity of the sample in the initial state was 4.41 mPa s for the acetate formulation sample and 4.2 mPa s for the histidine formulation sample, which are very low values even at a concentration of 100 mg/mL.
(h) The results of the sliding resistance measurement for the sample in the initial state showed very low sliding resistance values (7.66 N and 7.46 N) for both samples of the acetate formulation and the histidine formulation.
(i) The particle concentration obtained by subtracting the particle concentration in formulation solution from the particle concentration in each formulation containing the antibody before and after the stability evaluation test is shown in Table 21.

**[Table 21]**

| **Samples** | | **Concentration of particles in sample minus concentration of particles in solvent (particles/mL)** | | | | | |
|---|---|---|---|---|---|---|---|
| | | **Total** | **1-2 *µ*m** | **2-5 *µ*m** | **5-10 *µ*m** | **10 - 20 *µ*m** | **20 < *µ*m** |
| | **Initial** | 694 | 260 | 51 | 72 | 51 | 48 |
| | **5°C 1M** | 455 | 276 | 10 | 41 | 26 | 0 |
| | **25°C 1M** | 467 | 307 | -138 | 57 | 21 | 16 |
| | **40°C 1M** | 615 | 283 | -98 | 87 | 0 | 6 |
| Acetate formulation | **Shaking** | -817 | -639 | -427 | -16 | -14 | -24 |
| | **F/T** | 789 | 79 | -1,237 | 147 | 106 | -21 |
| | **5°C 3M** | 1,850 | 635 | 277 | 287 | 72 | 10 |
| | **25°C 3M** | 2.158 | 1,159 | 72 | 124 | 67 | 41 |
| | **40°C 3M** | 3.496 | 1.666 | 221 | 252 | 21 | 15 |
| | **Initial** | 17,670 | 8,416 | 7,221 | 1,425 | 496 | 113 |
| | **5°C 1M** | 24,694 | 10,848 | 6,459 | 2,408 | 2.141 | 2,839 |
| | **25°C 1M** | 51,969 | 25,333 | 11.294 | 5,151 | 5,644 | 4,548 |
| | **40°C 1M** | 161,331 | 85,343 | 53,011 | 12,975 | 6,327 | 3,676 |
| Histidine formulation | **Shaking** | 37,389 | 16,835 | 13,243 | 4,461 | 2,375 | 475 |
| | **F/T** | 17,379 | 10,767 | 6,171 | 816 | -157 | -217 |
| | **5°C 3M** | 29,185 | 12,698 | 9,594 | 3,439 | 2,266 | 1,189 |
| | **25°C 3M** | 5,383,794 | 2,165,937 | 3,103,558 | 68,699 | 25,050 | 20,550 |
| | **40°C 3M** | 60.627 | 33.799 | 21.360 | 3.778 | 1.216 | 528 |

The acetate formulation sample under the condition of 12-weeks storage at 40 °C, and the histidine formulation sample under the condition of 12-weeks storage at 25 °C had the highest particle concentration.

Under all conditions evaluated, the particle concentration of the histidine formulation was tens to thousands of times higher than that of the acetate formulation.

The results of the stability evaluation presented in the present example indicated that the acetate formulation (20 mM acetate buffer solution (pH 5.0), 285 mM trehalose, 0.1 % PS80) was considered more suitable as a formulation for Antibody α pertaining to the present invention.

### Example 5: Additives evaluation (3)

Further optimization studies were conducted on the acetate formulation (20 mM acetate buffer solution (pH 5.0), 285 mM trehalose, 0.1 % PS80) selected based on the evaluation in Example 4.

Salt concentrations were examined to ensure the stability of conductivity.

The 8 types of samples, N1 to N8, were prepared by adding sodium chloride to the selected acetate formulation (20 mM acetate buffer solution (pH 5.0), 285 mM trehalose, 0.1 % PS80) as shown in Table 22.

**[Table 22]**

| **Samples** | **Antibody concentration (mg/mL)** | **pH** | **Buffer solution, salt** | **Suger** | **Polysorbate** | **Conductivity of prepared formulation (mS/cm)** |
|---|---|---|---|---|---|---|
| **N1** | 20 | 5.0 | 20 mM acetate buffer solution | 285 mM trehalose | 0.1 % PS80 | 0.9 |
| **N2** | 20 | 5.0 | 20 mM acetate buffer solution | 285 mM trehalose | 0.1 % PS80 | 2.6 |
| **N3** | 20 | 5.0 | 20 mM acetate buffer solution | 285 mM trehalose | 0.1 % PS80 | 2.8 |
| **N4** | 20 | 5.0 | 20 mM acetate buffer solution | 285 mM trehalose | 0.1 % PS80 | 2.9 |
| **N5** | 100 | 5.0 | 20 mM acetate buffer solution | 285 mM trehalose | 0.1 % PS80 | 1.0 |
| **N6** | 100 | 5.0 | 20 mM acetate buffer solution | 285 mM trehalose | 0.1 % PS80 | 2.6 |
| **N7** | 100 | 5.0 | 20 mM acetate buffer solution | 285 mM trehalose | 0.1 % PS80 | 2.8 |
| **N8** | 100 | 5.0 | 20 mM acetate buffer solution | 285 mM trehalose | 0.1 % PS80 | 3.1 |

The 8 types of samples were sterilized with 0.22 µm filters, dispensed into sterile vials of 1 or 5 mL each, and evaluated for stability under the respective conditions listed in Table 23.

**[Table 23]**

| **Test items** | **Conditions** |
|---|---|
| **5°C/1M** | Stored at 5° C for 4 weeks |
| **25°C/1M** | Stored at 25° C for 4 weeks |
| **40°C/1M** | Stored at 40° C for 4 weeks |
| **5°C/3M** | Stored at 5° C for 12 weeks |
| **25°C/3M** | Stored at 25° C for 12 weeks |
| **40°C/3M** | Stored at 40° C for 12 weeks |
| **Shaking** | 7 days at room temperature and 3 days at 5° C |
| **F/T** | Frozen and thawed (frozen at -80° C and thawed at room temperature) x 3 times |
| **Flowing through syringe** | Samples were flowed through 4 types of injection needles. |
| | Needle size 25G / inner diameter 0.400 mm / length 25 mm |
| | Needle size 27G / inner diameter 0.300 mm / length 25 mm |
| | Needle size 30G / inner diameter 0.198 mm / length 8 mm |
| | Needle size 25G / inner diameter 0.090 mm / length 8 mm |

Samples subjected to the conditions listed in the above table were evaluated by (a) SEC-HPLC analysis, (b) IE-HPLC analysis, (c) RP-HPLC (reverse phase chromatography) analysis, (d) the binding activity, (e) FIA, and (f) the peptide mapping analysis (analysis of denatured sites by liquid chromatography mass spectrometry (LC-MS)), respectively.

Note that (a) SEC-HPLC analysis was performed under the condition shown in Table 24.

**[Table 24]**

| **Items** | **Details** |
|---|---|
| **Column** | TSKgel G3000SWXL |
| **Sample** | 1 mg/mL, 20 uL |
| **Column Temperature** | 25°C |
| **Flow Rate** | 0.5 mL/min |
| **Mobile phase** | 50 mM Sodium phosphate, 300 mM Sodium chloride, pH 7.0 |
| **Measurement time** | 30 min |
| **UV detection** | 214 nm |

(b) IE-HPLC analysis was performed under the condition in Table 25.

**[Table 25]**

| **Items** | **Details** |
|---|---|
| **Column** | Pro Pac WCX-10 (250 x 4 mm I.D.) |
| **Sample** | 1 mg/mL, 20 uL |
| **Column Temperature** | 30°C |
| **Flow rate** | 1.0 mL/min |
| **Mobile phase A** | 9.6 mM Tris, 6 mM Piperazin, 11 mM Imidazol, 50 mM NaCl (pH 6.0) |
| **Mobile phase B** | 9.6 mM Tris, 6 mM Piperazin, 11 mM Imidazol, 100 mM NaCl (pH 10.0) |
| **Measurement time** | 50 min |
| **Gradient condition** | 0 % B in 5 min,0 to 100 % B in 30 min 100% B in 5 min, 100 to 0 % B in 0.1 min, 0% B in 10 min |
| **UV detection** | 280 nm |

(c) RP-HPLC analysis was performed under the condition shown in Table 26.

Note that the value obtained from (fluorescence intensity value) / (UV 280 nm absorbance area value) × 100 was used as the coloring index. A ratio of the coloring index to that of the standard product ((coloring index of a sample) / (coloring index of the standard product)) was used as an index value indicating the amount of colored entities in a sample.

**[Table 26]**

| **Items** | **Details** |
|---|---|
| **Column** | Aeris WIDEPORE XB-C8 150 2.1 mm I.D. |
| **Sample** | 1 mg/mL, 20 uL |
| **Column Temperature** | 75°C |
| **Flow rate** | 0.33 mL/min |
| **Mobile phase A** | Water : Isopropyl alcohol = 98: 2, 0.1 % TFA |
| **Mobile phase B** | Water : Isopropyl alcohol : Acetonitrile = 7 : 2 : 1, 0.1 % TFA |
| **Measurement time** | 33 min |
| **Gradient condition** | 10 % B in 1.5 min, 10 to 25 % B in 1.5 min, 25 to 40 % B in 15.75 min, 40 to 100 % B in 0.1 min, 100 % B in 4.5 min, 100 to 10 % B in 0.1 min, 10 % B in 10 min |
| **UV detection** | 280 nm |
| **Fluorescence detection** | Ex: 380nm. Em: 560 nm |

(d) The binding activities to PD-1 and CD3 were measured by surface plasmon resonance (SPR).

(e) In FIA, aggregates larger than 2 µm in size were evaluated by measuring 500 µL of a sample at a flow rate of 0.1 mL/min using a flow imaging system.

(f) The peptide mapping analysis was performed using LC-MS.

### (Storage test)

(a) The percentages of high-molecular weight species (HMWS) before and after the storage test, evaluated by SEC-HPLC analysis, are shown in Table 27. At all concentrations, no significant increase in HMWS was observed under 12-weeks storage at 5 °C. At 25 °C, no increase in HMWS was observed in the formulations at 20 mg/mL antibody concentration, but a linear increasing trend in HMWS was observed in the formulations at 100 mg/mL. On the other hand, no difference in behavior with the conductivity was observed. At 40 °C, HMWS increased at all concentrations, but formulations at 20 mg/mL showed a trend of increasing HMWS along with increasing conductivity. At 5 °C and 25 °C, the formulation was found to be stable.

**[Table 27]**

| **Samples** | **Concentration (mg/mL)** | **Conductivity (mS/cm)** | **5°C** | | | **25°C** | | | **40°C** | | |
|---|---|---|---|---|---|---|---|---|---|---|---|
| | | | **OM** | **1M** | **3M** | **0M** | **1M** | **3M** | **0M** | **1M** | **3M** |
| **N1** | 20 | 0.9 | 1.0 | 0.9 | 0.9 | 1.0 | 0.8 | 0.8 | 1.0 | 0.7 | 1.2 |
| **N2** | | 2.6 | 1.0 | 0.9 | 1.0 | 1.0 | 0.9 | 1.1 | 1.0 | 1.3 | 2.5 |
| **N3** | | 2.8 | 1.0 | 1.0 | 1.0 | 1.0 | 0.8 | 1.0 | 1.0 | 1.2 | 2.0 |
| **N4** | | 3.0 | 1.0 | 0.9 | 1.0 | 1.0 | 0.9 | 1.1 | 1.0 | 1.5 | 2.9 |
| **N5** | 100 | 0.9 | 1.1 | 1.1 | 1.2 | 1.1 | 1.3 | 1.7 | 1.1 | 2.1 | 3.1 |
| **N6** | | 2.6 | 1.1 | 1.1 | 1.2 | 1.1 | 1.4 | 1.9 | 1.1 | 2.3 | 3.0 |
| **N7** | | 2.8 | 1.1 | 1.1 | 1.2 | 1.1 | 1.4 | 1.8 | 1.1 | 2.3 | 3.0 |
| **N8** | | 3.0 | 1.1 | 1.2 | 1.2 | 1.1 | 1.4 | 1.8 | 1.1 | 2.3 | 3.0 |

(b) The percentages of acidic molecular species before and after the storage test, evaluated by IE-HPLC analysis, are shown in Table 28.

**[Table 28]**

| **Samples** | **Concentration (mg/mL)** | **Conductivity (mS/cm)** | **5°C** | | | **25°C** | | | **40°C** | | |
|---|---|---|---|---|---|---|---|---|---|---|---|
| | | | **0M** | **1M** | **3M** | **0M** | **1M** | **3M** | **0M** | **1M** | **3M** |
| **N1** | 20 | 0.9 | 44.1 | 44.6 | 42.7 | 44.1 | 45.5 | 46.5 | 44.1 | 56.5 | 74.6 |
| **N2** | | 2.6 | 44.1 | 44.3 | 42.5 | 44.1 | 45.1 | 46.3 | 44.1 | 56.2 | 73.9 |
| **N3** | | 2.8 | 44.0 | 44.4 | 42.5 | 44.0 | 45.3 | 46.1 | 44.0 | 56.0 | 73.0 |
| **N4** | | 3.0 | 43.8 | 44.4 | 42.6 | 43.8 | 45.3 | 46.3 | 43.8 | 56.2 | 74.2 |
| **N5** | 100 | 0.9 | 43.9 | 44.5 | 42.7 | 43.9 | 45.3 | 46.2 | 43.9 | 55.9 | 73.1 |
| **N6** | | 2.6 | 43.8 | 44.4 | 42.4 | 43.8 | 44.8 | 45.6 | 43.8 | 55.2 | 71.1 |
| **N7** | | 2.8 | 43.9 | 44.2 | 42.5 | 43.9 | 44.8 | 45.6 | 43.9 | 54.6 | 70.8 |
| **N8** | | 3.0 | 44.0 | 44.2 | 42.4 | 44.0 | 44.8 | 45.5 | 44.0 | 54.7 | 70.7 |

At 40 °C, acidic molecular species tended to increase, but at 5 °C and 25 °C, no increase in acidic molecular species was observed, confirming that the formulation was stable. In addition, no effect of the conductivity was observed.
(c) The percentages of colored entities before and after the storage test, evaluated by RP-HPLC analysis, are shown in Table 29.

**[Table 29]**

| **Samples** | **Concentration (mg/mL)** | **Conductivity (mS/cm)** | **5°C** | | | **25°C** | | | **40°C** | | |
|---|---|---|---|---|---|---|---|---|---|---|---|
| | | | **0M** | **1M** | **3M** | **0M** | **1M** | **3M** | **0M** | **1M** | **3M** |
| **N1** | 20 | 0.9 | 0.87 | 0.87 | 0.89 | 0.87 | 0.94 | 1.01 | 0.87 | 1.09 | 1.29 |
| **N2** | | 2.6 | 0.86 | 0.87 | 0.88 | 0.86 | 0.93 | 1.01 | 0.86 | 1.09 | 1.29 |
| **N3** | | 2.8 | 0.86 | 0.88 | 0.89 | 0.86 | 0.93 | 1.00 | 0.86 | 1.08 | 1.27 |
| **N4** | | 3.0 | 0.85 | 0.87 | 0.88 | 0.85 | 0.93 | 1.00 | 0.85 | 1.09 | 1.33 |
| **N5** | 100 | 0.9 | 0.88 | 0.89 | 0.90 | 0.88 | 0.94 | 1.02 | 0.88 | 1.11 | 1.38 |
| **N6** | | 2.6 | 0.87 | 0.88 | 0.90 | 0.87 | 0.94 | 1.03 | 0.87 | 1.11 | 1.36 |
| **N7** | | 2.8 | 0.87 | 0.89 | 0.90 | 0.87 | 0.94 | 1.03 | 0.87 | 1.10 | 1.37 |
| **N8** | | 3.0 | 0.87 | 0.89 | 0.89 | 0.87 | 0.94 | 1.02 | 0.87 | 1.11 | 1.37 |

While there was no change at 5 °C, increases in the percentage of colored entities were observed at 25 °C and 40 °C. No difference in the conductivity was observed.
(d) The results of the binding activities to PD-1 before and after the storage test in Table 30 and those of the binding activities to CD3 in Table 31, evaluated by SPR, are shown, respectively. The values in each table represent the specific activity (%) of Antibody a to the standard product. No decrease in binding activity of both PD-1 and CD3 was observed at 5°C and 25°C. On the other hand, the decrease in binding activity to PD-1 was observed at 40 °C, and the decrease in the binding activity was more significant at 20 mg/mL antibody concentration.

**[Table 30]**

| **Samples** | **Concentration (mg/mL)** | **Conductivity (mS/cm)** | **5°C** | | | **25°C** | | | **40°C** | | |
|---|---|---|---|---|---|---|---|---|---|---|---|
| | | | **0M** | **1M** | **3M** | **0M** | **1M** | **3M** | **0M** | **1M** | **3M** |
| **N1** | 20 | 0.9 | 92 | 90 | 96 | 92 | 85 | 84 | 92 | 82 | 65 |
| **N2** | | 2.6 | 95 | 99 | 105 | 95 | 88 | 90 | 95 | 80 | 60 |
| **N3** | | 2.8 | 96 | 94 | 100 | 96 | 84 | 92 | 96 | 79 | 63 |
| **N4** | | 3.0 | 94 | 93 | 102 | 94 | 87 | 93 | 94 | 81 | 56 |
| **N5** | 100 | 0.9 | 98 | 99 | 105 | 98 | 94 | 94 | 98 | 92 | 79 |
| **N6** | | 2.6 | 97 | 96 | 87 | 97 | 89 | 83 | 97 | 91 | 77 |
| **N7** | | 2.8 | 101 | 92 | 97 | 101 | 94 | 83 | 101 | 94 | 78 |
| **N8** | | 3.0 | 95 | 92 | 99 | 95 | 87 | 90 | 95 | 90 | 71 |

**[Table 31]**

| **Samples** | **Concentration (mg/mL)** | **Conductivity (mS/cm)** | **5°C** | | | **25°C** | | | **40°C** | | |
|---|---|---|---|---|---|---|---|---|---|---|---|
| | | | **0M** | **1M** | **3M** | **0M** | **1M** | **3M** | **0M** | **1M** | **3M** |
| **N1** | 20 | 0.9 | 92 | 89 | 97 | 92 | 85 | 87 | 92 | 86 | 82 |
| **N2** | | 2.6 | 95 | 98 | 107 | 95 | 89 | 95 | 95 | 88 | 85 |
| **N3** | | 2.8 | 96 | 93 | 101 | 96 | 84 | 96 | 96 | 85 | 84 |
| **N4** | | 3.0 | 94 | 92 | 104 | 94 | 87 | 97 | 94 | 90 | 83 |
| **N5** | 100 | 0.9 | 98 | 98 | 107 | 98 | 94 | 97 | 98 | 96 | 88 |
| **N6** | | 2.6 | 97 | 95 | 88 | 97 | 90 | 87 | 97 | 95 | 85 |
| **N7** | | 2.8 | 101 | 92 | 99 | 101 | 95 | 87 | 101 | 98 | 87 |
| **N8** | | 3.0 | 95 | 91 | 101 | 95 | 87 | 94 | 95 | 94 | 80 |

(e) The number of particles in Samples N5 and N8 after the storage test were evaluated by FIA, respectively, but no significant difference in the number of particles or maximum number of particles was observed among the samples.

### (Shaking test)

(a) The percentage of HMWS before and after the shaking test was evaluated by SEC-HPLC, but no significant increase in HMWS was observed in all samples and conditions.
(b) The percentage of acidic molecular species before and after the shaking test was evaluated by IE-HPLC, but no significant increase in acidic molecular species was observed in all samples and conditions.
(c) The percentage of colored entities before and after the shaking test was evaluated by RP-HPLC, but no significant increase in colored entities was observed in all samples and conditions.
(d) The binding activities to PD-1 and CD3 before and after the shaking test were evaluated by SPR, respectively, but no significant difference in the decrease in the binding activities was observed among the samples.
(e) The number of particles in Samples N5 and N8 after the shaking test was evaluated by FIA, but no increase in the number of particles or maximum particle size due to shaking stress was observed in either sample.

### (Freeze-thaw)

(a) The percentage of HMWS before and after the freeze-thaw test was evaluated by SEC-HPLC, but all samples showed no significant change in the percentage of HMWS and were found to be stable to freeze-thaw stress.
(b) The percentage of acidic molecular species before and after the freeze-thaw test was evaluated by IE-HPLC, but all samples showed no significant change in the percentage of acidic molecular species and were similarly found to be stable to freeze-thaw stress.
(c) The percentage of colored entities before and after the freeze-thaw test was evaluated by RP-HPLC, but no significant change in the percentage of colored entities was also observed in all samples.
(d) The binding activities to PD-1 and CD3 before and after the freeze-thaw test were evaluated by SPR, but no significant change in the binding activities was observed in all samples.
(e) The number of particles in Samples N5 and N8 after the freeze-thaw test was evaluated by FIA, but no increase in the number of particles or maximum particle size due to freeze-thaw stress was observed in either sample. There were no significant differences among the samples evaluated.

### (Syringe flow test)

The percentage (%) of HMWS in Sample N8 before and after the syringe flow test was evaluated by SEC-HPLC, but no generation of HMWS was observed.

### (peptide mapping analysis)

As shown in Table 30, the factorial analysis by peptide mapping was performed on the decrease in binding activity to PD-1 by long-term storage at 40 °C. As a representative example, the oxidation degree (%) to the 101st methionine residue in the complementarity determining region for PD-1 (hereinafter may be abbreviated as "Met101") is shown in Table 32. At 5 °C and 25 °C, the oxidation degree to Met101 was low, and no differences were observed among the samples. In addition, significant oxidation was observed at 20 mg/mL compared to the case at 100 mg/mL antibody concentration. As shown in Figure 4, there was a correlation between the oxidation of Met101 and decrease in the binding activity to PD-1. It was recognized that such oxidation may be a cause of the decrease in binding activity.

**[Table 32]**

| **Samples** | **Concentration (mg/mL)** | **Conductivity (mS/cm)** | **Test items** | **Met101 Oxidation degree (%)** | **PD-1 binding activity (%)** |
|---|---|---|---|---|---|
| **N3** | 20 | 2.8 | 5°C/3M | 10.5 | 100.0 |
| **N7** | 100 | 2.8 | | 9.3 | 97.3 |
| **N3** | 20 | 2.8 | 25°C/3M | 15.7 | 92.0 |
| **N7** | 100 | 2.8 | | 12.7 | 83.4 |
| **N3** | 20 | 2.8 | 40°C/3M | 49.5 | 63.4 |
| **N7** | 100 | 2.8 | | 18.2 | 78.2 |

### Example 6: Stability evaluation (2)

Since it was suggested in Example 5 that the stability of Antibody a pertaining to the present invention may decrease at low concentration, the stability thereof at low concentration was evaluated.

Samples L1 and L2 containing Antibody α at 1 and 50 mg/mL, respectively, were prepared in the acetate formulation (20 mM acetate buffer solution (pH 5.0), 285 mM trehalose, 0.1 % PS80, 1.25 mg/mL sodium chloride), as shown in Table 33.

**[Table 33]**

| **Samples** | **Antibody concentration (mg/mL)** | **pH** | **Buffer solution, Salt** | **Suger** | **Polysorbate** | **Conductivity of prepared formulation (mS/cm)** |
|---|---|---|---|---|---|---|
| **L1** | 1 | 5.0 | 20 mM acetate buffer solution | 285 mM trehalose | 0.1 % PS80 | 3.0 |
| **L2** | 50 | 5.0 | 20 mM acetate buffer solution | 285 mM trehalose | 0.1 % PS80 | 3.0 |

These 2 types of samples were sterilized with 0.22 µm filters, and dispensed 1 or 5 mL each in sterile vials, and the stability evaluation was conducted under the conditions listed in Table 34.

**[Table 34]**

| **Test items** | **Conditions** |
|---|---|
| **5°C/1M** | Stored at 5° C for 4 weeks |
| **25°C/1M** | Stored at 25° C for 4 weeks |
| **40°C/1M** | Stored at 40° C for 4 weeks |
| **5°C/3M** | Stored at 5° C for 12 weeks |
| **25°C/3M** | Stored at 25° C for 12 weeks |
| **40°C/3M** | Stored at 40° C for 12 weeks |

For the sample stored under each condition, (a) SEC-HPLC analysis, (b) IE-HPLC analysis, and (c) measurement of the binding activities to PD-1 and CD3 were performed, respectively. Note that SEC-HPLC analysis was performed under the condition shown in Table 24, and IE-HPLC analysis was performed under the condition shown in Table 25. The binding activities to PD-1 and CD3 were measured by SPR.
(a) The percentage of HMWS before and after storage, evaluated by SEC-HPLC, is shown in Table 35. In all formulations, HMWS increased significantly during long-term storage at 40 °C, but the accumulation of HMWS was more significant at the low antibody concentration, L1. It was suggested that the potential for aggregation increases under low concentration conditions.

**[Table 35]**

| **Samples** | **Antibody concentration (mg/mL)** | **pH** | **Conductivity (mS/cm)** | **5°C** | | | **25°C** | | | **40°C** | | |
|---|---|---|---|---|---|---|---|---|---|---|---|---|
| | | | | **0M** | **1M** | **3M** | **0M** | **1M** | **3M** | **0M** | **1M** | **3M** |
| **L1** | 1 | 5.0 | 3.0 | 2.8 | 2.5 | 3.0 | 2.8 | 2.3 | 2.8 | 2.8 | 2.8 | 18.8 |
| **L2** | 50 | 5.0 | 3.0 | 0.7 | 1.0 | 1.7 | 0.7 | 1.0 | 2.0 | 0.7 | 1.4 | 3.6 |

(b) The percentage of acidic molecular species before and after storage, evaluated by IE-HPLC, is shown in Table 36. At all concentrations, acidic molecular species tended to increase at 40 °C, and the oxidation tended to proceed more rapidly at 1 mg/mL.

**[Table 36]**

| **Samples** | **Antibody concentration (mg/mL)** | **pH** | **Conductivity (mS/cm)** | **5°C** | | | **25°C** | | | **40°C** | | |
|---|---|---|---|---|---|---|---|---|---|---|---|---|
| | | | | **0M** | **1M** | **3M** | **OM** | **1M** | **3M** | **0M** | **1M** | **3M** |
| **L1** | 1 | 5.0 | 3.0 | 45.8 | 45.3 | 45.6 | 45.8 | 47.2 | 52.6 | 45.8 | 58.0 | 72.0 |
| **L2** | 50 | 5.0 | 3.0 | 46.1 | 44.4 | 45.1 | 46.1 | 45.4 | 48.3 | 46.1 | 53.4 | 69.6 |

(c) The results of the binding activity to PD-1 before and after storage, as evaluated by SPR, are shown in Table 37. At 5 and 25 °C, there was no decrease in the binding activities of both PD-1 and CD3, but at 40 °C, there was a marked decrease in the binding activity to PD-1 in both samples. It was suggested that the oxidation of complementarity determining regions on PD-1 may have facilitated oxidation to antibodies.

**[Table 37]**

| **Samples** | **Antibody concentration (mg/mL)** | **pH** | **Conductivity (mS/cm)** | **5°C** | | | **25°C** | | | **40°C** | | |
|---|---|---|---|---|---|---|---|---|---|---|---|---|
| | | | | **0M** | **1M** | **3M** | **0M** | **1M** | **3M** | **0M** | **1M** | **3M** |
| **L1** | 1 | 5.0 | 3.0 | 97 | 92 | 90 | 97 | 87 | 73 | 97 | 71 | 12 |
| **L2** | 50 | 5.0 | 3.0 | 99 | 92 | 93 | 99 | 94 | 92 | 99 | 89 | 73 |

### Example 7: Additives evaluation (4)

It was indicated in Example 6 that the effect of the long-term storage resulted in the progressive oxidation of Antibody α pertaining to the present invention, resulting in a decrease in the binding activities to PD-1 and CD3, and in particular, a significant decrease in the binding activity to PD-1.

The addition of antioxidant (L-methionine or DTPA) to the acetate formulation (20 mM acetate buffer solution (pH 5.0), 193 mM trehalose, 0.1 % PS80, 1.15 mg/mL sodium chloride) was investigated. Samples A1 to A8 containing Antibody a at 1 and 20 mg/mL, respectively, were prepared as shown in Table 38.

**[Table 38]**

| **Samples** | **Antibody concentration (mg/mL)** | **pH** | **Buffer solution. Salt** | **Suger** | **Polysorbate** | **Conductivity of prepared formulation (mS/cm)** | **Antioxidants** |
|---|---|---|---|---|---|---|---|
| **A1** | 1 | 5.0 | 20 mM acetate buffer solution | 193 mM trehalose | 0.1 % PS80 | 2.8 | - |
| **A2** | 1 | 5.0 | 20 mM acetate buffer solution | 193 mM trehalose | 0.1 % PS80 | 2.7 | L-methionine 1mM |
| **A3** | 1 | 5.0 | 20 mM acetate buffer solution | 193 mM trehalose | 0.1 % PS80 | 2.7 | L-methionine 10 mM |
| **A4** | 1 | 5.0 | 20 mM acetate buffer solution | 193 mM trehalose | 0.1 % PS80 | 2.8 | DTPA 20 um |
| **A5** | 20 | 5.0 | 20 mM acetate buffer solution | 193 mM trehalose | 0.1 % PS80 | 2.8 | - |
| **A6** | 20 | 5.0 | 20 mM acetate buffer solution | 193 mM trehalose | 0.1 % PS80 | 2.8 | L-methionine 1mM |
| **A7** | 20 | 5.0 | 20 mM acetate buffer solution | 193 mM trehalose | 0.1 % PS80 | 2.7 | L-methionine 10 mM |
| **A8** | 20 | 5.0 | 20 mM acetate buffer solution | 193 mM trehalose | 0.1 % PS80 | 2.8 | DTPA 20 um |

The 8 types of samples were sterilized with a 0.22 µm filter, dispensed 1 mL each into sterilized vials, and evaluated for the stability as shown in Table 34. In the same evaluation, (a) SEC-HPLC analysis, (b) IE-HPLC analysis, (c) RP-HPLC analysis, (d) measurements of the binding activities to PD-1 and CD3, and (e) peptide map analysis were performed for the samples stored under the conditions listed in Table 34, respectively. Note that SEC-HPLC analysis was performed under the condition shown in Table 24, IE-HPLC analysis was performed under the condition shown in Table 25, and RP-HPLC analysis was performed under the condition shown in Table 26. In addition, the binding activities to PD-1 and CD3 were measured by SPR, and the peptide mapping analysis was performed using LC-MS.
(a) The percentage (%) of HMWS before and after storage, evaluated by SEC-HPLC analysis, is shown in Table 39. There was no change at 5 °C and 25 °C even without the addition of antioxidant. On the other hand, the generation of HMWS was dramatically suppressed for the samples with 10 mM of L-methionine or 20 µM of DTPA when stored at 40 °C for 12 weeks. The suppressive effect of L-methionine or DTPA on the generation of HMWS was also observed at antibody concentrations of 1 and 20 mg/mL.

**[Table 39]**

| **Concentration (mg/mL)** | **Formulations** | **5°C** | | | **25°C** | | | **40°C** | | |
|---|---|---|---|---|---|---|---|---|---|---|
| | | **0M** | **1M** | **3M** | **0M** | **1M** | **3M** | **0M** | **1M** | **3M** |
| **1** | **(No antioxidant)** | 1.8 | 2.1 | 1.7 | 1.8 | 2.1 | 1.8 | 1.8 | 2.6 | 29.6 |
| | **L-methionine 1 mM** | | | | | | | 1.8 | 2.3 | 3.9 |
| | **L-methionine 10 mM** | | | | | | | 1.9 | 2.3 | 2.2 |
| | **DTPA 20 *µ*M** | | | | | | | 2.0 | 2.5 | 2.4 |
| **20** | **(No antioxidant)** | 0.2 | 0.3 | 0.4 | 0.2 | 0.4 | 0.8 | 0.2 | 1.2 | 4.9 |
| | **L-methionine 1 mM** | | | | | | | 0.3 | 1.1 | 3.3 |
| | **L-methionine 10 mM** | | | | | | | 0.2 | 0.8 | 1.6 |
| | **DTPA 20 *µ*M** | | | | | | | 0.2 | 0.5 | 1.1 |

(b) The percentage (%) of acidic molecular species before and after storage, evaluated by IE-HPLC analysis, is shown in Table 40. At both antibody concentrations of 1 and 20 mg/mL, the acidic molecular species tended to increase at 40 °C. In particular, at the antibody concentration of 1 mg/mL, the addition of 10 mM L-methionine or 20 µM of DTPA was found to be effective in suppressing the generation of acidic molecular species.

**[Table 40]**

| **Concentration (mg/mL)** | **Formulations** | **5°C** | | | **25°C** | | | **40°C** | | |
|---|---|---|---|---|---|---|---|---|---|---|
| | | **0M** | **1M** | **3M** | **0M** | **1M** | **3M** | **0M** | **1M** | **3M** |
| **1** | **(No antioxidant)** | 29.7 | 30.1 | 30.9 | 29.7 | 31.8 | 39.8 | 29.7 | 47.2 | 69.3 |
| | **L-methionine 1 mM** | | | | | | | 29.5 | 45.2 | 71.7 |
| | **L-methionine 10 mM** | | | | | | | 29.8 | 42.5 | 63.8 |
| | **DTPA 20 *µ*M** | | | | | | | 29.6 | 42.2 | 60.6 |
| **20** | **(No antioxidant)** | 30.0 | 29.8 | 30.2 | 30.0 | 30.6 | 34.7 | 30.0 | 42.4 | 62.7 |
| | **L-methionine 1 mM** | | | | | | | 30.1 | 42.2 | 61.7 |
| | **L-methionine 10 mM** | | | | | | | 30.1 | 41.8 | 60.8 |
| | **DTPA 20 *µ*M** | | | | | | | 30.4 | 41.0 | 58.8 |

(c) The coloring index before and after storage, evaluated by IE-HPLC analysis, is shown in Table 41. At both antibody concentrations of 1 and 20 mg/mL, the dramatic suppression of the generation of colored entities at 40 °C was observed. In particular, at the antibody concentration of 1 mg/mL, the addition of 10 mM of L-methionine or 20 µM of DTPA was found to be effective in suppressing the generation of colored entities.

**[Table 41]**

| **Concentration (mg/mL)** | **Formulations** | **5°C** | | | **25°C** | | | **40°C** | | |
|---|---|---|---|---|---|---|---|---|---|---|
| | | **0M** | **1M** | **3M** | **0M** | **1M** | **3M** | **0M** | **1M** | **3M** |
| **1** | **(No antioxidant)** | 0.37 | 0.39 | 0.43 | 0.37 | 0.43 | 0.84 | 0.37 | 0.86 | 5.47 |
| | **L-methionine 1 mM** | | | | | | | 0.37 | 0.64 | 1.60 |
| | **L-methionine 10 mM** | | | | | | | 0.37 | 0.46 | 0.81 |
| | **DTPA 20 *µ*M** | | | | | | | 0.37 | 0.43 | 0.60 |
| **20** | **(No antioxidant)** | 0.37 | 0.37 | 0.40 | 0.37 | 0.38 | 0.45 | 0.37 | 0.45 | 0.75 |
| | **L-methionine 1 mM** | | | | | | | 0.37 | 0.44 | 0.65 |
| | **L-methionine 10 mM** | | | | | | | 0.37 | 0.42 | 0.54 |
| | **DTPA 20 *µ*M** | | | | | | | 0.37 | 0.40 | 0.48 |

(d) The results of the binding activity (%) to PD-1 in Table 42 and those of the binding activity (%) to CD3 in Table 43, before and after storage, evaluated by SPR, are shown, respectively. When stored at 25 °C or more without antioxidant, both of the binding activities to PD-1 and CD3 decreased at both antibody concentrations of 1 and 20 mg/mL. On the other hand, at 40°C, the addition of antioxidant dramatically suppressed the decrease in the binding activities at both antibody concentrations of 1 and 20 mg/mL. It was suggested that the suppression of the generation of HMWS and acidic molecular species by the addition of antioxidant resulted in the suppression the decrease in the binding activity.

**[Table 42]**

| **Concentration (mg/mL)** | **Formulations** | **5°C** | | | **25°C** | | | **40°C** | | |
|---|---|---|---|---|---|---|---|---|---|---|
| | | **0M** | **1M** | **3M** | **0M** | **1M** | **3M** | **0M** | **1M** | **3M** |
| **1** | **(No antioxidant)** | 104 | 95 | 92 | 104 | 89 | 72 | 104 | 73 | 4 |
| | **L-methionine 1 mM** | | | | | | | 106 | 85 | 47 |
| | **L-methionine 10 mM** | | | | | | | 108 | 92 | 75 |
| | **DTPA 20 *µ*M** | | | | | | | 103 | 91 | 76 |
| **20** | **(No antioxidant)** | 100 | 105 | 98 | 100 | 92 | 91 | 100 | 92 | 62 |
| | **L-methionine 1 mM** | | | | | | | 93 | 93 | 72 |
| | **L-methionine 10 mM** | | | | | | | 103 | 95 | 83 |
| | **DTPA 20 *µ*M** | | | | | | | 96 | 96 | 88 |

**[Table 43]**

| **Concentration (mg/mL)** | **Formulations** | **5°C** | | | **25°C** | | | **40°C** | | |
|---|---|---|---|---|---|---|---|---|---|---|
| | | **0M** | **1M** | **3M** | **0M** | **1M** | **3M** | **0M** | **1M** | **3M** |
| **1** | **(No antioxidant)** | 101 | 93 | 91 | 101 | 91 | 86 | 101 | 89 | 37 |
| | **L-methionine 1 mM** | | | | | | | 103 | 92 | 76 |
| | **L-methionine 10 mM** | | | | | | | 105 | 93 | 86 |
| | **DTPA 20 *µ*M** | | | | | | | 100 | 93 | 87 |
| **20** | **(No antioxidant)** | 97 | 101 | 97 | 97 | 98 | 93 | 97 | 95 | 86 |
| | **L-methionine 1 mM** | | | | | | | 92 | 95 | 90 |
| | **L-methionine 10 mM** | | | | | | | 100 | 96 | 92 |
| | **DTPA 20 *µ*M** | | | | | | | 94 | 95 | 92 |

(e) The factorial analysis by peptide mapping was performed for the decrease in the binding activity to PD-1 during long-term storage at 40 °C, as shown in Table 42. As a representative example, the degree of oxidation (%) of the methionine residue (Met101) in the complementarity determining region to PD-1 is shown in Table 44. It was observed that Met101 was highly oxidized at the antibody concentration of 1 mg/mL and when stored at 40 °C. On the other hand, the addition of antioxidant dramatically suppressed the oxidation of Met101, and also dramatically suppressed the decrease in the binding activity to PD-1. Thus, it was confirmed that the addition of antioxidant is extremely useful for improving the stability of Antibody α.

**[Table 44]**

| **Storage conditions** | **Concentration (mg/mL)** | **Formulation conditions** | **Met101 oxidation degree(%)** | **PD-1 binding activity(%)** |
|---|---|---|---|---|
| **5°C 1M** | **1** | **(No antioxidant)** | 7.3 | 95.4 |
| **25°C 1M** | **1** | **(No antioxidant)** | 17.7 | 88.6 |
| **40°C 1M** | **1** | **(No antioxidant)** | 58 | 72.5 |
| | **1** | **L-methionine 1 mM** | 14.3 | 84.8 |
| | **1** | **L-methionine 10 mM** | 4.3 | 92.3 |
| | **1** | **DTPA 20 *µ*M** | 25.1 | 90.7 |
| | **20** | **L-methionine 10 mM** | 5.9 | 94.9 |
| | **20** | **DTPA 20 *µ*M** | 7.6 | 96.0 |

### Example 8: Stability test (3)

In the investigations of Examples 1 to 7, the condition under which Antibody a pertaining to the present invention could be stably stored was found. It was decided to determine a range of sugar concentration in order to establish even more stable conditions.

Samples T1 to T3 containing Antibody α at 1 mg/mL in the acetate formulation (20 mM acetate buffer solution (pH 5.0), 172 mM, 182.38 mM or 193 mM trehalose, 0.1 % PS80, 1.36 mg/mL sodium chloride, 10 mM L-methionine) were prepared as shown in Table 45. Note that each concentration of trehalose, "182 mM" in Table 45 and Tables 47 to 59, is an abbreviated notation of "182.38 mM".

**[Table 45]**

| **Samples** | **Antibody concentration (mg/mL)** | **pH** | **Buffer solution. Salt** | **Suger** | **Polysorbate** | **Conductivity of prepared formulation (mS/cm)** | **Antioxidant L-methionine** |
|---|---|---|---|---|---|---|---|
| **T1** | 1.0 | 5.0 | 20 mM acetate buffer solution | 193 mM trehalose | 0.1 % PS80 | 3.1 | 10 mM |
| **T2** | 1.0 | 5.0 | 20 mM acetate buffer solution | 182 mM trehalose | 0.1 % PS80 | 3.1 | 10 mM |
| **T3** | 1.0 | 5.0 | 20 mM acetate buffer solution | 172 **mM** trehalose | 0.1 % PS80 | 3.1 | 10 mM |

These 2 types of samples were sterilized with 0.22 µm filters and dispensed into sterile vials at 1 mL each and then the stability evaluation was performed under the conditions listed in Table 46.

**[Table 46]**

| **Test items** | **Conditions** |
|---|---|
| **5°C/0.5M** | Stored at 5° C for 2 weeks |
| **25°C/0.5M** | Stored at 25° C for 2 weeks |
| **40°C/0.5M** | Stored at 40° C for 2 weeks |
| **5°C/1M** | Stored at 5° C for 4 weeks |
| **25°C/1M** | Stored at 25° C for 4 weeks |
| **40°C /1M** | Stored at 40° C for 4 weeks |
| **5°C/3M** | Stored at 5° C for 12 weeks |
| **25°C/3M** | Stored at 25° C for 12 weeks |
| **40°C/3M** | Stored at 40° C for 12 weeks |

For samples stored under each condition, (a) IE-HPLC analysis, (b) RP-HPLC and (c) measurements of the binding activities to PD-1 and CD3, were performed, respectively. IE-HPLC analysis was performed under the condition listed in Table 25. RP-HPLC was performed under the condition listed in Table 26. The measurement of the binding activities to PD-1 and CD3 was performed by SPR.
(a) The percentage (%) of acidic molecular species before and after storage, evaluated by IE-HPLC, is shown in Table 47. In all formulations, the acidic molecular species increased during long-term storage at 25 °C and 40 °C. They were stable at 5 °C, the normal storage temperature. No differences were observed among the formulations.

**[Table 47]**

| **Samples** | **Concentration (mg/mL)** | **Formulations** | **5°C** | | | | **25°C** | | | | **40°C** | | | |
|---|---|---|---|---|---|---|---|---|---|---|---|---|---|---|
| | | | **0M** | **0.5M** | **1M** | **3M** | **0M** | **0.5M** | **1M** | **3M** | **0M** | **0.5M** | **1M** | **3M** |
| **T1** | **1** | 193 mM trehalose | 29.4 | | 28.8 | 28.7 | 29.4 | 28.9 | 30.0 | 34.8 | 29.4 | 35.1 | 43.0 | 66.5 |
| **T2** | | 182 mM trehalose | 29.4 | | 28.7 | 28.9 | 29.4 | 28.8 | 29.9 | 34.6 | 29.4 | 34.7 | 41.9 | 65.2 |
| **T3** | | 172 mM trehalose | 29.4 | | 28.9 | 28.9 | 29.4 | 28.9 | 29.8 | 34.6 | 29.4 | 34.6 | 42.0 | 66.1 |

(b) The percentage of colored entities before and after storage, evaluated by RP-HPLC, is shown in Table 48. In all formulations, the colored entities increased during long-term storage at 40 °C. No differences were observed among the formulations.

**[Table 48]**

| **Samples** | **Concentration (mg/mL)** | **Formulations** | **5°C** | | | | **25°C** | | | | **40°C** | | | |
|---|---|---|---|---|---|---|---|---|---|---|---|---|---|---|
| | | | **0M** | **0.5M** | **1M** | 3M | **0M** | **0.5M** | **1M** | **3M** | **0M** | **0.5M** | **1M** | **3M** |
| **T1** | 1 | 193 mM trehalose | 0.29 | | 0.27 | 0.28 | 0.29 | 0.30 | 0.28 | 0.35 | 0.29 | 0.34 | 0.38 | 0.62 |
| **T2** | | 182 mM trehalose | 0.28 | | 0.27 | 0.27 | 0.28 | 0.30 | 0.27 | 0.33 | 0.28 | 0.32 | 0.34 | 0.54 |
| **T3** | | 172 mM trehalose | 0.29 | | 0.27 | 0.27 | 0.29 | 0.30 | 0.27 | 0.32 | 0.29 | 0.33 | 0.33 | 0.58 |

(C) The results of the binding activity to PD-1 in Table 49 and the results of the binding activity to CD3 in Table 50, before and after storage test, evaluated by SPR, are shown, respectively. The values in each table represent the specific activity (%) of Antibody α to the standard product. No significant decrease in the binding activities to PD-1 and CD-3 was observed under any conditions. In addition, no difference was observed among the formulations.

**[Table 49]**

| **Samples** | **Concentration (mg/mL)** | **Formulations** | **5°C** | | | | **25°C** | | | | **40°C** | | | |
|---|---|---|---|---|---|---|---|---|---|---|---|---|---|---|
| | | | **0M** | **0.5M** | **1M** | **3M** | **0M** | **0.5M** | **1M** | **3M** | **0M** | **0.5M** | **1M** | **3M** |
| **T1** | 1 | 193 mM trehalose | 106 | | | | 106 | 100 | | | 106 | 93 | 92 | |
| **T2** | | 182 mM trehalose | 102 | | | | 102 | 98 | | | 102 | 96 | 94 | |
| **T3** | | 172 mM trehalose | 103 | | | | 103 | 95 | | | 103 | 96 | 93 | |

**[Table 50]**

| **Samples** | **Concentration (mg/mL)** | **Formulations** | **5°C** | | | | **25°C** | | | | **40°C** | | | |
|---|---|---|---|---|---|---|---|---|---|---|---|---|---|---|
| | | | **0M** | **0.5M** | **1M** | **3M** | **0M** | **0.5M** | **1M** | **3M** | **0M** | **0.5M** | **1M** | **3M** |
| **T1** | 1 | 193 mM trehalose | 102 | | | | 102 | 96 | | | 102 | 92 | 93 | |
| **T2** | | 182 mM trehalose | 100 | | | | 100 | 95 | | | 100 | 96 | 95 | |
| **T3** | | 172 mM trehalose | 101 | | | | 101 | 93 | | | 101 | 97 | 95 | |

### Example 9: Stability test (4)

In the investigations of Examples 1 to 8, the condition under which Antibody α pertaining to the present invention could be stably stored was found. A stability test was performed under the condition considered optimal by comprehensively taking them into consideration.

Samples F1 and F2 containing Antibody α at 1 mg/mL or 100 mg/mL, respectively, in the acetate formulation (20 mM acetate buffer solution (pH 5.0), 182.38 mM trehalose, 0.1 % PS80, 1.36 mg/mL sodium chloride, 10 mM L-methionine) were prepared as shown in Table 51.

**[Table 51]**

| **Samples** | **Antibody concentration (mg/mL)** | **pH** | **Buffer solution. Salt** | **Suger** | **Polysorbate** | **Conductivity of prepared formulation (mS/cm)** | **Antioxidant L-methionine** |
|---|---|---|---|---|---|---|---|
| **F1** | 1.0 | 5.0 | 20 mM acetate buffer solution | 182 mM trehalose | 0.1 % PS80 | 3.1 | 10 mM |
| **F2** | 100 | 5.0 | 20 mM acetate buffer solution | 182 mM trehalose | 0.1 % PS80 | 2.8 | 10 mM |

These 2 types of samples were sterilized with 0.22 µm filters and dispensed into sterile vials at 1 mL each, and then the stability evaluation was performed under the conditions listed in Table 52.

**[Table 52]**

| **Test items** | **Conditions** |
|---|---|
| **5°C/1M** | Stored at 5° C for 4 weeks |
| **25°C/1M** | Stored at 25° C for 4 weeks |
| **40°C/1M** | Stored at 40° C for 4 weeks |
| **5°C/3MM** | Stored at 5° C for 12 weeks |
| **25°C/3M** | Stored at 25° C for 12 weeks |
| **40°C/3M** | Stored at 40° C for 12 weeks |
| **5°C/6M** | Stored at 5° C for 24 weeks |
| **25°C/6M** | Stored at 25° C for 24 weeks |
| **40°C/6M** | Storage at 40° C for 24 weeks |

For samples stored under each condition, (a) SEC-HPLC, (b) IE-HPLC analysis, (c) isoelectric point electrophoresis (iCIEF), (d) RP-HPLC, and (e) measurements of the binding activities to PD-1 and CD3 were performed, respectively. SEC-HPLC analysis was performed under the condition shown in Table 24. IE-HPLC analysis was performed under the condition shown in Table 25. iCIEF was performed by capillary isoelectrophoresis. RP-HPLC was performed under the condition shown in Table 26. The measurement of the binding activities to PD-1 and CD3 was performed by SPR.
(a) The percentage of HMWS before and after storage, evaluated by SEC-HPLC, is shown in Table 53. HMWS increased under the condition of 100 mg/mL concentration at 25 °C and 40 °C, but there was no significant increase at 5 °C. It was considered to be stable at 5 °C.

**[Table 53]**

| **Sample** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **OM** | **1M** | **3M** | **6M** |
| F2 | 100 | 0.08 | 0.33 | 0.39 | 0.45 | 0.08 | 0.56 | 1.02 | 1.10 | 0.08 | 11.19 | 2.34 | - |

(b) The percentage of acidic molecular species before and after storage, evaluated by IE-HPLC, is shown in Table 54. The acidic molecular species increased under the conditions of long-term storage at 25 °C and 40 °C, but there was no significant increase at 5 °C. It was considered to be stable at 5 °C.

**[Table 54]**

| **Samples** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **0M** | **1M** | **3M** | **6M** |
| **F1** | 1.0 | 34.6 | 34.2 | 34.4 | 34.3 | 34.6 | 34.7 | 39.4 | 45.3 | 34.6 | 46.4 | 67.9 | 83.2 |
| **F2** | 100 | 34.3 | 33.9 | 34.0 | 34.6 | 34.3 | 34.4 | 38.2 | 42.9 | 34.3 | 45.7 | 65.4 | - |

(c) The percentage (%) of acidic molecular species before and after storage, evaluated by iCIEF, is shown in Table 55. The acidic molecular species increased under the conditions of long-term storage at 25 °C and 40 °C, but there was no significant increase at 5°C. It was considered to be stable at 5°C.

**[Table 55]**

| **Samples** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **0M** | **1M** | **3M** | **6M** |
| **F1** | 1.0 | 40.9 | 46.6 | 44.2 | - | 40.9 | 48.4 | 49.0 | - | 40.9 | 58.8 | 77.5 | - |
| **F2** | 100 | 43.8 | 43.5 | 43.4 | - | 43.8 | 45.1 | 48.0 | - | 43.8 | 59.0 | 76.0 | - |

(d) The percentage of colored entities before and after storage, evaluated by RP-HPLC, is shown in Table 56. The colored entities increased under the conditions of long-term storage at 25 °C and 40 °C, but there was no significant increase at 5 °C. It was considered to be stable at 5 °C.

**[Table 56]**

| **Samples** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **0M** | **1M** | **3M** | **6M** |
| **F1** | 1.0 | 0.47 | 0.52 | 0.47 | 0.43 | 0.47 | 0.53 | 0.54 | 0.61 | 0.47 | 0.60 | 0.84 | 1.21 |
| **F2** | 100 | 0.47 | 0.52 | 0.47 | 0.42 | 0.47 | 0.52 | 0.52 | 0.52 | 0.47 | 0.58 | 0.67 | - |

(e) The results of the binding activity to PD-1 in Table 57, and those of the binding activity to CD3 in Table 58, before and after storage test, evaluated by SPR, are shown, respectively. The values in each table represent the specific activities (%) to the standard product. At 40 °C, a decrease in the binding activity to PD-1 was observed. On the other hand, at 5 °C and 25 °C, there was no decrease in both of the binding activities of PD-1 and CD3, indicating that they are sufficiently stable.

**[Table 57]**

| **Samples** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **0M** | **1M** | **3M** | **6M** |
| **F1** | 1.0 | 96 | 113 | 103 | 97 | 96 | 110 | 89 | 101 | 96 | 106 | 72 | 73 |
| **F2** | 100 | 104 | 115 | 102 | 110 | 104 | 112 | 100 | 107 | 104 | 113 | 90 | - |

**[Table 58]**

| **Samples** | **Concentration (mg/mL)** | **5°C** | | | | **25°C** | | | | **40°C** | | | |
|---|---|---|---|---|---|---|---|---|---|---|---|---|---|
| | | **0M** | **1M** | **3M** | **6M** | **0M** | **1M** | **3M** | **6M** | **0M** | **1M** | **3M** | **6M** |
| **F1** | 1.0 | 93 | 108 | 99 | 94 | 93 | 108 | 89 | 104 | 93 | 113 | 83 | 95 |
| **F2** | 100 | 100 | 111 | 99 | 107 | 100 | 110 | 99 | 107 | 100 | 114 | 95 | - |

Samples L1 to L4 containing Antibody α at 0.01 mg/mL to 1.0 mg/mL, respectively, in the acetate formulation (20 mM acetate buffer solution (pH 5.0), 182.38 mM trehalose, 0.1 % PS80, 1.36 mg/mL sodium chloride, 10 mM L-methionine) were prepared as shown in Table 59 in order to assess the stability of formulation at lower concentration.

**[Table 59]**

| **Samples** | **Antibody concentration (mg/mL)** | **pH** | **Buffer solution, Salt** | **Suger** | **Polysorbate** | **Conductivity after adding sodium chloride (mS/cm)** | **Antioxidant L-methionine** |
|---|---|---|---|---|---|---|---|
| **L1** | 0.01 | 5.0 | 20 mM acetate buffer solution | 182 mM trehalose | 0.1 % PS80 | 3.10 | 10 mm |
| **L2** | 0.1 | 5.0 | 20 mM acetate buffer solution | 182 mM trehalose | 0.1 % PS80 | 3.10 | 10 mm |
| **L3** | 0.5 | 5.0 | 20 mM acetate buffer solution | 182 mM trehalose | 0.1 % PS80 | 3.10 | 10 mM |
| **L4** | 1.0 | 5.0 | 20 mM acetate buffer solution | 182 mM trehalose | 0.1 % PS80 | 3.10 | 10 mM |

These 2 types of samples were sterilized using 0.22 µm filters and dispensed into sterilized vials, and then the stability evaluation was performed under the conditions listed in Table 60.

**[Table 60]**

| **Test items** | **Conditions** |
|---|---|
| **5°C/0.5M** | Stored at 5° C for 2 weeks |
| **25°C/0.5M** | Stored at 25° C for 2 weeks |
| **40°C/0.5M** | Stored at 40° C for 2 weeks |
| **5°C/1M** | Stored at 5° C for 4 weeks |
| **25°C/1M** | Stored at 25° C for 4 weeks |
| **40°C/1M** | Stored at 40° C for 4 weeks |
| **5°C/3M** | Stored at 5° C for 12 weeks |
| **25°C/3M** | Stored at 25° C for 12 weeks |
| **40°C/3M** | Stored at 40° C for 12 weeks |
| **5°C/6M** | Stored at 5° C for 24 weeks |
| **25°C/6M** | Stored at 25° C for 24 weeks |
| **40°C/6M** | Stored at 40° C for 24 weeks |

Analytical evaluations as the above evaluations in the present examples were performed for the samples stored under each condition.
(a) The percentage of HMWS before and after storage, evaluated by SEC-HPLC, is shown in Table 61. At concentrations of 0.5 mg/mL and 1.0 mg/mL, no increase in the percentage of HMWS was observed under the conditions at 5, 25, and 40 °C.

**[Table 61]**

| **Samples** | **Concentration (mg/mL)** | **5°C** | | | | | | **25°C** | | | | | | **40°C** | | | | | |
|---|---|---|---|---|---|---|---|---|---|---|---|---|---|---|---|---|---|---|---|
| | | **0M** | **0.5M** | **1M** | **2M** | **3M** | **6M** | **0M** | **0.5M** | **1M** | **2M** | **3M** | **6M** | **0M** | **0.5M** | **1M** | **2M** | **3M** | **6M** |
| **L3** | 0.5 | 5.6 | 5.1 | 4.1 | 5.5 | 5.6 | 4.7 | 5.6 | 4.8 | 3.9 | 5.3 | 5.7 | 5.0 | 5.6 | 4.5 | 4.0 | 5.5 | 5.9 | - |
| **L4** | 1.0 | 3.2 | 2.6 | 2.2 | 3.0 | 3.0 | 2.4 | 3.2 | 2.6 | 2.1 | 2.9 | 3.1 | 2.6 | 3.2 | 2.3 | 2.0 | 2.9 | 3.3 | - |

(b) The ratio of acidic molecular species before and after storage, evaluated by IE-HPLC is shown in Table 62. An increase in acidic molecular species was observed under the conditions of long-term storage at 25 °C and 40 °C, but there was no significant increase was observed at 5 °C. It was considered to be stable at 5 °C.

**[Table 62]**

| **Samples** | **Concentration (mg/mL)** | **5°C** | | | | | | **25°C** | | | | | | **40°C** | | | | | |
|---|---|---|---|---|---|---|---|---|---|---|---|---|---|---|---|---|---|---|---|
| | | **0M** | **0.5M** | **1M** | **2M** | **3M** | **6M** | **0M** | **0.5M** | **1M** | **2M** | **3M** | **6M** | **0M** | **0.5M** | **1M** | **2M** | **3M** | **6M** |
| **L2** | 0.1 | 21.3 | 22.5 | 23.3 | 16.6 | 20.4 | 25.4 | 21.3 | 22.2 | 25.0 | 19.4 | 26.0 | 35.7 | 21.3 | 27.8 | 37.6 | 42.0 | 55.9 | - |
| **L3** | 0.5 | 23.1 | 22.4 | 23.7 | 22.4 | 23.5 | 24.7 | 23.1 | 23.2 | 25.1 | 25.6 | 28.3 | 35.4 | 23.1 | 30.0 | 38.1 | 46.8 | 58.5 | - |
| **L4** | 1.0 | 23.5 | 23.1 | 24.0 | 23.3 | 23.9 | 24.8 | 23.5 | 23.7 | 25.4 | 26.3 | 29.1 | 35.7 | 23.5 | 30.7 | 38.2 | 47.3 | 58.9 | - |

(c) The proportion (%) of acidic molecular species before and after storage, evaluated by iCIEF is shown in Table 63. An increase in acidic molecular species was observed under the conditions of long-term storage at 25 °C and 40 °C, but there was no significant increase was observed at 5 °C. It was considered to be stable at 5°C.

**[Table 63]**

| **Samples** | **Concentration (mg/mL)** | **5°C** | | | | | | **25°C** | | | | | | **40°C** | | | | | |
|---|---|---|---|---|---|---|---|---|---|---|---|---|---|---|---|---|---|---|---|
| | | **0M** | **0.5M** | **1M** | **2M** | **3M** | **6M** | **0M** | **0.5M** | **1M** | **2M** | **3M** | **6M** | **0M** | **0.5M** | **1M** | **2M** | **3M** | **6M** |
| **L3** | 0.5 | 32.5 | 36.2 | 32.8 | 31.9 | 31.2 | 32.4 | 32.5 | 35.8 | 36.6 | 36.9 | 37.7 | 38.1 | 32.5 | 42.3 | 47.1 | 56.6 | 63.4 | 78.6 |
| **L4** | 1.0 | 31.9 | 32.7 | 32.6 | 30.2 | 30.2 | 32.2 | 31.9 | 34.6 | 35.4 | 33.9 | 35.6 | 42.7 | 31.9 | 41.3 | 47.8 | 58.1 | 63.6 | 77.6 |

(d) The results of the binding activity to PD-1 in Table 64, and those of the binding activity to CD3 in Table 65, before and after the storage test evaluated by SPR, are shown, respectively. The numerical values in each table represent the specific activity (%) to the standard product. No decrease in the binding activity was observed for either PD-1 or CD3 across all temperature and concentration ranges, indicating that they are sufficiently stable.

**[Table 64]**

| **Samples** | **Concentration (mg/mL)** | **5°C** | | | | | | **25°C** | | | | | | **40°C** | | | | | |
|---|---|---|---|---|---|---|---|---|---|---|---|---|---|---|---|---|---|---|---|
| | | **0M** | **0.5M** | **1M** | **2M** | **3M** | **6M** | **0M** | **0.5M** | **1M** | **2M** | **3M** | **6M** | **0M** | **0.5M** | **1M** | **2M** | **3M** | **6M** |
| **L1** | 0.01 | 111 | 114 | 112 | 112 | 108 | 126 | 111 | 114 | 110 | 112 | 106 | 139 | 111 | 113 | 109 | 110 | 108 | - |
| **L2** | 0.1 | 94 | 107 | 107 | 107 | 100 | 114 | 94 | 102 | 105 | 107 | 102 | 104 | 94 | 103 | 108 | 93 | 94 | - |
| **L3** | 0.5 | 98 | 108 | 107 | 105 | 103 | 108 | 98 | 105 | 106 | 107 | 102 | 110 | 98 | 107 | 102 | 99 | 99 | - |
| **L4** | 1.0 | 108 | 109 | 106 | 111 | 101 | 108 | 108 | 106 | 107 | 109 | 104 | 103 | 108 | 105 | 102 | 100 | 95 | - |

**[Table 65]**

| **Samples** | **Concentration (mg/mL)** | **5°C** | | | | | | **25°C** | | | | | | **40°C** | | | | | |
|---|---|---|---|---|---|---|---|---|---|---|---|---|---|---|---|---|---|---|---|
| | | **0M** | **0.5M** | **1M** | **2M** | **3M** | **6M** | **0M** | **0.5M** | **1M** | **2M** | **3M** | **6M** | **0M** | **0.5M** | **1M** | **2M** | **3M** | **6M** |
| **L1** | **0.01** | 104 | 107 | 107 | 106 | 97 | 122 | 104 | 106 | 106 | 105 | 97 | 138 | 104 | 106 | 108 | 108 | 106 | - |
| **L2** | **0.1** | 88 | 102 | 104 | 102 | 90 | 109 | 88 | 97 | 102 | 102 | 95 | 103 | 88 | 98 | 107 | 92 | 83 | - |
| **L3** | **0.5** | 92 | 102 | 103 | 100 | 93 | 104 | 92 | 100 | 103 | 102 | 95 | 108 | 92 | 101 | 101 | 98 | 99 | - |
| **L4** | **1.0** | 101 | 103 | 101 | 104 | 85 | 104 | 101 | 100 | 104 | 104 | 96 | 101 | 101 | 100 | 101 | 100 | 94 | - |

### Example 10: Formulation preparation method

A method for preparing the aqueous pharmaceutical composition of the present invention is described below using Sample F2 in Example 9 as an example. Note that the aqueous pharmaceutical composition of the present invention at an antibody concentration different from Sample F2 can also be prepared in the same manner.
(a) Formulation solution 1 is prepared to contain 20 mM acetate buffer solution (pH 5.0), 182.38 mM trehalose, 1.36 mg/mL sodium chloride and 10 mM L-methionine.
(b) Formulation solution 2 is prepared to contain 20 mM acetate buffer solution (pH 5.0), 182.38 mM trehalose, 2% PS80, 1.36 mg/mL sodium chloride and 10 mM L-methionine.
(c) Formulation solution 3 is prepared to contain 20 mM acetate buffer solution (pH 5.0), 182.38 mM trehalose, 0.1% PS80, 1.36 mg/mL sodium chloride and 10 mM L-methionine.
(d) In the manufacturing process of Antibody α, the solution composition of the process liquid obtained from the virus filtration process is replaced to be Formulation solution 1 using ultrafiltration/diafiltration membrane.
(e) In the preceding item (d), the process liquid containing Antibody α, of which the solution composition was replaced with Formulation solution 1, is concentrated to be at about 140 mg/mL using ultrafiltration/diafiltration membrane.
(f) Formulation solution 2 containing a high concentration of PS80 is added to the process liquid containing Antibody α concentrated in the preceding item (e) to adjust the concentration of PS80 to 0.1%.
(g) Formulation Solution 3 is added to the process solution containing Antibody α adjusted to the desired PS80 concentration in the preceding item (f), and diluted so that the antibody concentration is the desired 100 mg/mL.

### [Industrial Availability]

The pharmaceutical composition or formulation thereof of the present invention, containing an anti-PD-1/CD3 bispecific antibody as an active ingredient. is useful in the prevention, suppression of symptom progression, suppression of recurrence or treatment of autoimmune disease or graft-versus-host disease (GVHD), or hematological cancer.

**[Sequence Listing]**

## Claims

1. An aqueous pharmaceutical composition or a formulation thereof for subcutaneous or intravenous administration, containing:
(a) 0.1 to 1.2 mg/mL of an anti-PD-1/CD3 bispecific antibody as an active ingredient; and further containing:
(b) 18 to 22 mM of an acetate buffer;
(c) 172 to 193 mM of trehalose;
(d) 5 to 15 mM of L-methionine;
(e) 0.05 to 0.3 % (w/v) of polysorbate 80; and
(f) 19.6 to 25.7 mM of sodium chloride, and
of which a pH is 4.9 to 5.1, and
wherein the anti-PD-1/CD3 bispecific antibody comprises:
(i) a heavy chain and a light chain constituting an antigen-binding site specifically binding to PD-1; and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

2. The aqueous pharmaceutical composition or formulation thereof according to claim 1,
wherein the anti-PD-1/CD3 bispecific antibody is at 0.5 mg/mL or 1 mg/mL.

3. An aqueous pharmaceutical composition or a formulation thereof for subcutaneous or intravenous administration, containing:
(a) 0.5 mg/mL or 1 mg/mL of an anti-PD-1/CD3 bispecific antibody as an active ingredient; and further containing:
(b) 20.07 mM of an acetate buffer;
(c) 182.38 mM of trehalose;
(d) 10.05 mM of L-methionine;
(e) 0.1% (w/v) of polysorbate 80; and
(f) 23.27 mM of sodium chloride, and
of which a pH is 5.0, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

4. An aqueous pharmaceutical composition or a formulation thereof for subcutaneous or intravenous administration, containing:
(a) 0.1 to 1.2 mg/mL of an anti-PD-1/CD3 bispecific antibody as an active ingredient; and further containing:
(b) an acetate buffer consisting of
(b1) 1.68 to 2.05 mg/mL of sodium acetate trihydrate; and
(b2) the amount of acetic acid that makes a pH 4.9 to 5.1, corresponding to the amount of sodium acetate trihydrate of the preceding item (b1),
(c) 65.1 to 72.6 mg/mL of trehalose dihydrate;
(d) 0.75 to 2.25 mg/mL of L-methionine;
(e) 0.5 to 3.0 mg/mL of polysorbate 80; and
(f) 1.15 to 1.5 mg/mL of sodium chloride, and
of which the pH is 4.9 to 5.1, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

5. The aqueous pharmaceutical composition or formulation thereof according to claim 4, wherein the anti-PD-1/CD3 bispecific antibody is at 0.5 mg/mL or 1 mg/mL.

6. An aqueous pharmaceutical composition or a formulation thereof for subcutaneous or intravenous administration, containing:
(a) 0.5 mg/mL or 1 mg/mL of an anti-PD-1/CD3 bispecific antibody as an active ingredient; and further containing:
(b) an acetate buffer consisting of 1.87 mg/mL of sodium acetate trihydrate and 0.38 mg/mL of acetic acid;
(c) 69 mg/mL of trehalose dihydrate;
(d) 1.5 mg/mL of L-methionine;
(e) 1.0 mg/mL of polysorbate 80; and
(f) 1.36 mg/mL of sodium chloride, and
of which a pH is 5.0, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

7. An aqueous pharmaceutical composition or a formulation thereof for subcutaneous or intravenous administration, containing:
(a) 0.5 to 6 mg of an anti-PD-1/CD3 bispecific antibody as an active ingredient; and further containing:
(b) an acetate buffer consisting of:
(b1) 8.4 to 10.2 mg of sodium acetate trihydrate; and
(b2) the amount of glacial acetic acid that makes a pH 4.9 to 5.1, corresponding to the amount of sodium acetate trihydrate of the preceding item (b1);
(c) 325 to 365.1 mg of trehalose dihydrate;
(d) 3.75 to 11.25 mg of L-methionine;
(e) 2.5 to 15.0 mg of polysorbate 80; and
(f) 5.8 to 7.5 mg of sodium chloride, and
of which the pH is 4.9 to 5.1, and a volume per formulation is 5 mL, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

8. The aqueous pharmaceutical composition or formulation thereof according to claim 7, wherein the anti-PD-1/CD3 bispecific antibody is at 2.5 mg or 5 mg.

9. An aqueous pharmaceutical composition or a formulation thereof for subcutaneous or intravenous administration, containing:
(a) 2.5 mg or 5 mg of an anti-PD-1/CD3 bispecific antibody as an active ingredient; and further containing:
(b) an acetate buffer consisting of 9.35 mg of sodium acetate trihydrate and 1.9 mg of glacial acetic acid;
(c) 345 mg of trehalose dihydrate;
(d) 7.5 mg of L-methionine;
(e) 5.0 mg of polysorbate 80; and
(f) 6.8 mg of sodium chloride, and
of which a pH is 5.0, and a volume per formulation is 5 mL, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

10. An aqueous pharmaceutical composition or a formulation thereof for subcutaneous or intravenous administration, containing:
(a) 0.2 to 2.4 mg of an anti-PD-1/CD3 bispecific antibody as an active ingredient; and further containing:
(b) an acetate buffer consisting of:
(b1) 3.36 to 4.1 mg of sodium acetate trihydrate; and
(b2) the amount of glacial acetic acid that makes a pH 4.9 to 5.1, corresponding to the amount of sodium acetate trihydrate of the preceding item (b1);
(c) 130.2 to 145.2 mg of trehalose dihydrate;
(d) 1.5 to 4.5 mg of L-methionine;
(e) 1.0 to 6.0 mg of polysorbate 80; and
(f) 2.3 to 3.0 mg of sodium chloride, and
of which the pH is 4.9 to 5.1, and a volume per formulation is 2 mL, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.

11. The aqueous pharmaceutical composition or formulation thereof according to claim 10, wherein the anti-PD-1/CD3 bispecific antibody is at 1 mg.

12. An aqueous pharmaceutical composition or a formulation thereof for subcutaneous or intravenous administration, containing:
(a) 1 mg of an anti-PD-1/CD3 bispecific antibody as an active ingredient; and further containing:
(b) an acetate buffer consisting of 3.74 mg of sodium acetate trihydrate and 0.76 mg of glacial acetic acid;
(c) 138 mg of trehalose dihydrate;
(d) 3.0 mg of L-methionine;
(e) 2.0 mg of polysorbate 80; and
(f) 2.72 mg of sodium chloride, and
of which a pH is 5.0, and a volume per formulation is 2 mL, and
wherein the anti-PD-1/CD3 bispecific antibody comprises:
(i) a heavy chain and a light chain constituting an antigen-binding site specifically binding to PD-1, and
(ii) a heavy chain and a light chain constituting an antigen-binding site specifically binding to CD3, and
wherein (α) the heavy chain of the preceding item (i) comprises the amino acid sequence set forth in SEQ ID NO:5,
(β) the heavy chain of the preceding item (ii) comprises the amino acid sequence set forth in SEQ ID NO:6, and
(γ) both of the light chains of the preceding items (i) and (ii) comprise the amino acid sequence set forth in SEQ ID NO:7.
